# EUROPEAN PATENT APPLICATION

(11) **EP 3 666 769 A1**
(43) Date of publication of application: **17.06.2020**
(21) Application number: 18212075.8
(22) Date of filing: 12.12.2018
(51) Int. Cl.: C07D 401/12, C07D 257/04, A61P 35/00, A61K 31/4427, A61K 31/41

(54) **NOVEL CASPASE INHIBITORS**

(71) Applicant: RIJKSUNIVERSITEIT GRONINGEN, 9712 CP Groningen (NL); Consiglio Nazionale Delle Ricerche - CNR, 00185 Roma (IT)
(72) Inventor: DÖMLING, Alexander Stephan Siegfried, 9713 AV Groningen (NL); SPANU, Pietro, 07100 Sassari / Sardegna (IT); ULGHERI, Fausta, 07100 Sassari / Sardegna (IT)
(74) Representative: Forstmeyer, Dietmar

(57) **Abstract**

The present invention provides novel compounds of formula (I) that are potent and selective non-covalent caspase inhibitors. These compounds nay be used for the treatment of inflammatory diseases.

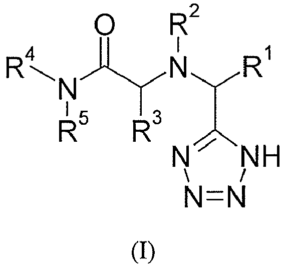

## Description

The present invention provides novel compounds that are potent and selective non-covalent caspase inhibitors. These compounds may be used for the treatment of inflammatory diseases.

Caspase-1 is the key enzyme driving inflammation through the activation of IL1b and IL18. It plays a key driving role in diseases such as Alzheimer, Parkinson, stroke, diabetes, multiple sclerosis, amyotrophic lateral sclerosis. Caspase-1 inhibitors are therefore of great interest for the treatment of a variety of diseases.

Several caspase inhibitors have already been elucidated in clinical trials and are all covalent inhibitors (e.g. Belnacasan, Pralnacasan). None of the compounds showed sufficient efficacy to be approved for use for any of the applications suggested to date. Covalent inhibitors might have secondary targets and therefore show toxic side effects. On the other hand non covalent inhibitors might be more selective and exhibit less side effects. Therefore, we present here a class of noncovalent Caspase-1 inhibitors.

The present invention provides compounds of formula (I): 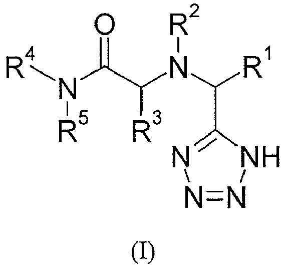 wherein
R¹ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R² is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R³ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R⁴ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted; and
R⁵ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
or a pharmaceutically acceptable salt, ester, solvate or hydrate or a pharmaceutically acceptable formulation thereof.

In the following, preferred embodiments of the present invention are disclosed. It is preferred that the preferred embodiments may be combined in any manner:
Preferably, R¹ is alkylcycloalkyl containing 4 to 16 carbon atoms; heteroalkylcycloalkyl containing 4 to 16 atoms selected from C, O, S and N; aralkyl containing 7 to 16 carbon atoms; or heteroaralkyl containing 6 to 16 atoms selected from C, O, S and N; wherein all these groups may optionally be substituted.

More preferably, R¹ is a group of formula -CH₂-X-R¹⁰ wherein X is O, S or NH (especially O) and R¹⁰ is a cycloalkyl, heterocycloalkyl, alkylcycloalkyl, heteroalkylcycloalkyl, aryl, heteroaryl, aralkyl or heteroaralkyl group, all of which groups may optionally be substituted. Preferably, R¹⁰ is cycloalkyl containing one or two rings and 3 to 10 carbon atoms; alkylcycloalkyl containing one or two rings and 4 to 16 carbon atoms; heterocycloalkyl containing one or two rings and 3 to 10 ring atoms selected from C, O, S and N; heteroalkylcycloalkyl containing one or two rings and 4 to 16 atoms selected from C, O, S and N; phenyl; naphthyl; heteroaryl containing one or two rings and 5 to 10 ring atoms selected from C, O, S and N; aralkyl containing one or two rings and 7 to 16 carbon atoms; or heteroaralkyl containing one or two rings and 6 to 16 atoms selected from C, O, S and N; wherein all these groups may optionally be substituted by halogen, OH, NO₂, OMe, Me, =O, NH₂, NHMe or NMe₂ or a group of formula -O-CH₂CH₂-O- or -O-CH₂-O-.

More preferably, R¹⁰ is phenyl, naphthyl or a heteroaryl group containing one or two rings and 5 to 10 ring atoms selected from C, O, S and N, wherein all these groups may optionally be substituted by halogen, OH, NO₂, OMe, Me, NH₂, NHMe or NMe₂ or a group of formula-O-CH₂CH₂-O- or -O-CH₂-O-.

Especially preferably, R¹⁰ is phenyl or a heteroaryl group containing 5 or 6 ring atoms selected from C, O, S and N, wherein all these groups may optionally be substituted by halogen, OH, NO₂, OMe, Me, NH₂, NHMe or NMe₂ or a group of formula -O-CH₂CH₂-O- or -O-CH₂-O-.

Most preferably, R¹⁰ is a phenyl group which may optionally be substituted by halogen, OH, NO₂, OMe, Me, NH₂, NHMe or NMe₂ or a group of formula -O-CH₂CH₂-O- or -O-CH₂-O-.

Especially preferably, R¹ is selected from the following groups:

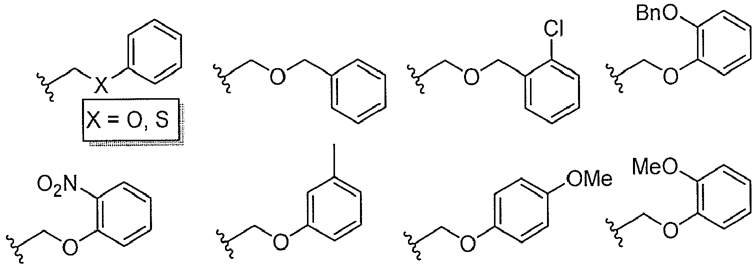

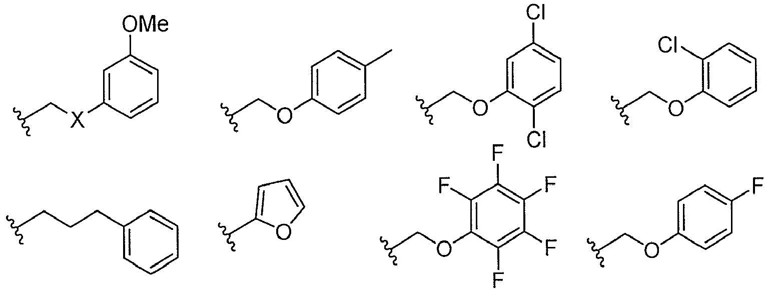

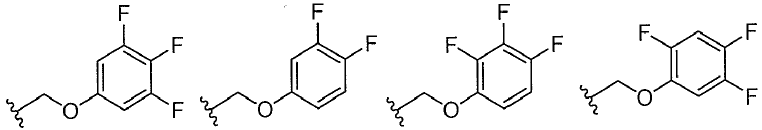

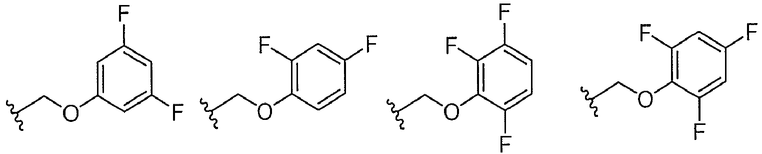

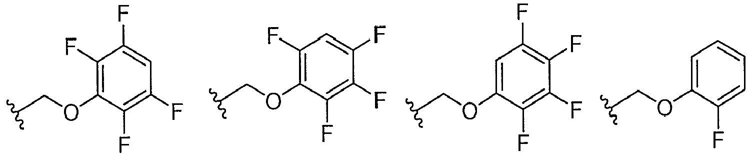

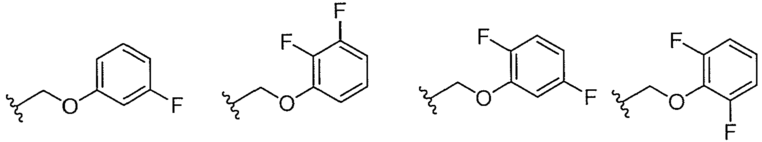

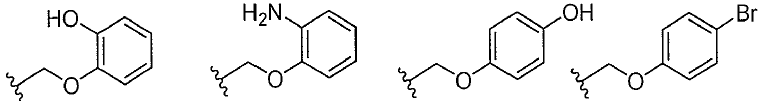

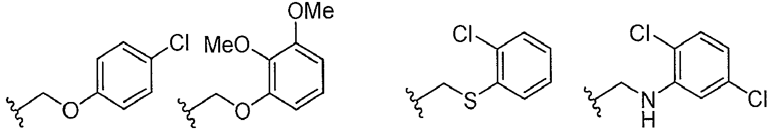

Further preferably, R² is Ci-6 alkyl; C₂₋₆ alkenyl; C₁₋₆ alkynyl; heteroalkyl containing 1 to 6 carbon atoms and 1 to 4 heteroatoms selected fom O, S and N; C₃₋₇ cycloalkyl; C₄₋₁₀ alkylcycloalkyl; heterocycloalkyl containing 3 to 7 ring atoms selected from C, O, S and N; heteroalkylcycloalkyl containing 4 to 10 atoms selected from C, O, S and N; phenyl; heteroaryl containing 5 or 6 ring atoms selected from C, O, S and N; aralkyl containing 7 to 12 carbon atoms; or heteroaralkyl containing 6 to 12 atoms selected from C, O, S and N; wherein all these groups may optionally be substituted by halogen, OH, NO₂, OMe, Me, =O, NH₂, NHMe or NMe₂.

More preferably, R² is selected from the following groups:

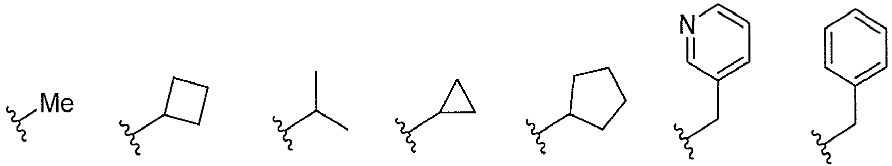

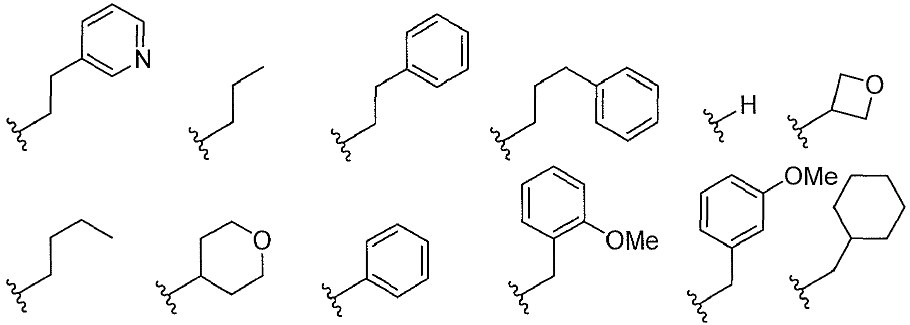

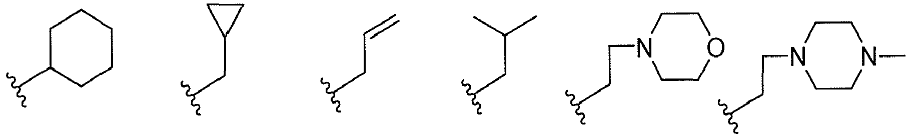

Especially preferably, R² is methyl.

Moreover preferably, R³ is hydrogen.

Further preferably, R⁴ is hydrogen.

Moreover preferably, R⁵ is an aryl, heteroaryl, aralkyl or heteroaralkyl group, all of which groups may optionally be substituted.

Further preferably, R⁵ is a group of formula -Ar-L-Cy, wherein Ar is an arylene, heteroarylene, aralkylene or heteroaralkylene group, all of which groups may optionally be substituted; L is a bond, -O-, -NH-, -S- or a C₁₋₄ alkyl group or a heteroalkyl group containing 1 to 6 atoms selected from C, O, S and N; and Cy is a cycloalkyl, heterocycloalkyl, alkylcycloalkyl, heteroalkylcycloalkyl, aryl, heteroaryl, aralkyl or heteroaralkyl group, all of which groups may optionally be substituted.

Further preferably, Ar is an optionally substituted phenylene group or an optionally substituted heteroarylene group containing 5 or 6 ring atoms selected from C, O, S and N.

Especially preferably, Ar is an optionally substituted phenylene group.

Moreover preferably, Ar is unsubstituted or substituted by a halogen atom or a methoxy group.

Further preferably, L is -O-, -CH₂O- or -OCH₂-; especially -O-.

Moreover preferably, Cy is cycloalkyl containing one or two rings and 3 to 10 carbon atoms; heterocycloalkyl containing one or two rings and 3 to 10 ring atoms selected from C, O, S and N; phenyl; naphthyl; or heteroaryl containing one or two rings and 5 to 10 ring atoms selected from C, O, S and N; all of which groups may optionally be substituted.

More preferably, Cy is phenyl, naphthyl or heteroaryl containing one or two rings and 5 to 10 ring atoms selected from C, O, S and N; all of which groups may optionally be substituted.

Further preferably, Cy is phenyl or heteroaryl containing 5 or 6 ring atoms selected from C, O, S and N; all of which groups may optionally be substituted.

Most preferably, Cy is an optionally substituted phenyl group.

Further preferably, Cy is unsubstituted or substituted by halogen, O-C₁₋₄ alkyl, C₁₋₄ alkyl or a group of formula -O-CH₂CH₂-O- or -O-CH₂-O-.

More preferably, R⁵ is selected from the following groups:

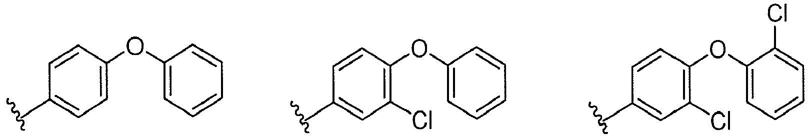

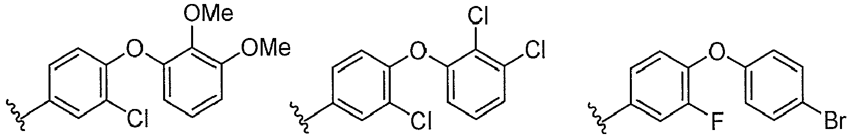

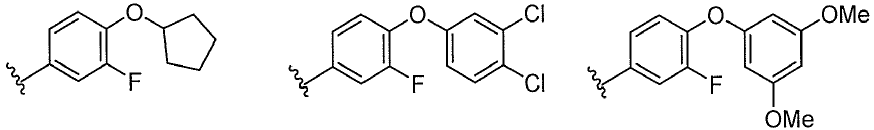

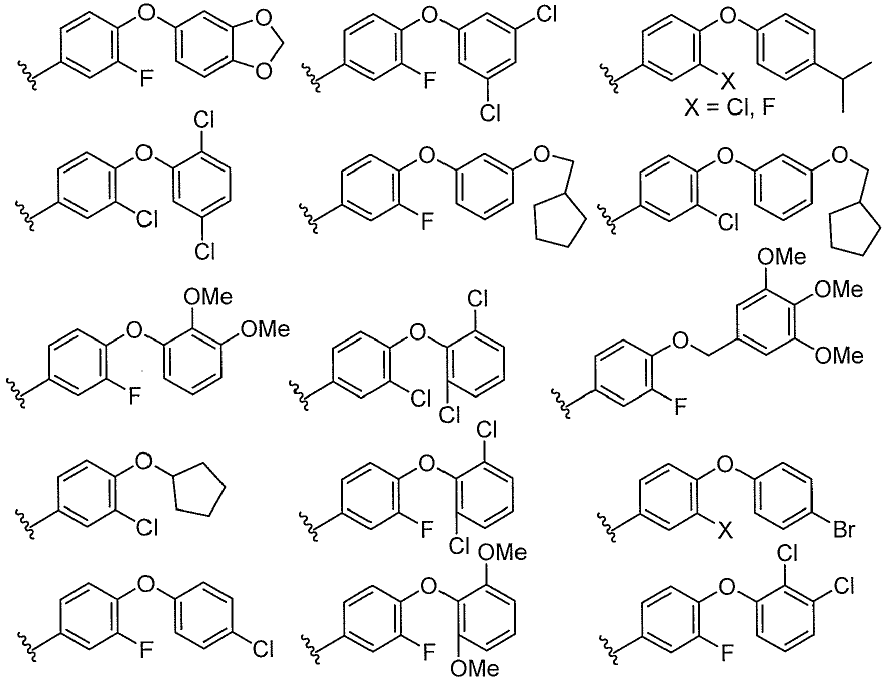

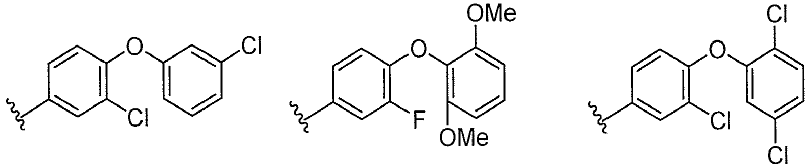

Especially preferably, the compounds of the present invention have the following formula (II): 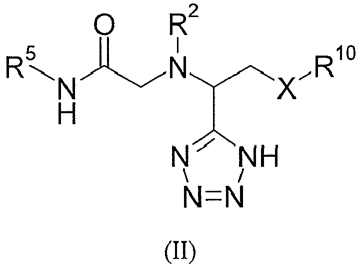 wherein X, R², R³ and R¹⁰ are as defined above.

Further preferably, the following compound is excluded from the scope of the present invention:

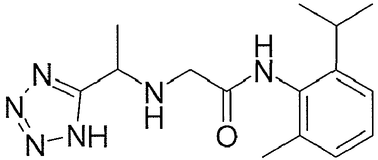

The expression alkyl refers to a saturated, straight-chain or branched hydrocarbon group that contains from 1 to 20 carbon atoms, preferably from 1 to 12 carbon atoms, especially from 1 to 6 (e.g. 1, 2, 3 or 4) carbon atoms, for example a methyl, ethyl, propyl, iso-propyl, n-butyl, iso-butyl, sec-butyl, tert-butyl, n-pentyl, iso-pentyl, n-hexyl, 2,2-dimethylbutyl or n-octyl group. Furthermore, the term alkyl refers to groups in which one or more hydrogen atoms have been replaced by a halogen atom (preferably F or Cl) such as, for example, a 2,2,2-trichloroethyl or a trifluoromethyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 (e.g. 2, 3 or 4) carbon atoms, for example an ethenyl (vinyl), propenyl (allyl), iso-propenyl, butenyl, ethinyl, propinyl, butinyl, acetylenyl, propargyl, isoprenyl or hex-2-enyl group. Preferably, alkenyl groups have one or two (especially preferably one) double bond(s), and alkynyl groups have one or two (especially preferably one) triple bond(s). Furthermore, the terms alkenyl and alkynyl refer to groups in which one or more hydrogen atoms have been replaced by a halogen atom (preferably F or Cl).

The expression heteroalkyl refers to an alkyl, alkenyl or alkynyl group in which one or more (preferably 1, 2 or 3) carbon atoms have been replaced by an oxygen, nitrogen, phosphorus, boron, selenium, silicon or sulfur atom (preferably by an oxygen, sulfur or nitrogen atom) or a SO group or a SO₂ group. The expression heteroalkyl furthermore refers to a carboxylic acid or to a group derived from a carboxylic acid, such as, for example, acyl, acylalkyl, alkoxy-carbonyl, acyloxy, acyloxyalkyl, carboxyalkylamide or alkoxycarbonyloxy.

Preferably, a heteroalkyl group contains from 1 to 12 carbon atoms and from 1 to 4 heteroatoms selected from oxygen, nitrogen and sulphur (especially oxygen and nitrogen). Especially preferably, a heteroalkyl group contains from 1 to 6 (e.g. 1, 2, 3 or 4) carbon atoms and 1, 2 or 3 (especially 1 or 2) hetero atoms selected from oxygen, nitrogen and sulphur (especially oxygen and nitrogen). The term C₁-C₆ heteroalkyl refers to a heteroalkyl group containing from 1 to 6 carbon atoms and 1, 2 or 3 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N). Furthermore, the term heteroalkyl refers to groups in which one or more hydrogen atoms have been replaced by a halogen atom (preferably F or Cl).

Examples of heteroalkyl groups are groups of formulae: R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a direct bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, n-propyloxy, isopropyloxy, butoxy, *tert*-butyloxy, methoxymethyl, ethoxymethyl, -CH₂CH₂OH, -CH₂OH, methoxyethyl, 1-methoxyethyl, 1-ethoxyethyl, 2-methoxyethyl or 2-ethoxyethyl, methyl-amino, ethylamino, propylamino, isopropylamino, dimethylamino, diethylamino, isopropyl-ethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile, isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group.

The expression heterocycloalkyl refers to a cycloalkyl group as defined above in which one or more (preferably 1, 2, 3 or 4) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) containing from 3 to 10 (especially 3, 4, 5, 6 or 7) ring atoms (preferably secected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl, urotro-pinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and also alkyl, alkenyl or alkynyl groups in accordance with the above definitions, for example alkylcyclo-alkyl, cycloalkylalkyl, alkylcycloalkenyl, alkenylcycloalkyl and alkynylcycloalkyl groups. An alkylcycloalkyl group preferably contains a cycloalkyl group that contains one or two rings having from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2, 3, 4 or 5) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings containing from 6 to 14 ring carbon atoms, preferably from 6 to 10 (especially 6) ring carbon atoms. The expression aryl refers furthermore to groups in which one or more hydrogen atoms have been replaced by fluorine, chlorine, bromine or iodine atoms or by OH, SH, NH₂, N₃ or NO₂ groups. Examples are the phenyl, naphthyl, biphenyl, 2-fluorophenyl, anilinyl, 3-nitrophenyl or 4-hydroxyphenyl group.

The expression heteroaryl refers to an aromatic group that contains one or more rings containing from 5 to 14 ring atoms, preferably from 5 to 10 (especially 5 or 6 or 9 or 10) ring atoms, and contains one or more (preferably 1, 2, 3, 4 or 5) oxygen, nitrogen, phosphorus or sulfur ring atoms (preferably O, S or N). The expression heteroaryl refers furthermore to groups in which one or more hydrogen atoms have been replaced by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl,thiadiazolyl, indolyl, indazolyl, tetrazolyl, pyrazinyl, pyrimidinyl, pyridazinyl, oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetraline, dihydronaphthalene, indanone, phenylcyclopentyl, cumene, cyclohexylphenyl, fluorene and indane. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulfur atom (preferably oxygen, sulfur or nitrogen), that is to say to groups containing both aryl or heteroaryl, respectively, and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, wherein 1, 2, 3, 4, 5 or 6 of these carbon atoms have been replaced by oxygen, sulfur or nitrogen atoms.

Examples are arylheteroalkyl, arylheterocycloalkyl, arylheterocycloalkenyl, arylalkyl-heterocycloalkyl, arylalkenylheterocycloalkyl, arylalkynylheterocycloalkyl, arylalkylhetero-cycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, hetero-arylheterocycloalkenyl, heteroarylalkylcycloalkyl, heteroarylalkylheterocycloalkenyl, hetero-arylheteroalkylcycloalkyl, heteroarylheteroalkylcycloalkenyl and heteroarylheteroalkylhetero-cycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The term halogen or halogen atom refers to F, Cl, Br or I.

The expression "optionally substituted" especially refers to groups in which one, two, three or more hydrogen atoms may have been replaced by fluorine, chlorine, bromine or iodine atoms or by OH, =O, SH, =S, NH₂, =NH, SO₃H, N₃ or NO₂ groups. This expression refers furthermore especially to groups that may be substituted by one, two, three or more preferably unsubstituted C₁-C₁₀ alkyl, C₂-C₁₀ alkenyl, C₂-C₁₀ alkynyl, C₁-C₁₀ heteroalkyl, C₃-C₁₈ cyclo-alkyl, C₂-C₁₇ heterocycloalkyl, C₄-C₂₀ alkylcycloalkyl, C₂-C₁₉ heteroalkylcycloalkyl, C₆-C₁₈ aryl, C₁-C₁₇ heteroaryl, C₇-C₂₀ aralkyl or C₂-C₁₉ heteroaralkyl groups. This expression refers furthermore especially to groups that may be substituted by one, two, three or more preferably unsubstituted C₁-C₆ alkyl, C₂-C₆ alkenyl, C₂-C₆ alkynyl, C₁-C₆ heteroalkyl, C₃-C₁₀ cycloalkyl, C₂-C₉ heterocycloalkyl, C₇-C₁₂ alkylcycloalkyl, C₂-C₁₁ heteroalkylcycloalkyl, C₆-C₁₀ aryl, C₁-C₉ heteroaryl, C₇-C₁₂ aralkyl or C₂-C₁₁ heteroaralkyl groups.

Preferred substituents are halogen, methyl, methoxy, OH, =O, NH₂, NHMe, NMe₂, NO₂, CN, CF₃, ethyl, n-propyl and isopropyl.

According to a preferred embodiment, all alkyl, alkenyl, alkynyl, heteroalkyl, aryl, heteroaryl, cycloalkyl, heterocycloalkyl, alkylcycloalkyl, heteroalkylcycloalkyl, aralkyl and heteroaralkyl groups described herein may optionally be substituted.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated.

The compounds of the present invention are cell penetrable, they do not covalently bind to the active site cysteine of caspase-1, they show reasonable water solubility and the synthesis of these compounds is short, convergent and cheap.

The present invention further provides pharmaceutical compositions comprising one or more compounds of formula (I) as defined herein or a pharmaceutically acceptable ester, prodrug, hydrate, solvate or salt thereof, optionally in combination with a pharmaceutically acceptable carrier.

It is a further object of the present invention to provide a compound of formula (I) as defined herein or a pharmaceutical composition as defined herein for the preparation of a medicament for the treatment of inflammatory diseases.

Preferably the compounds of the present invention may be used for the treatment and/or prevention of inflammatory diseases.

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage may be adjusted to the individual requirements in each particular case including the specific compound being administered, the route of administration, the condition being treated, as well as the patient being treated.

Examples of pharmacologically acceptable salts of sufficiently basic compounds of formula (I) are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound of formula (I) may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of formula (I). Compounds of formula (I) may be solvated, especially hydrated. The hydratization/hydration may occur during the process of production or as a consequence of the hygroscopic nature of the initially water free compounds of formula (I). The solvates and/or hydrates may e.g. be present in solid or liquid form.

It should be appreciated that certain compounds of formula (I) may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). All these tautomeric forms, geometrical or optical isomers (as well as racemates and diastereomers) and polymorphous forms are included in the invention. Since the compounds of formula (I) may contain asymmetric C-atoms, they may be present either as achiral compounds, mixtures of diastereomers, mixtures of enantiomers or as optically pure compounds. The present invention comprises both all pure enantiomers and all pure diastereomers, and also the mixtures thereof in any mixing ratio.

The therapeutic use of compounds according to formula (I), their pharmacologically acceptable salts, solvates and hydrates, respectively, as well as formulations and pharmaceutical compositions also lie within the scope of the present invention.

The pharmaceutical compositions according to the present invention comprise at least one compound of formula (I) as an active ingredient and, optionally, carrier substances and/or adjuvants.

The present invention also relates to pro-drugs which are composed of a compound of formula (I) and at least one pharmacologically acceptable protective group which will be cleaved off under physiological conditions, such as an alkoxy-, arylalkyloxy-, acyl-, acyloxymethyl group (e.g. pivaloyloxymethyl), an 2-alkyl-, 2-aryl- or 2-arylalkyl-oxycarbonyl-2-alkylidene ethyl group or an acyloxy group as defined herein, e.g. ethoxy, benzyloxy, acetyl or acetyloxy or, especially for a compound of formula (I), carrying a hydroxy group (-OH): a sulfate, a phosphate (-OPO₃ or -OCH₂OPO₃) or an ester of an amino acid.

Preferably, the present invention also relates to a prodrug, a biohydrolyzable ester, a biohydrolyzable amide, a polymorph, tautomer, stereoisomer, metabolite, N-oxide, biohydrolyzable carbamate, biohydrolyzable ether, physiologically functional derivative, atropisomer, or in vivo-hydrolysable precursor, diastereomer or mixture of diastereomers, chemically protected form, affinity reagent, complex, chelate and a stereoisomer of the compounds of formula (I).

As used herein, the term pharmaceutically acceptable ester especially refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates.

As mentioned above, therapeutically useful agents that contain compounds of formula (I), their solvates, salts or formulations are also comprised in the scope of the present invention. In general, compounds of formula (I) will be administered by using the known and acceptable modes known in the art, either alone or in combination with any other therapeutic agent.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 10 mg to about 10,000 mg, preferably from about 20 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

Examples for inflammatory diseases are:
Cancer, inflammatory bowel disease, arthritis, rheumatoid arthritis, autoimmune diseases, appendicitis, salpingitis, Crohn's disease, peritonitis, colitis, atherosclerosis, asthma, ulcerative colitis, cervicitis, ankylosing spondylitis, pneumonia, thrombosis, metabolic syndrome, erythema, vaginitis, vasculitis, gout, and urethritis.

### Examples

The compounds of the present invention may be synthesized using the Ugi four-component reaction (U-4CR).

As shown in Scheme 1, an Ugi reaction, using secondary amine **1,** aldehyde **2,** isocyanide **3,** and trimethylsilyl azide **4,** in methanol gave the desired condensation product **5** after stirring at room temperature for 2 days. The corresponding isocyanide-deprotected product **6** was obtained by the subsequent deprotection of Ugi-product **5.**

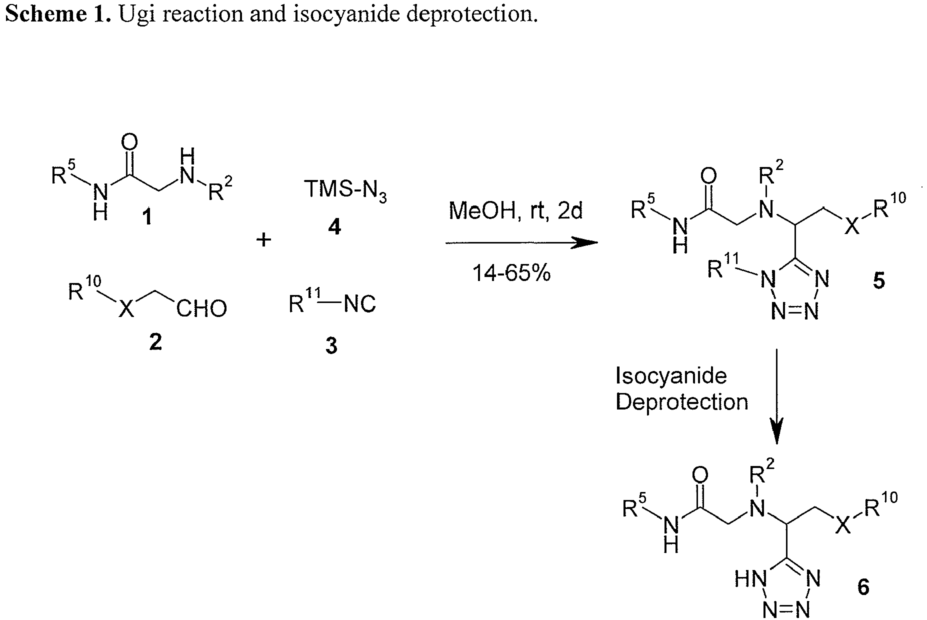

Therein, R¹¹ is the residue of a suitable cleavable isocyanide such as -CH₂CH₂CN, -C(CH₃)₃, -C(Me)2CH₂C(CH₃)₃, -benzyl, -p-MeO benzyl, -trityl or -(3,4)dimethoxy benzyl.

Secondary amines **1** were synthesized as illustrated in Scheme 2 by coupling a primary amine 7 to 2-chloroacetyl chloride **8** to give the corresponding amide **9** followed by the coupling of a primary amine **10.**

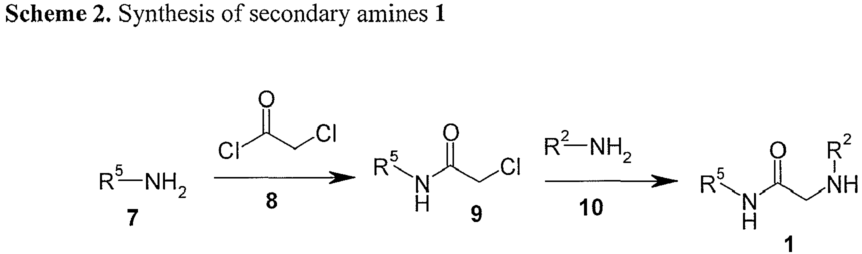

Some primary amines **7a** were synthesized according to scheme 3. Thereby a coupling reaction between phenol derivative **11** and 4-fluoronitrobenzene derivative **12** was carried out, followed by reduction of the formed nitro-derivative 13 by Pd/C, H₂ to give primary amine **7a.**

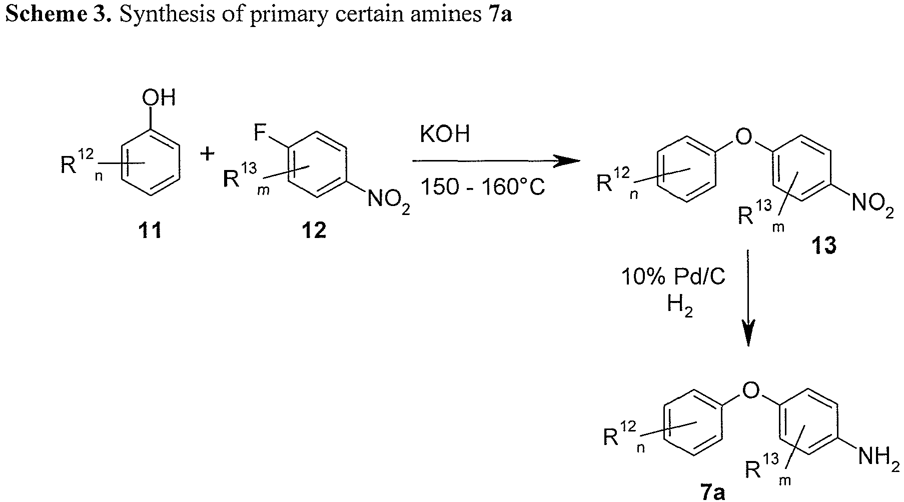

Therein n is an integer of from 0 to 5, m is an integer of from 0 to 4 and R¹¹ and R¹² are independently suitable substituents.

Aldehydes 2 were synthesized as illustrated in Scheme 4 by first coupling an alcohol, amine or thiol derivative **14** with 2-bromo-1,1-diethoxyethane **15** to give the corresponding coupled product **16** followed by hydrolysis by HCl to form aldehyde **2.**

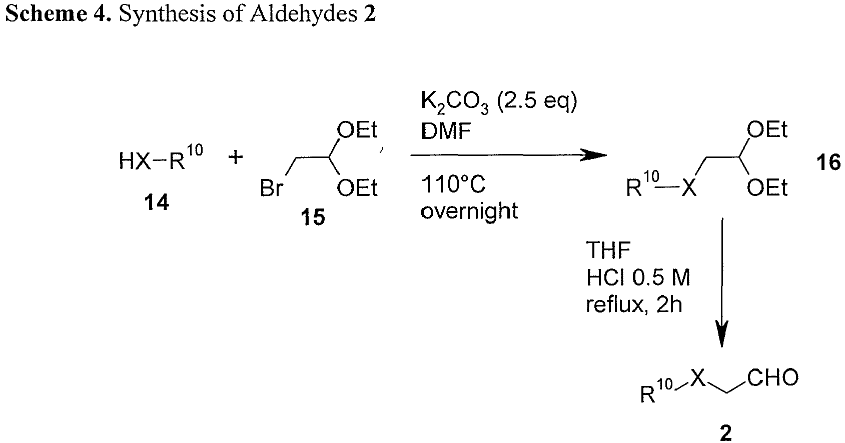

### General procedures:

### Synthesis of certain secondary amines 1a:

### Method 1: General procedure for the coupling reaction of aniline derivative 7a and 2-chloroacetyl chloride 8

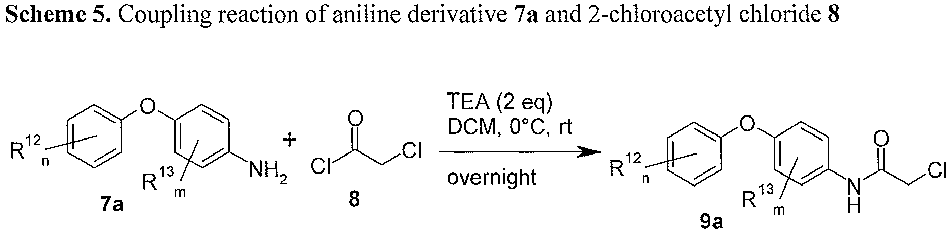

4-phenoxyaniline derivative **7a** (4 mmol) and TEA (8 mmol) were added to 60 ml DCM at 0°C in a round-bottomed flask (RBF). The 2-chloroacetyl chloride **8** (4.4 mmol, 0.35 ml) in 5 ml DCM was slowly added to the reaction mixture though a dropping funnel. The reaction mixture was stirred overnight (reaction monitored by TLC). Then, the reaction mixture was added to water and extracted with DCM. The solvent was removed under reduced pressure and the residue was purified by flash chromatography using petroleum ether:ethyl acetate on silica gel to afford product **9a.**

### Method 2: General procedure for the coupling reaction of 9a and primary amine 10

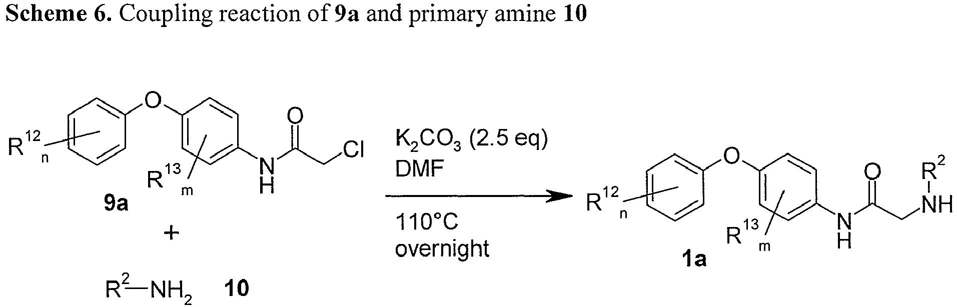

2-Chloro-N-(4-phenoxyphenyl)acetamide derivative **9a** (2.7 mmol) and primary amine **10** (4.05 mmol) were added to 10 ml DMF in a RBF. Then K₂CO₃ (931 mg, 6.75 mmol) was added to the reaction mixture, heated to 60 °C and stirred overnight (reaction monitored by TLC). Then, the reaction mixture was added to water and extracted with ethyl acetate. The solvent was removed under reduced pressure and the residue was purified by flash chromatography using petroleum ether:ethyl acetate on silica gel to afford product **1a.**

### Method 3: General procedure for the coupling reaction of phenol derivative 11 and 4-fluoronitrobenzene derivative 12

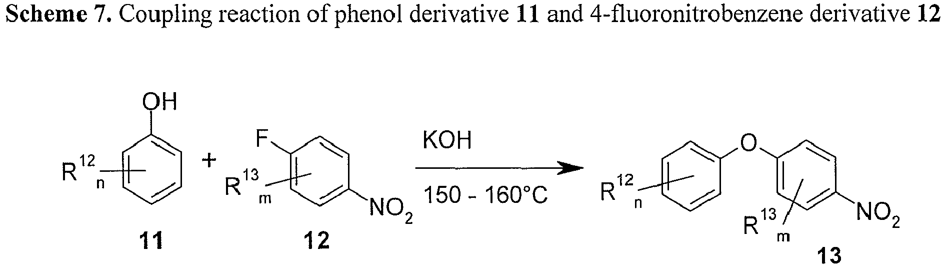

KOH (1 eq.) was dissolved in phenol derivative **11** (3eq). Then, 4-fluoronitrobenzene derivative 12 (1 eq.) was added to the solution. The mixture was heated in an oil-bath at 150-160°C for 30 min. Then, the mixture was poured into a diluted NaOH solution (3 mmol, 3 ml 1,0 M). The solid product was collected on a filter. It was recrystallized from alcohol: petroleum ether (7:3) to yield product **13.**

### Method 4: General method for the reduction of nitro-derivative 13

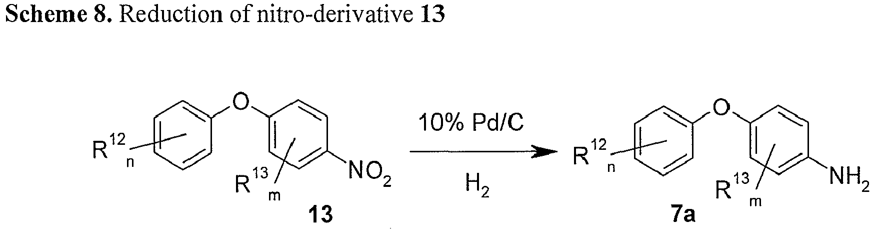

Nitro-derivative **13** (4 mmol) was dissolved in 50 ml ethyl acetate. The solution was flushed with nitrogen. 175 mg of 10% Pd/C were added to the solution. The flask was placed in an PARR apparatus and shaken thereon. The reaction was monitored by TLC (ethyl acetate: petroleum ether 30:70). Then, the reaction mixture was filtered over Celite and the solvent evaporated to yield product **7a.**

### Synthesis of aldehyde 2:

### Method 5: General procedure for the coupling reaction with 2-bromo-1,1-diethoxyethane

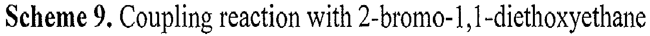

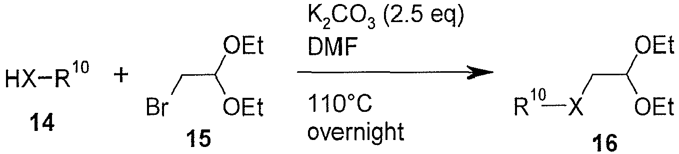

Phenol, aniline or thiol derivative **14** (5 mmol) and K₂CO₃ (12.5 mmol) were dissolved in 5 ml DMF in a RBF. 2-Bromo-1,1-diethoxyethane **15** (900 µl, 5 mmol) was slowly added to the reaction mixture. The reaction mixture was heated to 110 °C and stirred overnight (reaction monitored by TLC). Then, the reaction mixture was added to water and extracted with DCM. The solvent was removed under reduced pressure and the residue was purified by flash chromatography by using petroleum ether:ethyl acetate on silica gel to afford product **16.**

### Method 6: General procedure for the hydrolysis reaction

Reference: J. Org. Chem., 1997, 62(14), 4552-4553,

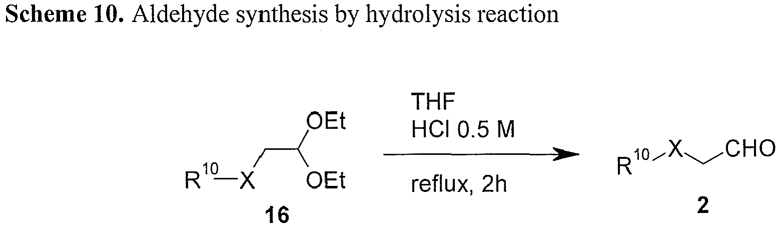

A solution of 1-(2,2-diethoxyethoxy)-phenol/aniline/thiol derivative **16** (3.4 mmol) in THF (15 mL) and HCl (0.5 M, 20 mL) was refluxed for 1-4 h. The solution was cooled and diluted with water (30 mL) and the product was extracted with ethyl acetate. The combined organic layers were were dried (MgSO₄) and the solvent was removed under reduced pressure. The crude product was purified by flash chromatography using petroleum ether:ethyl acetate on silica gel to afford product **2.**

### General procedures for the Ugi reaction and deprotection:

### Method 7: General procedure for Ugi reaction

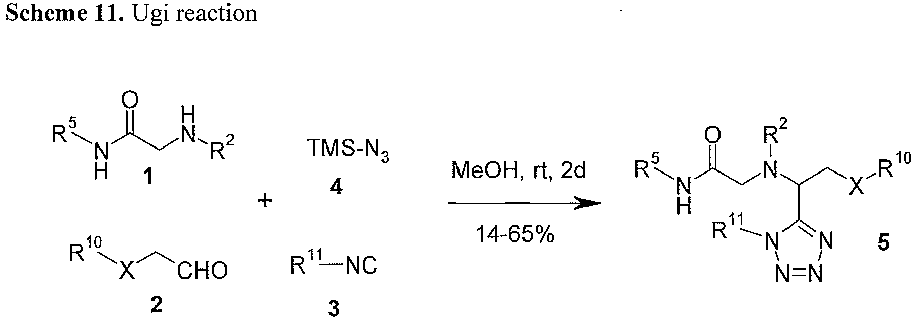

To a solution of amine **1** (1 mmol) in MeOH (1mL), aldehyde **2** (1 mmol), isocyanide **3** (1 mmol) and trimethylsilyl azide **4** (1 mmol) were added. The reaction mixture stirred at rt for 24-48h. The solvent was removed under reduced pressure followed by purification by flash chromatography on silica gel eluted with hexane-ethyl acetate to afford product **5.**

### Method 8: General procedure for isocyanide (3-isocyanopropanenitrile) deprotection from a certain Ugi-product 5a

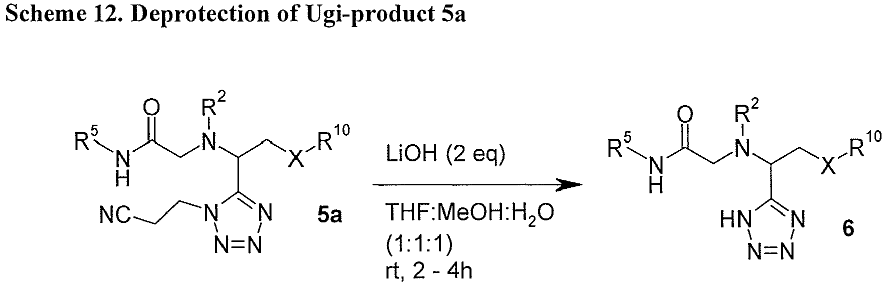

2-((2-(2-chlorophenoxy)-1-(1-(2-cyanoethyl)-1H-tetrazol-5-yl)-ethyl)(methyl)amino)-N-(4-phenoxyphenyl)acetamide (110 mg, 0.2 mmol) was added to THF:MeOH:H2O (1:1:1, 4 ml) and cooled to 0 °C in a 25 ml RBF followed by the addition of LiOH (17 mg, 0.4 mmol) to the reaction mixture and allowed to stir at rt for 2 h. Then, the solvent was removed under reduced pressure and the residue was dissolved in water and cooled in ice. Addition of HCl (1 N) gave a precipitated product. The solvent was removed under reduced pressure and the residue was purified by flash column chromatography on silica gel and eluted with methanol:DCM to afford product **6.**

### Following examples were prepared by using Method 1: Method for coupling reaction of aniline derivative and 2-chloroacetyl chloride

### Example 1: 2-chloro-N-(4-phenoxyphenyl)acetamide

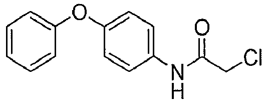

White solid, Yield: 8.5 gm (60 %); ¹H NMR (500 MHz, CDCl₃) δ 8.21 (s, 1H), 7.51 (d, *J* = 8.9, 2H), 7.38 - 7.30 (m, 2H), 7.10 (t, *J =* 7.4, 1H), 7.04 - 6.97 (m, 4H), 4.20 (s, 2H). ¹³C NMR (126 MHz, CDCl₃) δ 163.7, 157.2, 154.4, 131.9, 129.8, 123.3, 121.9, 119.5, 118.7, 42.9 ppm. (ESI) m/z calculated [M+H]⁺: 262.70; found [M+H]⁺: 262.07.

### Following examples were prepared by using Method 2: General procedure for coupling reaction of aniline derivative 9 and 2-chloroacetyl chloride

### Example 2: 2-(benzylamino)-N-(4-phenoxyphenyl)acetamide

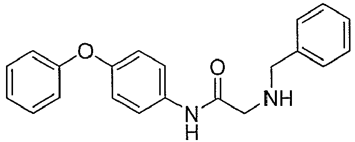

Yellow solid, Yield: 370 mg (42 %); ¹H NMR (500 MHz, CDCl₃) δ 9.24 (s, 1H), 7.52 (d, *J=* 8.9, 2H), 7.38 -7.29 (m, 7H), 7.08 (t, *J* = 7.3, 1H), 7.02 - 6.95 (m, 4H), 3.86 (s, 2H), 3.44 (s, 2H), 1.61 (s, 1H). ¹³C NMR (126 MHz, CDCl₃) δ 169.5, 153.3, 139.1, 133.2, 129.7, 128.8, 128.7, 128.1, 127.6, 127.5, 127.4, 123.0, 121.1, 119.8, 118.3, 54.1, 52.4 ppm. (ESI) m/z calculated [M+H]⁺: 333.15; found [M+H]⁺: 333.29.

### Example 3: 2-(butylamino)-N-(4-phenoxyphenyl)acetamide

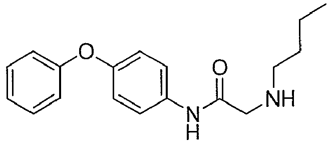

Pale yellow liquid, Yield: 447 mg (50 %); ¹H NMR (500 MHz, CDCl₃) δ 9.36 (s, 1H), 7.56 (d, *J* = 8.9, 2H), 7.34 - 7.29 (m, 2H), 7.07 (t, *J* = 7.4, 1H), 7.02 - 6.96 (m, 4H), 3.37 (s, 2H), 2.68 (t, *J* = 7.0, 2H), 1.58 (s, 1H), 1.55 - 1.47 (m, 2H), 1.46 - 1.35 (m, 2H), 0.95 (t, *J=* 7.3, 3H). ¹³C NMR (126 MHz, CDCl₃) δ 170.0, 157.7, 153.2, 133.4, 129.7, 123.0, 121.4, 121.0, 119.8, 119.7, 118.3, 53.0, 50.1, 32.3, 20.3, 14.0 ppm. MS (ESI) m/z calculated [M+H]⁺: 299.17; found [M+H]⁺: 299.31

### Example 4: 2-((cyclopropylmethyl)amino)-N-(4-phenoxyphenyl)acetamide

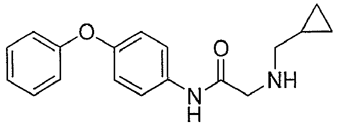

Yellow solid, Yield: 85 mg (41 %); ¹H NMR (500 MHz, CDCl₃) δ 9.38 (s, 1H), 7.57 (d, *J* = 8.9, 2H), 7.32 (t, *J* = 7.9, 2H), 7.08 (t, *J* = 7.4, 1H), 7.02 - 6.96 (m, 4H), 3.40 (d, *J* = 12.9, 2H), 2.55 (d, *J* = 6.9, 2H), 1.82 (s, 1H), 1.03 - 0.92 (m, 1H), 0.57 - 0.50 (m, 2H), 0.17 (q, *J =* 5.0, 2H). ¹³C NMR (126 MHz, CDCl₃) δ 169.9, 133.4, 129.7, 122.9, 121.0, 119.8, 118.3, 55.3, 52.7, 11.3, 3.5 ppm.

### Example 5: 2-(isopropylamino)-N-(4-phenoxyphenyl)acetamide

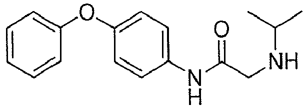

Brown liquid, Yield: 860 mg (76 %); ¹H NMR (500 MHz, CDCl₃) δ 9.43 (s, 1H), 7.56 (d, *J* = 8.9, 2H), 7.31 (t, *J* = 7.9, 2H), 7.07 (t, *J* = 7.3, 1H), 7.03 - 6.94 (m, 4H), 3.38 (s, 2H), 2.94 - 2.80 (m, 1H), 1.69 (s, 2H), 1.12 (d, *J=* 6.3, 6H). ¹³C NMR (126 MHz, CDCl₃) δ 170.4, 157.7, 153.16, 133.4, 129.7, 122.9, 121.0, 119.8, 118.3, 50.8, 49.9, 23.1 ppm. MS (ESI) m/z calculated [M+H]⁺: 285.15; found [M+H]⁺: 285.27.

### Example 6: 2-(phenethylamino)-N-(4-phenoxyphenyl)acetamide

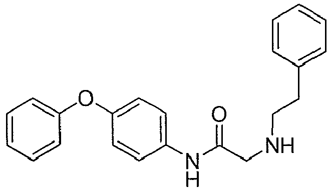

Yellow liquid, Yield: 511 mg (51 %); ¹H NMR (500 MHz, CDCl₃) δ 9.08 (s, 1H), 7.38 - 7.29 (m, 6H), 7.26 - 7.21 (m, 3H), 7.07 (t, *J* = 7.4, 1H), 7.00 - 6.93 (m, 4H), 3.36 (s, 2H), 2.97 (t, *J* = 6.5, 2H), 2.82 (t, *J* = 6.5, 2H), 1.60 (s, 1H). ¹³C NMR (126 MHz, CDCl₃) δ 169.6, 153.1, 139.5, 133.3, 129.7, 128.8, 128.7, 126.5, 122.9, 121.0, 119.7, 118.3, 52.6, 51.2, 36.4 ppm. MS (ESI) m/z calculated [M+H]⁺: 347.17; found [M+H]⁺: 347.33.

### Example 7: N-(4-phenoxyphenyl)-2-((pyridin-3-ylmethyl)amino)acetamide

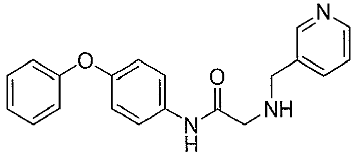

Brown liquid, Yield: 409 mg (50 %); ¹H NMR (500 MHz, CDCl₃) δ 9.15 (s, 1H), 8.61 (d, *J* = 1.9, 1H), 8.57 - 8.52 (m, 1H), 7.69 - 7.65 (m, 1H), 7.52 (d, *J=* 8.9, 2H), 7.34 - 7.28 (m, 3H), 7.08 (t, *J* = 7.4, 1H), 7.00 - 6.95 (m, 4H), 3.87 (s, 2H), 3.43 (s, 2H), 1.98 (s, 1H). ¹³C NMR (126 MHz, CDCl₃) δ 169.1, 157.6, 153.4, 149.6, 149.0, 148.3, 135.8, 134.9, 134.5, 133.1, 129.7, 123.6, 123.1, 121.2, 119.7, 118.4, 52.4, 51.4 ppm. MS (ESI) m/z calculated [M+H]⁺: 334.15; found [M+H]⁺: 334.26

### Example 8: 2-(cyclobutylamino)-N-(4-phenoxyphenyl)acetamide

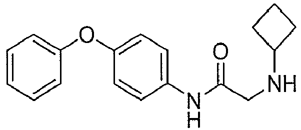

White liquid, Yield: 379 mg (43 %); ¹H NMR (500 MHz, CDCl₃) δ 9.26 (s, 1H), 7.55 (d, *J* = 8.9, 2H), 7.32 (t, *J* = 7.9, 2H), 7.07 (t, *J* = 7.4, 1H), 7.03 - 6.95 (m, 4H), 3.36 - 3.24 (m, 3H), 2.33 - 2.20 (m, 2H), 1.81 - 1.69 (m, 3H), 1.68 - 1.56 (m, 2H). ¹³C NMR (126 MHz, CDCl₃) δ 169.9, 157.7, 153.2, 133.3, 129.7, 123.0, 121.5, 121.1, 119.8, 119.7, 118.4, 118.3, 54.2, 49.99, 30.8, 14.5 ppm.

### Example 9: 2-((2-methoxybenzyl)amino)-N-(4-phenoxyphenyl)acetamide

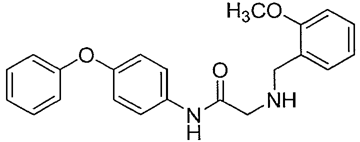

Brown liquid, Yield: 409 mg (50 %); ¹H NMR (500 MHz, CDCl₃) δ 8.94 (s, 1H), 7.46 (d, *J* = 8.9, 2H), 7.32 (M, 2H), 7.09 (t, *J* = 7.4, 1H), 7.01 - 6.95 (m, 5H), 3.91 (s, 1H), 3.86 (s, 2H), 3.44 (s, 2H). ¹³C NMR (126 MHz, CDCl₃) δ 168.5, 157.9, 157.4, 153.8, 132.9, 132.1, 130.0, 129.8, 123.2, 122.0, 121.4, 119.6, 118.6, 111.4, 60.4, 59.4, 56.2 ppm.

### Example 10: N-(4-phenoxyphenyl)-2-(propylamino)acetamide

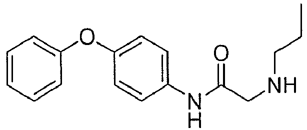

Brown liquid, Yield: 580 mg (51 %); ¹H NMR (500 MHz, CDCl₃) δ 9.42 (s, 1H), 7.57 (d, *J* = 8.9, 1H), 7.49 (d, *J* = 9.0, 1H), 7.36 - 7.29 (m, 2H), 7.13 - 7.06 (m, 1H), 7.05 - 6.93 (m, 4H), 3.45 (s, 1H), 3.41 (s, 1H), 2.67 (t, *J* = 7.1, 1H), 2.64 - 2.56 (m, 1H), 1.62 - 1.51 (m, 2H), 0.98 (t, *J* = 7.4, 3H). ¹³C NMR (126 MHz, CDCl₃) δ 169.6, 153.2, 133.3, 129.7, 123.0, 121.1, 119.7, 118.6, 118.3, 57.2, 52.6, 52.0, 23.0, 11.6 ppm; MS (ESI) m/z calculated [M+H]⁺: 285.15; found [M+H]⁺: 285.27.

### Example 11: 2-(cyclopropylamino)-N-(4-phenoxyphenyl)acetamide

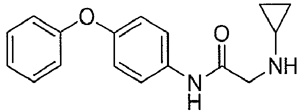

Brown liquid, Yield: 860 mg (76 %); ¹H NMR (500 MHz, CDCl₃) δ 8.98 (s, 1H), 7.55 - 7.49 (m, 2H), 7.36 - 7.28 (m, 2H), 7.07 (t, *J* = 7.4, 1H), 7.01 - 6.94 (m, 4H), 3.49 (s, 2H), 2.29 (tt, *J=* 6.7, 3.6, 1H), 2.02 (s, 1H), 0.57 - 0.50 (m, 2H), 0.46 - 0.40 (m, 2H). ¹³C NMR (126 MHz, CDCl₃) δ 170.0, 157.7, 153.3, 133.2, 129.7, 123.0, 121.1, 119.8, 119.6, 118.3, 53.2, 31.5, 6.6. MS (ESI) m/z calculated [M+H]⁺: 283.14; found [M+H]⁺: 283.25.

### Example 12: 2-(cyclopropylamino)-N-(4-phenoxyphenyl)acetamide

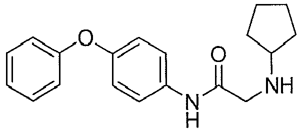

Brown liquid, Yield: 1047 mg (84 %); ¹H NMR (500 MHz, CDCl₃) δ 9.41 (s, 1H), 7.56 (d, *J* = 8.9, 2H), 7.36 - 7.29 (m, 2H), 7.07 (t, *J* = 7.4, 1H), 7.03 - 6.92 (m, 4H), 3.38 (s, 2H), 3.16 (p, *J* = 6.2, 1H), 1.87 - 1.82 (m, 2H), 1.74 - 1.69 (m, 2H), 1.62 - 1.56 (m, 2H), 1.44 - 1.33 (m, 2H). ¹³C NMR (126 MHz, CDCl₃) δ 170.3, 157.7, 153.1, 133.4, 129.7, 122.9, 121.0, 119.8, 118.3, 60.6, 51.7, 33.0, 23.7 ppm; MS (ESI) m/z calculated [M+H]⁺: 311.17; found [M+H]⁺: 311.28.

### Example 13: 2-((2-morpholinoethyl)amino)-N-(4-phenoxyphenyl)acetamide

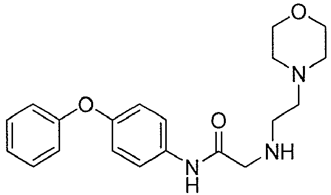

Brown liquid, Yield: 1070 mg (75 %); ¹H NMR (500 MHz, CDCl₃) δ 9.41 (s, 1H), 7.56 (d, *J* = 8.9, 2H), 7.31 (t, *J* = 7.9, 2H), 7.07 (t, *J* = 7.4, 1H), 7.01 - 6.95 (m, 4H), 3.73 - 3.68 (m, 4H), 3.39 (s, 2H), 2.83 - 2.75 (m, 2H), 2.57 - 2.48 (m, 2H), 2.46 (s, 4H), 2.16 (s, 1H). ¹³C NMR (126 MHz, CDCl₃) δ 169.9, 157.6, 153.3, 133.3, 129.7, 123.0, 121.2, 119.7, 118.3, 66.9, 58.1, 53.7, 53.0, 46.3 ppm. MS (ESI) m/z calculated [M+H]⁺: 356.19; found [M+H]⁺: 356.33.

### Example 14: N-(4-phenoxyphenyl)-2-((2-(pyridin-3-yl)ethyl)amino)acetamide

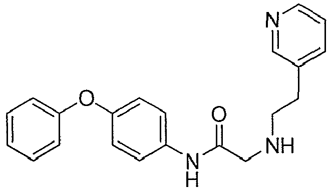

Brown liquid, Yield: 1070 mg (77 %); ¹H NMR (500 MHz, CDCl₃) δ 9.37 (s, 1H), 8.54 (d, *J* = 4.7, 1H), 7.62 (td, *J* = 7.7, 1.8, 1H), 7.48 (d, *J* = 8.9, 2H), 7.31 (t, *J* = 8.0, 2H), 7.23 - 7.14 (m, 2H), 7.07 (t, *J* = 7.4, 1H), 6.98 (d, *J* = 8.8, 4H), 3.40 (s, 2H), 3.13 (t, *J* = 6.3, 2H), 3.01 (t, *J=* 6.3, 2H), 2.17 (s, 1H). MS (ESI) m/z calculated [M+H]⁺: 348.16; found [M+H]⁺: 348.30.

### Example 15: N-(4-phenoxyphenyl)-2-((3-phenylpropyl)amino)acetamide

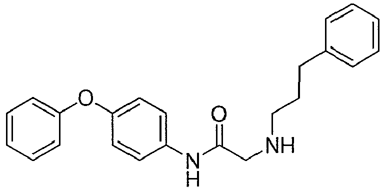

Yellow solid, Yield: 1.83 gm (86 %); ¹H NMR (500 MHz, CDCl₃) δ 9.28 (s, 1H), 7.53 (d, *J=* 8.8, 2H), 7.36 -7.27 (m, 4H), 7.20 (t, *J* = 7.7, 3H), 7.08 (t, *J* = 7.2, 1H), 6.99 (t, *J* = 8.7, 4H), 3.37 (s, 2H), 2.75 - 2.67 (m, 4H), 1.94 - 1.81 (m, 2H), 1.54 (s, 1H). ¹³C NMR (126 MHz, CDCl₃) δ 169.78, 157.70, 153.20, 141.55, 133.32, 129.71, 128.51, 128.31, 126.05, 122.97, 121.05, 119.80, 118.29, 52.95, 49.85, 33.50, 31.77 ppm. MS (ESI) m/z calculated [M+H]⁺: 361.18; found [M+H]⁺: 361.30.

### Following examples were prepared by using Method 3: General procedure for coupling reaction of phenol derivative and 4-fluoronitrobenzene derivatives

### Example 16: 2-chloro-1-(4-chlorophenoxy)-4-nitrobenzene

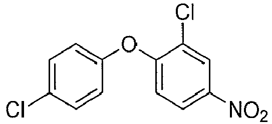

White crystals, Yield: 0,2458 gm (87%); ¹H-NMR (500 MHz, CDCl₃): δ 8.39 (s, 1H), 8.08 (d, *J* = 9.1, 1H), 7.41 (d, *J* = 8.9, 2H), 7.04 (d, *J* = 8.8, 2H), 6.91 (d, *J* = 9.1, 1H).

### Example 17: 2-chloro-1-(2-chlorophenoxy)-4-nitrobenzene

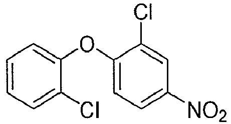

White crystals, Yield: 0,2053 gm (72 %), ¹H-NMR (500 MHz, CDCl₃): δ 8.40 (d, J = 2.6, 1H), 8.04 (dd, J = 9.1, 2.4, 1H), 7.54 (dd, J = 8.0, 1.3, 1H), 7.37 (td, J = 7.6, 1.1, 1H), 7.31 - 7.27 (m, 1H), 7.17 (dd, J = 8.0, 1.3, 1H), 6.69 (d, J = 9.1, 1H). ¹³C-NMR (125 MHz, CDCl₃): δ 158.2, 149.9, 142.8, 131.4, 128.6, 127.2, 126.7, 126.6, 124.1, 123.6, 122.6, 115.5.

### Example 18: 1-(2-chloro-4-nitrophenoxy)-2,3-dimethoxybenzene

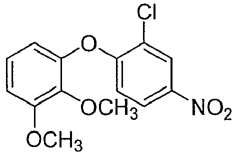

Brown viscous liquid, Yield: 0,8616 gm (70%), ¹H-NMR (500 MHz, CDCl₃): δ 8.37 (d, J = 2.6, 1H), 8.01 (dd, J = 9.1, 2.7, 1H), 7.11 (t, J = 8.3, 1H), 6.88 (dd, J = 8.4, 1.0, 1H), 6.77 - 6.73 (m, 2H), 3.91 (s, 3H), 3.81 - 3.78 (m, 3H). ¹³C-NMR (126 MHz, CDCl₃): δ 159.1, 154.4, 147.3, 142.3, 141.1, 126.3, 124.4, 123.6, 123.4, 115.4, 114.3, 110.1, 61.3, 56.2.

### Example 19: 1,2-dichloro-3-(2-chloro-4-nitrophenoxy)benzene

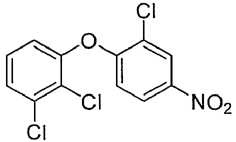

Yellow solid, Yield: 1,3448 g (99%), ¹H-NMR (500 MHz, DMSO): δ 8.53 (d, *J* = 2.7, 1H), 8.18 (dd, *J* = 9.1, 2.7, 1H), 7.67 (dd, *J* = 8.2, 1.1, 1H), 7.52 (t, *J* = 8.2, 1H), 7.38 (dd, *J* = 8.2, 1.1, 1H), 7.06 (d, J = 9.1, 1H). ¹³C-NMR (126 MHz, DMSO): δ 157.3, 151.3, 143.6, 133.8, 130.1, 128.3, 126.8, 125.1, 124.5, 123.6, 121.4, 117.7.

### Example 20: 1-(4-bromophenoxy)-2-fluoro-4-nitrobenzene

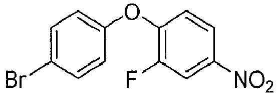

Green powder, Yield: 1,1648 gm (93%), ¹H-NMR (500 MHz, CDCl₃): δ 8.10 (dd, *J* = 10.2, 2.6, 1H), 8.01 (ddd, *J* = 9.1, 2.0, 1.3, 1H), 7.57 - 7.50 (m, 2H), 7.03 - 6.95 (m, 3H). ¹³C-NMR (125 MHz, CDCl₃): δ 153.7, 151.8, 150.1, 142.7, 133.0, 120.9, 120.6, 118.6, 117.7, 113.0.

### Example 21: 1-(cyclopentyloxy)-2-fluoro-4-nitrobenzene

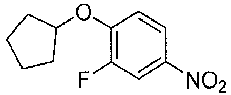

Dark brown oil, Yield: 0,6773 gm (75%), ¹H NMR (500 MHz, CDCₗ₃): δ 8.03 (d, *J* = 9.1, 1H), 7.97 (dd, *J=* 10.7, 2.6, 1H), 7.01 (t, *J* = 8.5, 1H), 4.96 - 4.88 (m, 1H), 2.03 - 1.61 (m, 8H). ¹³C-NMR (126 MHz, CDCl₃): δ 152.7, 152.2, 150.7, 120.8, 114.0, 112.3, 81.8, 32.8, 24.0.

### Example 22: 1,2-dichloro-4-(2-fluoro-4-nitrophenoxy)benzene

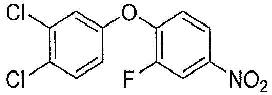

Green powder, Yield: 1,282 gm (99%), ¹H-NMR (500 MHz, DMSO): δ 8.39 (dd, J = 10.7, 2.4, 1H), 8.14 - 8.08 (m, 1H), 7.75 (d, *J* = 8.8, 1H), 7.63 (d, *J* = 2.7, 1H), 7.35 (t, *J* = 8.6, 1H), 7.26 (dd, *J* = 8.5, 2.4, 1H). ¹³C-NMR (125 MHz, DMSO): δ 154.5, 152.2, 149.7, 143.7, 132.9, 132.5, 128.0, 121.9, 121.7, 120.5, 120.1, 113.9.

### Example 23: 1-(3,5-dimethoxyphenoxy)-2-fluoro-4-nitrobenzene

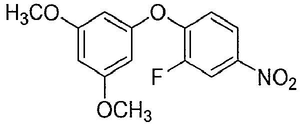

Brown solid, Yield: 0,8055 gm, (69%), ¹H-NMR (500 MHz, CDCl₃): δ 8.12 - 8.09 (m, 1H), 8.03 - 8.00 (m, 1H), 7.10 - 7.05 (m, 1H), 6.36 (t, *J* = 2.2, 1H), 6.26 (d, *J* = 2.1, 2H), 3.81 (s, 6H). ¹³C-NMR (125 MHz, CDCl₃) δ 161.9, 156.4, 152.2, 150.9, 142.8, 120.6, 118.5, 113.17, 98.1, 97.4, 55.6.

### Example 24: 5-(2-fluoro-4-nitrophenoxy)benzo[d][1,3]dioxole

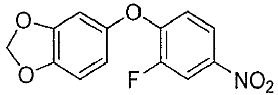

Dark brown solid, Yield: 1,4343 gm (129%), ¹H NMR (500 MHz, DMSO): δ 8.31 (dd, *J* = 10.8, 2.7, 1H), 8.08 (d, *J* = 9.1, 1H), 7.07 (t, *J* = 8.7, 1H), 7.01 (d, *J* = 8.4, 1H), 6.97 (d, *J* = 2.4, 1H), 6.70 (dd, *J* = 8.4, 2.4, 1H), 6.09 (d, *J* = 20.5, 2H). ¹³C NMR (125 MHz, DMSO): δ 151.9, 151.3, 148.9, 148.8, 145.4, 142.3, 121.8, 118.0, 113.3, 113.1, 109.1, 103.0, 102.5.

### Example 25: 1-(3,5-dichlorophenoxy)-2-fluoro-4-nitrobenzene

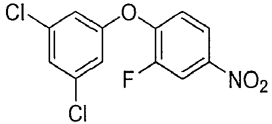

Yellow powder, Yield: 1,247 gm (99%), ¹H NMR (500 MHz, CDCl₃): δ 8.13 (dd, *J* = 10.0, 2.6, 1H), 8.10 - 8.06 (m, 1H), 7.23 (t, *J* = 1.5, 1H), 7.19 - 7.13 (m, 1H), 6.96 (d, *J* = 1.6, 2H). ¹³C NMR (126 MHz, CDCl₃): δ 156.4, 152.7, 148.9, 136.2, 125.3, 120.8, 120.8, 120.4, 117.4, 113.8.

### Example 26: 2-fluoro-1-(4-isopropylphenoxy)-4-nitrobenzene

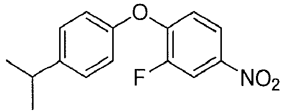

Brown oil, Yield: 0,9113 gm (83%), ¹H NMR (500 MHz, CDCl₃): δ 8.07 (dd, *J* = 10.3, 2.6, 1H), 7.98 - 7.93 (m, 1H), 7.28 (d, *J* = 8.4, 2H), 7.02 (d, *J* = 8.4, 2H), 6.94 (t, *J* = 8.5, 1H), 2.95 (dt, *J* = 13.8, 6.9, 1H), 1.27 (d, *J* = 7.0, 6H). ¹³C NMR (126 MHz, CDCl₃): δ 152.4, 152.0, 151.8, 146.3, 142.3, 128.2, 120.5, 119.7, 117.5, 113.1, 33.6, 24.0.

### Example 27: 1-(3-chlorophenoxy)-2-fluoro-4-nitrobenzene

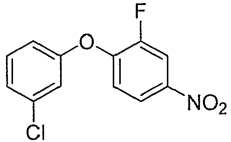

Brown powder, Yield: 1,0404 gm (97%), ¹H NMR (500 MHz, CDCl₃): δ 8.11 (dd, *J* = 10.1, 2.3, 1H), 8.03 (d, *J* = 9.0, 1H), 7.35 (t, *J* = 8.1, 1H), 7.23 (d, *J* = 8.0, 1H), 7.11 - 7.03 (m, 2H), 6.98 (dd, *J* = 8.2, 1.2, 1H). ¹³C NMR (125 MHz, CDCl₃) δ 155.7, 152.5, 150.1, 143.3, 135.6, 131.0, 125.5, 120.7, 119.6, 119.2, 117.3, 113.4.

### Example 28: 2-chloro-1-(4-isopropylphenoxy)-4-nitrobenzene

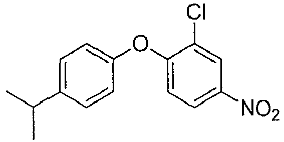

Light brown solid, Yield: 1516mg (87%); ¹H NMR (500 MHz, CDCl₃) δ ¹H NMR (500 MHz, CDCl₃) δ 8.37 (d, *J* = 2.6, 1H), 8.03 (dd, *J* = 9.1, 2.7, 1H), 7.29 (d, *J* = 8.5, 2H), 7.02 (d, *J* = 8.5, 2H), 6.86 (d, *J* = 9.1, 1H), 2.95 (dt, *J* = 13.8, 6.9, 1H), 1.28 (d, *J* = 6.9, 6H). ¹³C NMR (126 MHz, CDCl₃) δ 159.5, 152.3, 146.6, 142.4, 128.3, 126.5, 124.3, 123.6, 120.1, 116.3, 33.6, 24.1.

### Example 29: 1,4-dichloro-2-(2-chloro-4-nitrophenoxy)benzene

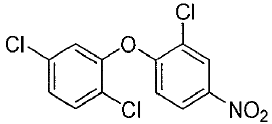

White solid, Yield: 2175mg (99%); ¹H NMR (500 MHz, CDCl₃) δ 8.41 (d, *J* = 2.6, 1H), 8.09 (dd, *J* = 9.1, 2.6, 1H), 7.47 (d, *J* = 8.6, 1H), 7.25 (dd, *J* = 8.6, 2.3, 1H), 7.14 (d, *J* = 2.3, 1H), 6.79 (d, *J* = 9.1, 1H). ¹³C NMR (126 MHz, CDCl₃) 8 157.4, 150.5, 143.4, 133.8, 131.9, 127.2, 126.8, 125.0, 124.6, 123.7, 122.4, 116.4.

### Example 30: 1-(cyclopentylmethoxy)-2-fluoro-4-nitrobenzene

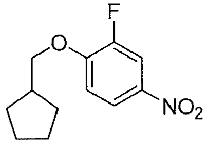

Dark brown oil, Yield: 1284mg (89 %); ¹H NMR (500 MHz, CDCl₃) δ 8.06 - 8.01 (m, 1H), 7.98 (dd, *J* = 10.7, 2.7, 1H), 7.02 (t, *J* = 8.5, 1H), 4.01 (d, *J* = 6.9, 2H), 2.49 - 2.39 (m, 1H), 1.93 - 1.84 (m, 2H), 1.70 - 1.59 (m, 4H), 1.42 - 1.33 (m, 2H). ¹³C NMR (126 MHz, CDCl₃) δ 153.2, 151.2, 140.6, 120.9, 112.9, 112.3, 73.9, 38.8, 29.4, 25.4.

### Example 31: 2-chloro-1-(cyclopentylmethoxy)-4-nitrobenzene

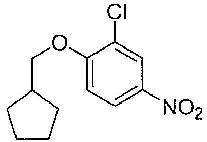

Dark brown oil, Yield: 1273mg (83%); ¹H NMR (500 MHz, CDCl₃) δ 8.29 (d, J = 2.7, 1H), 8.14 (dd, J = 9.1, 2.7, 1H), 6.97 (d, J = 9.1, 1H), 4.02 (d, J = 6.8, 2H), 2.51 - 2.40 (m, 1H), 1.89 (td, J = 12.2, 7.4, 2H), 1.73 - 1.58 (m, 4H), 1.46 - 1.37 (m, 2H). ¹³C NMR (126 MHz, CDCl₃) δ 159.9, 140.9, 126.0, 124.0, 123.5, 111.7, 73.9, 38.8, 29.4, 25.4.

### Example 32: 1-(2-fluoro-4-nitrophenoxy)-2,3-dimethoxybenzene

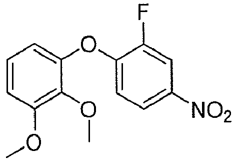

Dark brown solid, Yield: 1321mg (75%); ¹H NMR (500 MHz, CDCl₃) δ 8.08 (dd, *J* = 10.3, 2.6, 1H), 7.95 - 7.91 (m, 1H), 7.10 (t, *J* = 8.3, 1H), 6.87 (d, *J* = 8.4, 1H), 6.83 (t, *J* = 8.6, 1H), 6.74 (d, *J* = 8.3, 1H), 3.91 (s, 3H), 3.80 (s, 3H). ¹³C NMR (126 MHz, CDCl₃) δ 154.4, 151.7, 151.3, 147.4, 142.1, 141.1, 124.3, 120.5, 116.6, 113.9, 112.9, 110.0, 61.2, 56.2.

### Example 33: 5-((2-fluoro-4-nitrophenoxy)methyl)-1,2,3-trimethoxybenzene

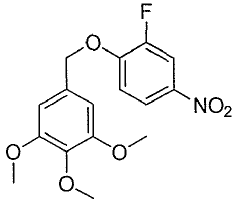

Brown/red solid, Yield: 1821mg (94%); ¹H NMR (500 MHz, CDCl₃) δ 8.06 - 8.00 (m, 2H), 7.09 (t, *J* = 8.3, 1H), 6.66 (s, 2H), 5.17 (s, 2H), 3.88 (s, 6H), 3.86 (s, 3H). ¹³C NMR (126 MHz, CDCl₃) δ 153.6, 152.3, 151.4, 141.1, 138.2, 130.5, 120.8, 113.8, 112.5, 104.6, 71.8, 60.9, 56.2.

### Example 34: 2-chloro-1-(cyclopentyloxy)-4-nitrobenzene

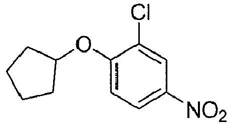

Dark brown oil, Yield: 1165mg (80%); ¹H NMR (500 MHz, CDCl₃) δ 8.28 (d, *J* = 2.7, 1H), 8.13 (dd, *J* = 9.1, 2.7, 1H), 6.97 (d, *J* = 9.1, 1H), 4.92 (tt, *J* = 5.5, 2.6, 1H), 2.04 - 1.93 (m, 4H), 1.86 (ddd, *J* = 15.8, 8.1, 2.7, 2H), 1.73 - 1.64 (m, 2H). ¹³C NMR (126 MHz, CDCl₃) δ 159.0, 140.7, 126.1, 123.9, 123.8, 112.8, 81.9, 32.8, 24.0.

### Example 35: 1,3-dichloro-2-(2-fluoro-4-nitrophenoxy)benzene

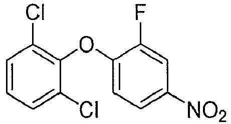

Light yellow solid, Yield: 1924mg (99%); ¹H NMR (500 MHz, CDCl₃) δ 8.13 (dd, *J=* 10.2, 2.6, 1H), 7.95 (ddd, *J* = 9.0, 2.4, 1.4, 1H), 7.46 (d, *J* = 8.1, 2H), 7.25 (t, *J* = 6.7, 1H), 6.63 (t, *J* = 8.5, 1H). ¹³C NMR (126 MHz, CDCl₃) δ 151.9, 149.8, 147.9, 142.7, 129.5, 129.3, 127.7, 120.5, 114.9, 113.3.

### Example 36: 1-(4-bromophenoxy)-2-chloro-4-nitrobenzene

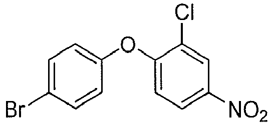

Light yellow solid, Yield: 2166mg (99%); ¹H NMR (500 MHz, CDCl₃) δ 8.38 (d, *J* = 2.7, 1H), 8.07 (dd, *J* = 9.1, 2.6, 1H), 7.58 - 7.53 (m, 2H), 7.00 - 6.94 (m, 2H), 6.91 (d, *J* = 9.1, 1H). ¹³C NMR (126 MHz, CDCl₃) δ 158.4, 153.8, 143.1, 133.4, 126.7, 125.1, 123.7, 121.7, 118.5, 117.3.

### Example 37: 2-(2-fluoro-4-nitrophenoxy)-1,3-dimethoxybenzene

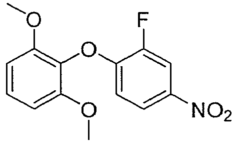

Dark brown solid, Yield: 1289 mg (99%); ¹H NMR (500 MHz, DMSO) δ 8.28 (dd, *J=* 11.0, 2.7, 1H), 8.01 (dd, *J* = 9.1, 1.5, 1H), 7.31 (t, *J* = 8.5, 1H), 6.88 (d, *J* = 8.5, 2H), 6.72 (t, *J* = 8.8, 1H), 3.76 (s, 6H). ¹³C NMR (126 MHz, DMSO) δ 152.7, 151.8, 150.6, 141.6, 129.7, 127.6, 121.8, 115.3, 113.4, 106.0, 56.6.

### Example 38: 1,2-dichloro-3-(2-fluoro-4-nitrophenoxy)benzene

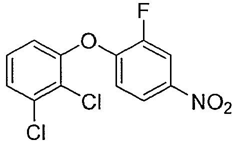

Yellow solid, Yield: 1160mg (96%), ¹H NMR (500 MHz, CDCl₃) δ 8.12 (dd, *J* = 10.1, 2.6, 1H), 8.00 (d, *J* = 9.1, 1H), 7.42 (d, *J* = 8.1, 1H), 7.31 - 7.25 (m, 1H), 7.05 (d, *J* = 8.2, 1H), 6.86 (t, *J* = 8.5, 1H). ¹³C NMR (126 MHz, CDCl₃) δ 151.3, 151.2, 149.8, 142.9, 134.6, 128.2, 127.5, 125.2, 120.6, 119.6, 117.4, 113.2.

### Example 39: 1-(4-chlorophenoxy)-2-fluoro-4-nitrobenzene

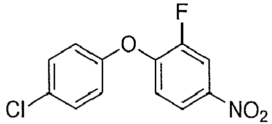

Yellow solid, Yield: 1897 mg (99%); ¹H NMR (500 MHz, CDCl₃) δ 8.10 (dd, *J* = 10.2, 2.6, 1H), 8.01 (ddd, *J* = 9.1, 2.4, 1.5, 1H), 7.42 - 7.37 (m, 2H), 7.06 - 7.01 (m, 2H), 6.99 (d, *J* = 8.2, 1H). ¹³C NMR (126 MHz, CDCl₃) δ 153.4, 152.4, 150.7, 143.0, 130.4, 129.5, 120.7, 120.6, 118.4, 113.4.

### Following examples were prepared by using Method 4: Method for reduction of nitro-derivative

### Example 40: 3-chloro-4-(3-chlorophenoxy)aniline

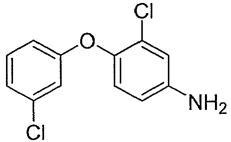

Dark brown solid, Yield: 1060 mg (73%); ¹H NMR (500 MHz, CDCl₃) δ 7.19 (t, *J* = 8.1, 1H), 7.01 - 6.97 (m, 1H), 6.93 - 6.90 (m, 1H), 6.84 (t, *J* = 2.1, 1H), 6.78 (dd, *J* = 8.6, 2.5, 2H), 6.58 (dd, *J* = 8.6, 2.7, 1H), 3.70 (s, 2H). ¹³C NMR (126 MHz, CDCl₃) δ 159.2, 144.5, 142.6, 135.0, 130.3, 127.5, 123.7, 122.3, 116.6, 116.4, 114.6, 114.4. MS (ESI) m/z calculated [M+H]⁺: 255.11; found [M+H]⁺: 254.12

### Example 41: 3-fluoro-4-(4-isopropylphenoxy)aniline

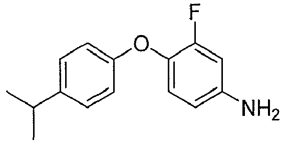

Light brown solid Yield: 635 mg (71 %); ¹H NMR (500 MHz, CDCl₃) δ 7.12 (d, *J* = 8.6, 2H), 6.90 (t, *J* = 8.8, 1H), 6.84 (d, *J* = 8.6, 2H), 6.51 (dd, *J* = 12.0, 2.6, 1H), 6.43 - 6.34 (m, 1H), 3.67 (s, 2H), 2.86 (dt, *J* = 13.8, 6.9, 1H), 1.23 (s, 3H), 1.21 (s, 3H). ¹³C NMR (126 MHz, CDCl₃) δ 156.7, 155.3, 144.2, 142.7, 135.0, 127.3, 123.8, 115.8, 110.8, 103.8, 33.3, 24.2. MS (ESI) m/z calculated [M+H]⁺: 246.30; found [M+H]⁺: 246.23.

### Example 42: 3-chloro-4-(4-isopropylphenoxy)aniline

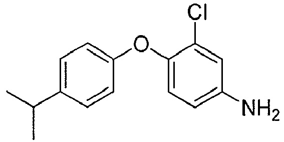

Black solid, Yield: 1242 mg (95%); ¹H NMR (500 MHz, CDCl₃) δ 7.13 (d, *J* = 8.5, 2H), 6.87 (t, *J* = 7.9, 1H), 6.81 (d, *J* = 8.5, 2H), 6.77 (s, 1H), 6.57 - 6.49 (m, 1H), 3.40 (s, 2H), 2.86 (dt, *J* = 13.8, 6.9, 1H), 1.23 (s, 3H), 1.21 (s, 3H). ¹³C NMR (126 MHz, CDCl₃) δ 156.3, 144.0, 143.7, 142.7, 127.4, 123.1, 117.3, 116.6, 116.2, 114.6, 33.4, 24.2. Mass: MS (ESI) m/z calculated [M+H]⁺: 262.75; found [M+H]⁺: 262,19

### Example 43: 4-(4-bromophenoxy)-3-fluoroaniline

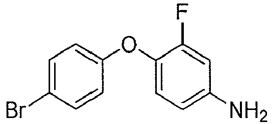

Brown solid, Yield: 838mg (80%); ¹H NMR (500 MHz, CDCl₃) δ 7.39 - 7.35 (m, 1H), 6.91 (t, *J* = 8.7, 1H), 6.80 (d, *J* = 8.9, 1H), 6.54 - 6.48 (m, 1H), 6.43 (dd, *J* = 8.6, 2.1, 1H), 3.71 (s, 2H). ¹³C NMR (126 MHz, CDCl₃) δ 157.9, 155.2, 144.8, 133.0, 132.4, 123.9, 120.0, 117.6, 110.9, 103.9. Mass: MS (ESI) m/z calculated [M+H]⁺: 283.11; found [M+H]⁺: 282.25

### Example 44: 3-chloro-4-(2,5-dichlorophenoxy)aniline

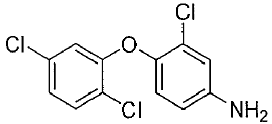

Brown solid, Yield: 1413 mg (79%); ¹H NMR (500 MHz, CDCl₃) δ 7.34 (d, *J* = 8.5, 1H), 6.97 - 6.94 (m, 1H), 6.91 (d, *J* = 8.6, 1H), 6.79 (d, J = 2.7, 1H), 6.60 (t, *J* = 2.4, 1H), 6.59 (dd, *J* = 8.6, 2.6, 1H), 3.72 (s, 2H). ¹³C NMR (126 MHz, CDCl₃) δ 154.4, 144.9, 142.2, 133.0, 131.1, 127.2, 123.4, 123.0, 121.5, 116.7, 116.4, 114.6. Mass: MS (ESI) m/z calculated [M+H]⁺: 289.06; found [M+H]⁺: 288.06

### Example 45: 4-(3,4-dichlorophenoxy)-3-fluoroaniline

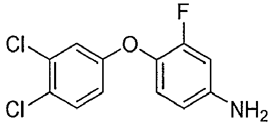

Brown solid, Yield: 619mg (57%); ¹H NMR (500 MHz, CDCl₃) δ 7.35 (d, *J* = 8.9, 1H), 7.00 (d, *J* = 2.9, 1H), 6.97 - 6.91 (m, 1H), 6.81 (dd, *J* = 8.9, 2.9, 1H), 6.54 (dd, *J* = 12.0, 2.7, 1H), 6.46 (ddd, *J* = 9.7, 5.7, 4.1, 1H), 3.77 (s, 2H). ¹³C NMR (126 MHz, CDCl₃) δ 157.8, 155.0, 145.2, 133.5, 133.0, 130.8, 125.5, 124.0, 117.6, 115.5, 111.0, 103.7. Mass: MS (ESI) m/z calculated [M-H]⁻: 271.10; found [M-H]⁻: 272.17

### Example 46: 4-(cyclopentylmethoxy)-3-fluoroaniline

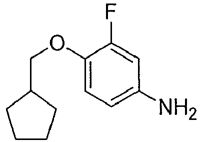

Brown liquid; Yield: 1036 mg (99%); ¹H NMR (500 MHz, CDCl₃) δ 6.79 (t, *J* = 8.9, 1H), 6.43 (dd, *J* = 12.7, 2.7, 1H), 6.33 (ddd, *J* = 8.7, 2.6, 1.2, 1H), 3.79 (d, *J* = 7.1, 2H), 3.51 (t*, J* = 23.8, 2H), 2.33 (dt, *J* = 15.0, 7.5, 1H), 1.88 - 1.75 (m, 2H), 1.67 - 1.51 (m, 4H), 1.39 - 1.29 (m, 2H). ¹³C NMR (126 MHz, CDCl₃) δ 153.8, 141.3, 139.6, 118.1, 110.4, 104.1, 75.5, 39.3, 29.4, 25.4. Mass: MS (ESI) m/z calculated [M+H]⁺: 210.26; found [M+H]⁺: 210.18

### Example 47: 3-chloro-4-(cyclopentylmethoxy)aniline

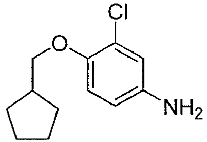

Black oil, Yield: 982 mg (87%); ¹H NMR (500 MHz, CDCl₃) δ 6.79 - 6.75 (m, 1H), 6.73 (d, *J* = 2.7, 1H), 6.52 (dd, J = 8.6, 2.8, 1H), 3.80 (d, *J* = 6.9, 2H), 3.64 - 3.22 (m, 2H), 2.37 (dt, *J* = 14.9, 7.4, 1H), 1.90 - 1.75 (m, 2H), 1.71 - 1.51 (m, 4H), 1.38 (td, *J* = 14.0, 7.1, 2H). ¹³C NMR (126 MHz, CDCl₃) δ 147.8, 140.8, 124.2, 117.2, 116.3, 114.3, 74.7, 39.2, 29.4, 25.5. Mass: MS (ESI) m/z calculated [M+H]⁺: 226,72; found [M+H]⁺: 226,14

### Example 48: 4-(3,5-dimethoxyphenoxy)-3-fluoroaniline

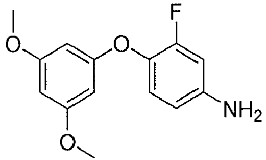

Dark brown solid, Yield: 660 mg (96%); ¹H NMR (500 MHz, CDCl₃) δ 6.92 (t, *J* = 8.7, 1H), 6.52 - 6.47 (m, 1H), 6.43 - 6.40 (m, 1H), 6.14 (t, *J* = 2.1, 1H), 6.09 (d, *J* = 2.1, 2H), 3.73 (s, 6H), 3.69 (s, 2H). ¹³C NMR (126 MHz, CDCl₃) δ 161.5, 160.7, 155.2, 144.6, 134.1, 124.1, 110.9, 103.7, 94.7, 94.4, 55.4. Mass: MS (ESI) m/z calculated [M+H]⁺: 224.27; found [M+H]⁺: 264.20

### Example 49: 4-(2,3-dimethoxyphenoxy)-3-fluoroaniline

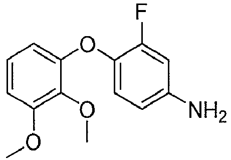

Brown solid, Yield: 1053 mg (90%); ¹H NMR (500 MHz, CDCl₃) δ ¹H NMR (500 MHz, CDCl₃) δ 6.89 (td, *J* = 8.6, 2.4, 2H), 6.63 (dd, *J* = 8.4, 1.1, 1H), 6.50 (dd, *J=* 12.1, 2.7, 1H), 6.42 - 6.34 (m, 2H), 3.93 (s, 3H), 3.88 (s, 3H), 3.63 (s, 2H). ¹³C NMR (126 MHz, CDCl₃) δ 155.8, 153.9, 152.9, 144.1, 139.0, 135.1, 123.4, 123.2, 110.7, 109.3, 106.6, 103.8, 61.1, 56.2. Mass: MS (ESI) m/z calculated [M+H]⁺: 264.27; found [M+H]⁺: 264.20

### Example 50: 4-(3-chlorophenoxy)-3-fluoroaniline

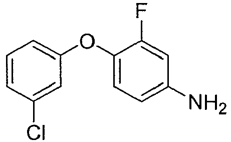

Light brown solid, Yield: 842 mg (92%); ¹H NMR (500 MHz, CDCl₃) δ 7.19 (t*, J* = 8.1, 1H), 7.01 - 6.97 (m, 1H), 6.92 (t, *J* = 8.7, 1H), 6.88 (t, *J* = 2.1, 1H), 6.81 (dd, *J* = 8.3, 2.3, 1H), 6.51 *(dd, J* = 11.9, 2.4, 1H), 6.44 (d, *J* = 8.3, 1H), 3.88 (s, 2H). ¹³C NMR (126 MHz, CDCl₃) δ δ 159.5, 155.0, 144.9, 134.9, 130.3, 124.1, 122.3, 116.1, 115.9, 114.2, 110.9, 103.9. Mass: MS (ESI) m/z calculated [M+H]⁺: 238.66; found [M+H]⁺: 238.10

### Example 51: 3-fluoro-4-((3,4,5-trimethoxybenzyl)oxy)aniline

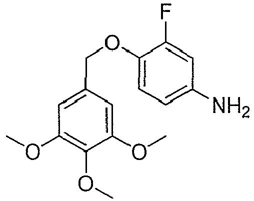

Dark brown solid, Yield: 1614 mg (99%); ¹H NMR (500 MHz, CDCl₃) δ 6.83 (t, *J* = 8.9, 1H), 6.65 (s, 2H), 6.47 (dd, *J* = 12.6, 2.7, 1H), 6.36 - 6.32 (m, 1H), 4.96 (s, 2H), 3.87 (d, *J* = 7.4, 6H), 3.84 (s, 3H), 3.54 (s, 2H). ¹³C NMR (126 MHz, CDCl₃) δ 153.8, 153.3, 141.9, 133.6, 132.8, 119.1, 110.4, 105.9, 104.6, 104.0, 73.5, 60.9, 56.1. Mass: MS (ESI) m/z calculated [M+H]⁺: 308.32; found [M+Na]⁺: 330.22

### Example 52: 3-chloro-4-(4-chlorophenoxy)aniline

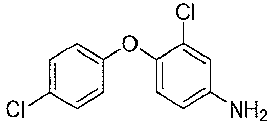

Dark brown solid, Yield: 767 mg (96%); ¹H NMR (500 MHz, CDCl₃) δ 7.25 - 7.20 (m, 2H), 6.89 (d, *J* = 8.6, 1H), 6.82 - 6.79 (m, 2H), 6.78 (d, *J* = 2.7, 1H), 6.57 (dd, *J* = 8.6, 2.7, 1H), 3.68 (s, 2H). ¹³C NMR (126 MHz, CDCl₃) δ 157.1, 144.3, 143.1, 129.5, 127.1, 123.4, 118.4, 117.4, 116.6, 114.6. Mass: MS (ESI) m/z calculated [M+H]⁺: 255.11; found [M+H]⁺: 254.12

### Example 53: 4-(cyclopentyloxy)-3-fluoroaniline

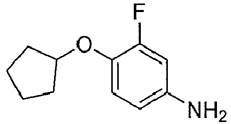

Dark brown oil, Yield: 498 mg (85%); ¹H NMR (500 MHz, CDCl₃) δ 6.79 (t, *J* = 8.9, 1H), 6.47 - 6.42 (m, 1H), 6.37 - 6.33 (m, 1H), 4.69 - 4.59 (m, 1H), 3.50 (s, 2H), 1.93 - 1.70 (m, 6H), 1.69 - 1.51 (m, 2H). ¹³C NMR (126 MHz, CDCl₃) δ 154.6, 141.3, 138.3, 119.9, 110.4, 104.0, 82.6, 32.7, 23.7. Mass: MS (ESI) m/z calculated [M+H]⁺: 196.24; found [M+H]⁺: 196.23

### Example 54: 3-chloro-4-(cyclopcntyloxy)aniline

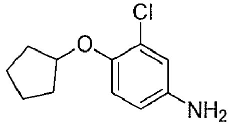

Dark brown oil, Yield: 899 mg (97 %); ¹H NMR (500 MHz, CDCl₃) δ 6.78 (d, *J=* 8.6, 1H), 6.72 *(d, J* = 2.7, 1H), 6.52 (dd, *J* = 8.6, 2.8, 1H), 4.66 (dt, *J* = 5.3, 2.5, 1H), 3.59 (s, *J* = 125.6, 12.1, 2H), 1.95 - 1.72 (m, 6H), 1.67 - 1.51 (m, 2H). ¹³C NMR (126 MHz, CDCl₃) δ 146.6, 140.8, 125.0, 118.0, 117.2, 114.3, 82.1, 32.7, 23.8 Mass: MS (ESI) m/z calculated [M+H]⁺: 212,69; found [M+H]⁺: 212,23

### Example 55: 4-(2,6-dichlorophenoxy)-3-fluoroaniline

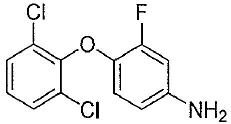

Brown solid, Yield: 1239 mg (81%); ¹H NMR (500 MHz, CDCl₃) δ 7.36 (d, *J* = 8.1, 2H), 7.11 (t*, J* = 8.1, 1H), 6.53 (dt, *J* = 11.9, 3.6, 1H), 6.39 (t, *J* = 8.9, 1H), 6.27 (dd, *J* = 8.7, 1.1, 1H), 3.57 (s, 2H). ¹³C NMR (126 MHz, CDCl₃) δ 152.3, 147.9, 142.5, 137.1, 129.2, 126.1, 123.1, 116.8, 110.2, 104.2. Mass: MS (ESI) m/z calculated [M+H]⁺: 273.10; found [M+H]⁺: 272.10, 274.12

### Example 56: 4-(3,5-dichlorophenoxy)-3-fluoroaniline

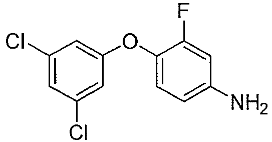

Light yellow solid, Yield: 957 mg (95%); Mass: MS (ESI) m/z calculated [M-H]⁻: 271.10; found [M-H]⁻: 271.23

### Example 57: 3-chloro-4-(2,3-dimethoxyphenoxy)aniline

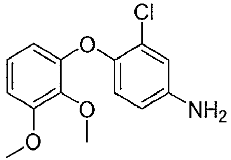

Brown solid, Yield: 575 mg (86%); ¹H NMR (500 MHz, CDCl₃) δ ¹H NMR (500 MHz, CDCl₃) δ 6.90 (t, *J* = 8.4, 1H), 6.85 (d, *J* = 8.6, 1H), 6.78 (d, *J* = 2.7, 1H), 6.65 (dd, *J* = 9.5, 8.4, 1H), 6.54 (dd, *J* = 8.6, 2.7, 1H), 6.31 (dd, *J* = 8.3, 1.0, 1H), 3.94 (s, 3H), 3.88 (s, 3H), 3.50 (s, *J* = 108.4, 35.3, 2H). ¹³C NMR (126 MHz, CDCl₃) δ 154.0, 151.6, 143.9, 143.7, 139.0, 123.4, 122.4, 120.0, 116.6, 114.5, 109.6, 106.7, 61.1, 56.1. Mass: MS (ESI) m/z calculated [M-H]⁻: 278.79; found [M-H]⁻: 278.08

### Example 58: 4-(4-bromophenoxy)-3-chloroaniline

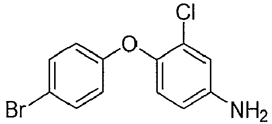

Brown solid, Yield: 1212 mg (71%); ¹H NMR (500 MHz, CDCl₃) δ 7.39 - 7.35 (m, 1H), 7.28 (dd, *J* = 8.3, 7.6, 1H), 6.93 - 6.85 (m, 2H), 6.79 (dd, *J* = 7.0, 2.6, 1H), 6.77 - 6.72 (m, 1H), 6.57 (dd, *J* = 8.6, 2.5, 1H), 3.70 (d, *J* = 72.5, 2H). ¹³C NMR (126 MHz, CDCl₃) δ 157.6, 144.3, 143.8, 132.4, 129.6, 123.4, 122.2, 117.9, 116.3, 114.7. Mass: MS (ESI) m/z calculated [M+H]⁺: 299.56; found [M+H]⁺: 298.10, 300.09.

### Example 59: 4-(2,6-dimethoxyphenoxy)-3-fluoroaniline

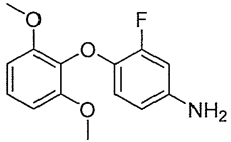

White solid, Yield: 817 mg (71%); ¹H NMR (500 MHz, CDCl₃) δ 7.10 (t, *J* = 8.4, 1H), 6.63 (d, *J* = 8.4, 2H), 6.49 (dd, *J* = 12.4, 2.6, 1H), 6.45 (t, *J* = 9.0, 1H), 6.26 - 6.20 (m, 1H), 3.78 (s, 6H), 3.48 (s, 2H). ¹³C NMR (126 MHz, CDCl₃) δ 153.5, 152.5, 141.3, 139.1, 133.2, 125.2, 116.8, 110.1, 105.5, 104.1, 56.3. Mass: MS (ESI) m/z calculated [M+H]⁺: 264.10; found [M+H]⁺: 264.20.

### Example 60: 4-(benzo[d][1,3]dioxol-5-yloxy)-3-fluoroaniline

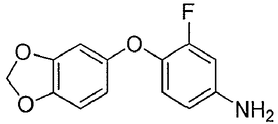

Black solid, Yield: 766 mg (66%); ¹H NMR (500 MHz, CDCl₃) δ 8.23 (ddd, *J* = 13.7, 11.9, 2.4, 1H), 8.05 - 7.79 (m, 1H), 6.99 (t, *J* = 8.6, 1H), 6.79 (dd, *J* = 8.3, 7.1, 1H), 6.63 (d, *J* = 2.4, 1H), 6.57 - 6.52 (m, 1H), 6.01 (d, *J* = 6.4, 2H), 3.95 - 3.40 (m, 1H). Mass: MS (ESI) m/z calculated [M+H]⁺: 248.23; found [M+H]⁺: 248.06, 248.21

### Example 61: 3-chloro-4-(2,3-dichlorophenoxy)aniline

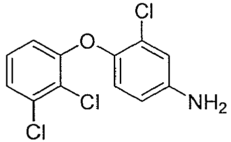

Brown solid, Yield: 750 mg (87%); ¹H NMR (500 MHz, CDCl₃) δ 7.14 (dd, *J* = 8.1, 1.2, 1H), 7.06 - 7.02 (m, 1H), 6.90 - 6.87 (m, 1H), 6.78 (d, *J* = 2.7, 1H), 6.59 - 6.54 (m, 2H), 3.71 (s, 2H). ¹³C NMR (126 MHz, CDCl₃) δ 155.3, 144.7, 142.7, 134.1, 127.2, 123.9, 123.2, 122.2, 120.8, 116.6, 114.5, 114.2. Mass: MS (ESI) m/z calculated [M+H]⁺: 289.55; found [M+H]⁺: 288.03

### Example 62: 4-(2,3-dichlorophenoxy)-3-fluoroaniline

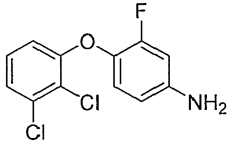

Dark brown solid, Yield: 883 mg (85%); ¹H NMR (500 MHz, CDCl₃) δ 7.14 (d, *J=* 8.1, 1H), 7.05 (t*, J* = 8.2, 1H), 6.91 (t*, J* = 8.7, 1H), 6.64 (d, *J* = 8.3, 1H), 6.51 (dd, *J* = 12.0, 2.2, 1H), 6.43 (d, *J* = 8.5, 1H), 3.74 (s, 2H). ¹³C NMR (126 MHz, CDCl₃) δ 155.8, 154.7, 145.1, 133.8, 130.6, 127.2, 123.8, 123.6, 116.2, 113.8, 110.9, 103.7. Mass: MS (ESI) m/z calculated [M+H]⁺: 272,00; found [M+H]⁺: 272,03.

### Example 63: 3-chloro-4-(3-chlorophenoxy)aniline

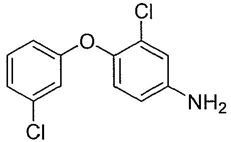

Dark brown solid, Yield: 1060 mg (73%); ¹H NMR (500 MHz, CDCl₃) δ 7.19 (t*, J* = 8.1, 1H), 7.01 - 6.97 (m, 1H), 6.93 - 6.90 (m, 1H), 6.84 (t, *J* = 2.1, 1H), 6.78 (dd, *J* = 8.6, 2.5, 2H), 6.58 (dd, *J* = 8.6, 2.7, 1H), 3.70 (s, 2H). ¹³C NMR (126 MHz, CDCl₃) δ 159.2, 144.5, 142.6, 135.0, 130.3, 127.5, 123.7, 122.3, 116.6, 116.4, 114.6, 114.4. Mass: MS (ESI) m/z calculated [M+H]⁺: 254.01; found [M+H]⁺: 254.12.

### Following examples were prepared by using Method 1: Method for coupling reaction of aniline derivative and 2-chloroacetyl chloride

### Example 64: 2-chloro-N-(3-chloro-4-(3-chlorophenoxy)phenyl)acetamide

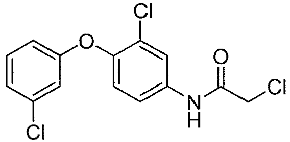

Light brown solid, Yield: 1210 mg (92%); ¹H NMR (500 MHz, CDCl₃) δ 8.26 (s, 1H), 7.80 (d, *J* = 2.6, 1H), 7.43 (dd, *J* = 8.8, 2.6, 1H), 7.24 (t, *J* = 8.2, 1H), 7.06 (t, *J* = 8.5, 2H), 6.91 (t, *J=* 2.1, 1H), 6.82 (dd, *J=* 8.3, 2.3, 1H), 4.21 (s, 2H). ¹³C NMR (126 MHz, CDCl₃) δ 163.9, 157.9, 148.5, 135.2, 133.9, 130.6, 127.0, 123.4, 122.6, 122.1, 119.9, 117.5, 115.4, 42.8. Mass: MS (ESI) m/z calculated [M-H]⁻: 329,59; found [M-H]⁻: 330.06, 329,87

### Example 65: 2-chloro-N-(3-fluoro-4-(4-isopropylphenoxy)phenyl)acetamide

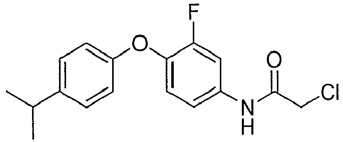

Light brown solid, Yield: 600 mg (78%); ¹H NMR (500 MHz, CDCl₃) δ 8.23 (s, 1H), 7.63 (dd, *J* = 12.0, 2.5, 1H), 7.19 - 7.16 (m, 2H), 7.16 - 7.12 (m, 1H), 7.02 (t, *J* = 8.8, 1H), 6.92 - 6.86 (m, 2H), 4.20 (s, 2H), 2.89 (hept, *J* = 6.8, 1H), 1.24 (s, 3H), 1.23 (s, 3H). ¹³C NMR (126 MHz, CDCl₃) δ 163.8, 155.2, 154.0, 143.9, 141.2, 133.0, 127.6, 121.8, 117.1, 116.0, 109.7, 42.8, 33.4, 24.1. Mass: MS (ESI) m/z calculated [M-H]⁻: 320,78; found [M-H]⁻: 320,18.

### Example 66: 2-chloro-N-(3-chloro-4-(4-isopropylphenoxy)phenyl)acetamide

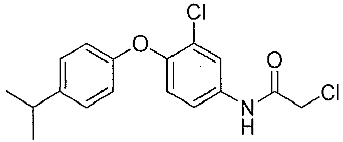

Light brown powder, Yield: 1412 mg (93%); ¹H NMR (500 MHz, CDCl₃) δ 8.23 (s, 1H), 7.75 (d, *J* = 2.6, 1H), 7.3 5 (dd, *J* = 8.8, 2.6, 1H), 7.22 - 7.14 (m, 2H), 6.96 (d, *J* = 8.8, 1H), 6.91 - 6.86 (m, 2H), 4.19 (s, 2H), 2.89 (dt, *J* = 13.8, 6.9, 1H), 1.25 - 1.23 (m, 6H). ¹³C NMR (126 MHz, CDCl₃) δ 163.9, 154.8, 150.1, 144.1, 132.8, 127.7, 126.0, 122.6, 120.7, 119.8, 117.8, 42.8, 33.5, 24.1. Mass: MS (ESI) m/z calculated [M-H]⁻: 337.23; found [M-H]⁻: 336.30, 336.50.

### Example 67: N-(4-(4-bromophenoxy)-3-fluorophenyl)-2-chloroacetamide

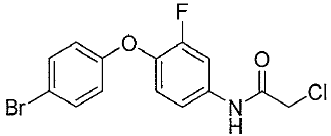

Brown solid, Yield: 823 mg (82%); ¹H NMR (500 MHz, CDCl₃) δ 8.27 (s, 1H), 7.68 - 7.63 (m, 1H), 7.44 - 7.39 (m, 2H), 7.18 (d, *J=* 8.7, 1H), 7.08 - 7.03 (m, 1H), 6.86 - 6.81 (m, 2H), 4.21 (s, 2H), 2.76 (s, 1H). ¹³C NMR (126 MHz, CDCl₃) δ 163.9, 155.7, 153.1, 140.1, 133.8, 132.6, 122.4, 118.6, 116.2, 115.6, 109.8, 42.8. Mass: MS (ESI) m/z calculated [M-H]⁻: 357.59; found [M-H]⁻: 357.82.

### Example 68: 2-chloro-N-(3-chloro-4-(2,5-dichlorophenoxy)phenyl)acetamide

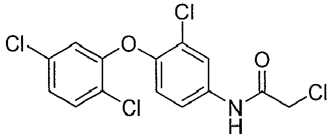

Brown solid, Yield: 977 mg (99%); ¹H NMR (500 MHz, CDCl₃) δ 8.31 (s, 1H), 7.82 (d, *J* = 2.6, 1H), 7.44 (dd, *J* = 8.8, 2.6, 1H), 7.40 - 7.37 (m, 1H), 7.06 - 7.03 (m, 1H), 6.98 (dd, *J* = 8.2, 3.6, 1H), 6.74 (d, *J* = 2.3, 1H), 4.21 (s, 2H). ¹³C NMR (126 MHz, CDCl₃) δ 164.0, 153.1, 148.1, 134.2, 133.2, 131.4, 126.4, 124.4, 122.8, 122.7, 121.2, 119.9, 118.3, 42.8. Mass: MS (ESI) m/z calculated [M-H]⁻: 365,03; found [M-H]⁻: 364,19.

### Example 69: 2-chloro-N-(4-(3,4-dichlorophenoxy)-3-fluorophenyl)acetamide

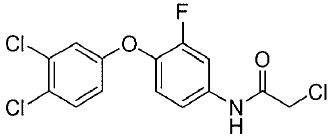

Dark red solid, Yield: 630 mg (86%); ¹H NMR (500 MHz, CDCl₃) δ 8.31 (s, 1H), 7.68 (dd, *J* = 11.9, 2.2, 1H), 7.36 (d, *J* = 8.8, 1H), 7.24 - 7.18 (m, 1H), 7.10 (t, *J* = 8.7, 1H), 7.03 (d, *J* = 2.7, 1H), 6.82 (dd, *J* = 8.8, 2.6, 1H), 4.22 (d, *J* = 7.8, 2H). ¹³C NMR (126 MHz, CDCl₃) δ 164.1, 156.6, 154.1, 139.4, 134.3, 133.3, 131.0, 126.5, 122.8, 122.8, 118.5, 110.0, 42.8, 40.5. Mass: MS (ESI) m/z calculated [M-H]⁻: 347.58; found [M-H]⁻: 347.87, 349.89.

### Example 70: 2-chloro-N-(4-(cyclopentylmethoxy)-3-fluorophenyl)acetamide

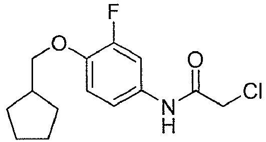

Brown solid, Yield: 619 mg (45%); ¹H NMR (500 MHz, CDCl₃) δ 8.15 (s, 1H), 7.45 (dd, *J* = 12.5, 2.6, 1H), 7.15 - 7.11 (m, 1H), 6.93 (t, *J* = 8.9, 1H), 4.18 (s, 2H), 3.89 (d, *J* = 7.0, 2H), 2.44 - 2.33 (m, 1H), 1.84 (ddd, *J* = 12.0, 9.0, 5.2, 2H), 1.68 - 1.54 (m, 4H), 1.41 - 1.31 (m, 2H). ¹³C NMR (126 MHz, CDCl₃) δ 163.7, 153.4, 147.9, 129.8, 115.9, 115.4, 109.5, 74.1, 42.8, 39.1, 29.4, 25.4. Mass: MS (ESI) m/z calculated [M-H]⁻: 285,74; found [M-H]⁻: 283,97.

### Example 71: 2-chloro-N-(3-chloro-4-(cyclopentylmethoxy)phenyl)acetamide

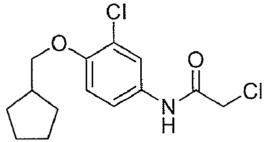

Dark brown solid, Yield: 1204 mg (99%); ¹H NMR (500 MHz, CDCl₃) δ 8.16 (s, 1H), 7.58 (d, *J* = 2.6, 1H), 7.37 (dd, *J* = 8.8, 2.6, 1H), 6.89 (d, *J* = 8.9, 1H), 4.18 (s, 2H), 3.88 (d, *J* = 6.8, 2H), 2.41 (dt, *J* = 14.8, 7.4, 1H), 1.85 (td, *J* = 11.9, 7.0, 2H), 1.72 - 1.54 (m, 4H), 1.40 (td, *J* = 14.1, 7.3, 2H). ¹³C NMR (126 MHz, CDCl₃) δ 163.9, 152.4, 129.9, 123.3, 122.8, 119.9, 113.7, 73.6, 42.8, 39.0, 29.4, 25.5. Mass: MS (ESI) m/z calculated [M-H]⁻: 301.19; found [M-H]⁻: 300.10, 300.49.

### Example 72: 2-chloro-N-(4-(3,5-dimethoxyphenoxy)-3-fluorophenyl)acetamide

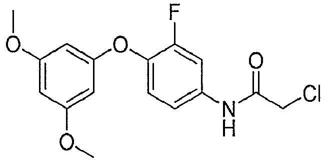

Light brown solid, Yield: 530 mg (71%); ¹H NMR (500 MHz, CDCl₃) δ 8.27 (s, 1H), 7.64 (dd, *J* = 11.9, 2.5, 1H), 7.19 -7.14 (m, 1H), 7.08 (t, *J* = 8.7, 1H), 6.20 (t, *J* = 2.2, 1H), 6.12 (d, *J* = 2.2, 2H), 4.21 (s, 2H), 3.75 (s, 6H). ¹³C NMR (126 MHz, CDCl₃) δ 163.9, 161.6, 159.3, 154.1, 140.2, 133.6, 122.6, 116.1, 109.6, 95.7, 95.3, 55.5, 42.8. Mass: MS (ESI) m/z calculated [M-H]⁻: 338.75; found [M-H]⁻: 338.06.

### Example 73: 2-chloro-N-(4-(2,3-dimethoxyphenoxy)-3-fluorophenyl)acetamide

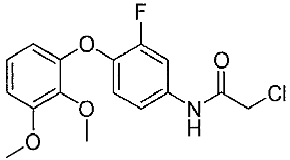

Brown solid, Yield: 1106 mg (81%); ¹H NMR (500 MHz, CDCl₃) δ 8.21 (s, 1H), 7.64 (dd, *J=* 12.1, 2.5, 1H), 7.12 -7.08 (m, 1H), 6.96 (td, *J* = 8.5, 6.0, 2H), 6.72 (dd, *J* = 8.4, 1.1, 1H), 6.51 (d, *J* = 8.4, 1H), 4.20 (s, 2H), 3.90 (s, 3H), 3.88 (s, 3H). ¹³C NMR (126 MHz, CDCl₃) δ 163.7, 154.3, 152.3, 152.1, 141.7, 132.7, 123.7, 120.6, 115.9, 111.4, 109.7, 109.5, 107.9, 61.1, 56.2, 42.8. Mass: MS (ESI) m/z calculated [M-H]⁻: 339,75; found [M-H]⁻: 338,06.

### Example 74: 2-chloro-N-(4-(3-chlorophenoxy)-3-fluorophenyl)acetamide

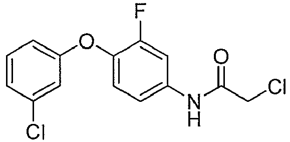

Light brown solid, Yield: 980 mg (92%); ¹H NMR (500 MHz, CDCl₃) δ 8.35 (s, 1H), 7.67 (dd, *J* = 11.9,2.5, 1H), 7.24 - 7.19 (m, 2H), 7.09 (*t, J* = 8.7, 1H), 7.07 - 7.04 (m, 1H), 6.93 (t, *J* = 2.1, 1H), 6.84 (dd, *J* = 8.3, 2.2, 1H), 4.20 (d, *J* = 2.4, 2H). ¹³C NMR (126 MHz, CDCl₃) δ 164.0, 155.7, 155.2, 139.6, 134.2, 130.5, 129.7, 123.2, 122.8, 117.1, 116.2, 114.9, 109.8, 42.8. Mass: MS (ESI) m/z calculated [M-H]⁻: 313.14; found [M-H]⁻: 313.88.

### Example 75: 2-chloro-N-(3-fluoro-4-((3,4,5-trimethoxybenzyl)oxy)phenyl)acetamide

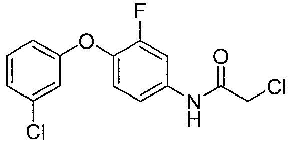

Red/brown solid, Yield: 513 mg (28%); Mass: MS (ESI) m/z calculated [M-H]⁻: 382.8; found [M-H]⁻: 382.07, 382.26.

### Example 76: 2-chloro-N-(3-chloro-4-(4-chlorophenoxy)phenyl)acetamide

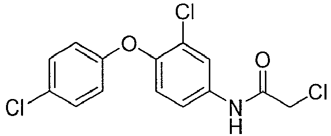

Light brown solid, Yield: 850mg (92%); ¹H NMR (500 MHz, CDCl₃) δ 8.26 (s, 1H), 7.78 (d, *J* = 2.6, 1H), 7.40 (dd, *J* = 8.8, 2.6, 1H), 7.28 (dt, *J* = 6.1, 3.6, 2H), 7.00 (dd, *J* = 9.0, 4.0, 1H), 6.89 - 6.85 (m, 2H), 4.21 (s, 2H). ¹³C NMR (126 MHz, CDCl₃) δ 163.9, 155.7, 149.0, 133.6, 129.8, 128.4, 126.7, 122.6, 121.5, 119.9, 118.7, 42.8. Mass: MS (ESI) m/z calculated [M-H]⁻: 329.59; found [M-H]⁻: 328.37.

### Example 77: 2-chloro-N-(4-(cyclopentyloxy)-3-fluorophenyl)acetamide

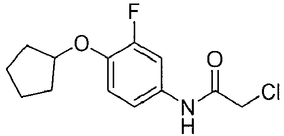

Brown/red solid, Yield: 551mg (99%); ¹H NMR (500 MHz, CDCl₃) δ 8.21 (s, 1H), 7.46 - 7.40 (m, 1H), 7.12 (d, *J* = 0.8, 1H), 6.93 (t, *J* = 8.9, 1H), 4.78 (s, 1H), 4.18 (s, 2H), 1.89 - 1.85 (m, 2H), 1.86 - 1.79 (m, 4H), 1.62 (d, *J* = 2.9, 2H). ¹³C NMR (126 MHz, CDCl₃) δ 163.8, 152.9, 143.5, 129.9, 116.9, 116.0, 109.6, 81.6, 50.8, 42.8, 32.7, 23.8. Mass: MS (ESI) m/z calculated [M-H]⁻: 270.72; found [M-H]⁻: 270.06.

### Example 78: 2-chloro-N-(3-chloro-4-(cyclopentyloxy)phenyl)acetamide

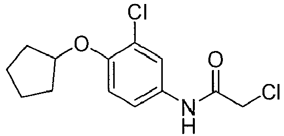

Light brown solid, Yield: 582 mg (99%); ¹H NMR (500 MHz, CDCl₃) δ 8.14 (s, 0H), 7.60 (d, *J* = 2.6, 1H), 7.39 (dd, *J* = 8.9, 2.7, 1H), 6.93 (d, *J* = 8.9, 1H), 4.81 (dd, *J* = 7.8, 3.8, 1H), 4.21 (s, 2H), 1.98 - 1.82 (m, 6H), 1.65 (td, *J* = 8.4, 5.0, 2H). ¹³C NMR (126 MHz, CDCl₃) δ 163.7, 158.2, 151.4, 129.7, 122.8, 119.8, 115.2, 81.2, 42.8, 32.8, 23.9. Mass: MS (ESI) m/z calculated [M-H]⁻: 287.17; found [M-H]⁻: 286.12, 288.07.

### Example 79: 2-chloro-N-(4-(2,6-dichlorophenoxy)-3-fluorophenyl)acetamide

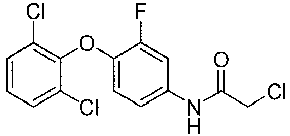

White solid, Yield: 724 mg (99%); ¹H NMR (500 MHz, CDCl₃) δ 8.18 (s, 1H), 7.62 (dd, *J* = 12.1, 2.5, 1H), 7.40 (t, *J* = 6.2, 2H), 7.17 (t, *J* = 8.1, 1H), 7.08 -7.03 (m, 1H), 6.53 (t*, J* = 8.9, 1H), 4.19 (s, 2H). ¹³C NMR (126 MHz, CDCl₃) δ 163.8, 148.7, 129.6, 129.3, 127.9, 126.7, 124.5, 121.2, 115.7, 109.8, 42.8, 30.9. Mass: MS (ESI) m/z calculated [M-H]⁻: 347,58; found [M-H]⁻: 347,87.

### Example 80: 2-chloro-N-(4-(3,5-dichlorophenoxy)-3-fluorophenyl)acetamide

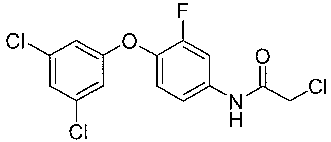

Light brown solid, Yield: 396 mg (43%); ¹H NMR (500 MHz, CDCl₃) δ 8.28 (s, 1H), 7.71 (*dd, J* = 11.9, 2.5, 1H), 7.24 (*dd, J* = 8.8, 1.3, 1H), 7.12 (t*, J* = 8.7, 1H), 7.07 (*t, J* = 1.7, 1H), 6.82 (d, *J* = 1.6, 2H), 4.22 (s, 2H). ¹³C NMR (126 MHz, CDCl₃) δ 163.9, 157.0, 153.3, 138.7, 135.7, 134.8, 123,3, 116.3, 115.3, 109.8, 100.0, 42.8. Mass: MS (ESI) m/z calculated [M-H]⁻: 347,58; found [M-H]⁻: 347,94.

### Example 81: 2-chloro-N-(3-chloro-4-(2,3-dimethoxyphenoxy)phenyl)acetamide

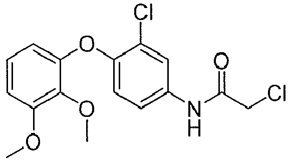

Light brown solid, Yield: 441 mg (63%), ¹H NMR (500 MHz, CDCl₃) δ 8.21 (s, 1H), 7.76 (t, *J* = 7.1, 1H), 7.30 (dd, *J* = 8.8, 2.6, 1H), 7.01 - 6.95 (m, 1H), 6.86 (d, *J=* 8.8, 1H), 6.74 (dd, *J* = 8.3, 1.0, 1H), 6.50 (dd, *J* = 8.3, 1.2, 1H), 4.19 (s, 2H), 3.89 (s, 3H), 3.88 (s, 3H). ¹³C NMR (126 MHz, CDCl₃) δ 163.8, 154.2, 150.2, 149.7, 140.2, 132.5, 123.8, 122.5, 121.9, 119.7, 119.3, 112.0, 108.2, 61.2, 56.2, 42.8. Mass: MS (ESI) m/z calculated [M-H]⁻: 355,20; found [M-H]⁻: 355,93.

### Example 82: N-(4-(4-bromophenoxy)-3-chlorophenyl)-2-chloroacetamide

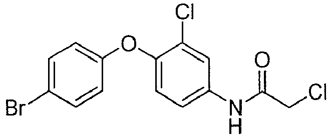

Light brown solid, Yield: 1096 mg (73%); ¹H NMR (500 MHz, CDCl₃) δ 8.25 (d, *J* = 8.4, 1H), 7.78 (dd, *J* = 6.1, 2.6, 1H), 7.42 (dd, *J* = 7.1, 5.1, 1H), 7.38 (dd, *J* = 8.9, 2.7, 1H), 7.35 - 7.30 (m, 1H), 7.00 (t, *J* = 8.5, 1H), 6.96 - 6.92 (m, 1H), 6.83 - 6.79 (m, 1H), 4.20 (s, 2H). ¹³C NMR (126 MHz, CDCl₃) δ 163.9, 156.6, 149.2, 132.7, 129.8, 123.4, 121.7, 121.3, 121.1, 119.1, 117.7, 42.8. Mass: MS (ESI) m/z calculated [M-H]⁻: 374,04; found [M-H]⁻: 374,14.

### Example 83: 2-chloro-N-(4-(2,6-dimethoxyphenoxy)-3-fluorophenyl)acetamide

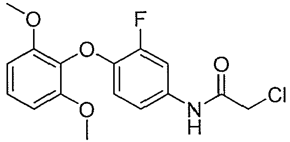

Light brown solid, Yield: 572 mg (53%); ¹H NMR (500 MHz, CDCl₃) δ 8.13 (s, 1H), 7.51 (dt, *J* = 22.9, 11.4, 1H), 7.15 (t, *J* = 8.4, 1H), 7.00 (d, *J* = 8.9, 1H), 6.65 (d, *J* = 8.5, 2H), 6.58 (t, *J* = 8.9, 1H), 4.17 (s, 2H), 3.79 (s, 6H). ¹³C NMR (126 MHz, CDCl₃) δ 163.6, 153.3, 151.6, 130.7, 125.8, 115.7, 115.6, 109.5, 105.4, 103.0, 100.0, 56.3, 42.8. Mass: MS (ESI) m/z calculated [M-H]⁻: 338,75; found [M-H]⁻: 338,12.0.

### Example 84: N-(4-(benzo[d][1,3]dioxol-5-yloxy)-3-fluorophenyl)-2-chloroacetamide

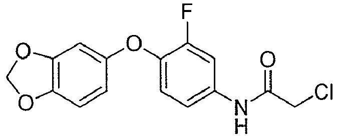

Dark brown solid, Yield: 219 mg (23%); ¹H NMR (500 MHz, CDCl₃) δ 8.22 (s, 1H), 7.61 (dd, *J* = 12.0, 2.5, 1H), 7.16 - 7.11 (m, 1H), 7.01 - 6.95 (m, 1H), 6.73 (d, *J* = 8.4, 1H), 6.56 (d, *J* = 2.5, 1H), 6.43 (dd, *J* = 8.4, 2.5, 1H), 5.97 (s, 2H), 4.20 (s, 2H). ¹³C NMR (126 MHz, CDCl₃) δ 163.8, 153.4, 152.7, 148.4, 143.7, 141.8, 132.8, 121.1, 116.0, 110.0, 109.70, 108.2, 101.6, 100.6, 42.8. Mass: MS (ESI) m/z calculated [M-H]⁻: 322,70; found [M-H]⁻: 322,00.

### Example 85: 2-chloro-N-(3-chloro-4-(2,3-dichlorophenoxy)phenyl)acetamide

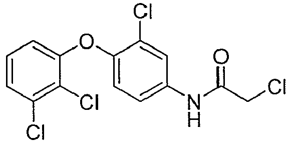

Red/brown solid, Yield: 313 mg (99%); ¹H NMR (500 MHz, CDCl₃) δ 8.24 (s, 1H), 7.80 (d, *J* = 2.5, 1H), 7.41 (dd, *J* = 8.8, 2.6, 1H), 7.25 (dd, *J* = 8.2, 1.3, 1H), 7.15 - 7.10 (m, 1H), 6.95 (d, *J* = 8.8, 1H), 6.70 (dd, *J* = 8.3, 1.2, 1H), 4.20 (d, *J* = 13.8, 2H). ¹³C NMR (126 MHz, CDCl₃) δ 170.7, 164.1, 153.8, 148.7, 133.7, 127.5, 125.3, 122.7, 122.2, 120.8, 119.9, 119.0, 116.4, 42.8. Mass: MS (ESI) m/z calculated [M-H]⁻: 364,03; found [M-H]⁻: 364,00.

### Example 86: 2-chloro-N-(4-(2,3-dichlorophenoxy)-3-fluorophenyl)acetamide

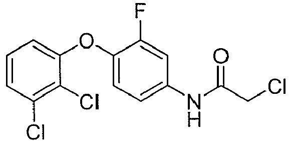

Red/brown solid, Yield: 1067 mg (99%); ¹H NMR (500 MHz, CDCl₃) δ 8.27 (s, 1H), 7.69 (dd, *J* = 11.9, 2.5, 1H), 7.23 (dd, *J* = 8.1, 1.2, 1H), 7.18 *(dd, J* = 6.1, 4.5, 1H), 7.11 (t, *J* = 8.2, 1H), 7.02 (t, J = 8.7, 1H), 6.74 (d, *J* = 8.3, 1H), 4.21 (s, 2H). ¹³C NMR (126 MHz, CDCl₃) δ 163.8, 154.4, 152.7, 134.3, 127.9, 127.4, 125.1, 124.5, 121.8, 118.4, 116.1, 115.6, 109.8, 42.8. Mass: MS (ESI) m/z calculated [M-H]⁻: 347.58; found [M-H]⁻: 346.25, 348.26.

### Example 87: 2-chloro-N-(4-(4-chlorophenoxy)-3-fluorophenyl)acetamide

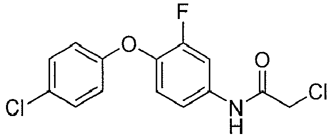

Light pink solid, Yield: 1353 mg (86%); ¹H NMR (500 MHz, CDCl₃) δ 8.26 (s, 1H), 7.68 - 7.64 (m, 1H), 7.29 - 7.25 (m, 2H), 7.20 - 7.16 (m, 1H), 7.06 (t, *J* = 8.7, 1H), 6.91 - 6.87 (m, 2H), 4.21 (s, 2H). ¹³C NMR (126 MHz, CDCl₃) δ 163.8, 155.5, 153.1, 140.3, 133.9, 129.7, 128.2, 122.3, 118.2, 116.2, 109.8, 42.8. Mass: MS (ESI) m/z calculated [M-H]⁻: 313.14; found [M-H]⁻: 312.97.

### Following examples were synthesized by using Method 2: Method for coupling reaction of primary amines 9 and 2-chloroacetyl chloride

### Example 88: N-(3-chloro-4-(3-chlorophenoxy)phenyl)-2-(methylamino)acetamide

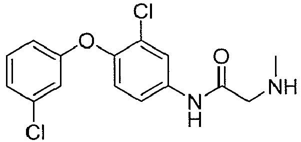

Brown oil, Yield: 878 mg (77%); ¹H NMR 500 MHz, CDCl₃) δ 9.53 (s, 1H), 7.92 (d, *J* = 2.5, 1H), 7.44 (dd, *J* = 8.8, 2.5, 1H), 7.19 (t, *J* = 8.2, 1H), 7.00 (d, *J* = 8.7, 2H), 6.88 (t, *J* = 2.1, 1H), 6.78 (dd, *J* = 8.3, 2.3, 1H), 3.34 (s, 2H), 2.46 (s, 3H), 2.19 (s, 1H). ¹³C NMR (126 MHz, CDCl₃) δ 170.2, 158.2, 147.0, 135.4, 135.0, 130.6, 126.6, 123.0, 122.3, 121.7, 119.3, 117.1, 115.1, 54.9, 36.7. Mass: MS (ESI) m/z calculated [M-H]⁻: 324.19; found [M-H]⁻: 323.24, 323,37.

### Example 89: N-(3-fluoro-4-(4-isopropylphenoxy)phenyl)-2-(methylamino)acetamide

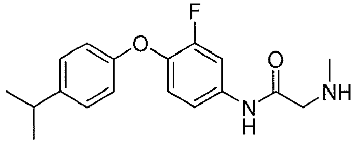

Yellow oil, Yield: 506mg (80%); ¹H NMR (500 MHz, CDCl₃) δ 9.51 (s, 1H), 7.76 (dd, *J* = 12.5, 2.3, 1H), 7.16 (d, *J* = 8.8, 1H), 7.12 (d, *J* = 8.5, 2H), 6.96 (t*, J* = 8.8, 1H), 6.86 (d, *J* = 8.5, 2H), 3.29 (d, *J* = 9.4, 2H), 2.90-2.78 (m, 1H), 2.41 (s, 3H), 2.18 (s, 1H), 1.20 (d, *J* = 7.1, 6H). ¹³C NMR (126 MHz, CDCl₃) δ 170.2, 155.6, 154.0, 143.5, 139.7, 134.8, 127.5, 122.0, 116.7, 115.5, 108.9, 54.9, 36.6, 33.3, 24.1. Mass: MS (ESI) m/z calculated [M-H]⁻:315.38; found [M-H]⁻: 315.11, 315,37.

### Example 90: N-(3-chloro-4-(4-isopropylphenoxy)phenyl)-2-(methylamino)acetamide

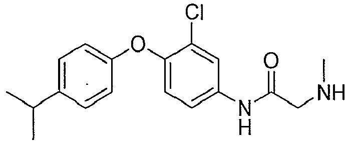

Brown oil, Yield: 538 mg (77%); ¹H NMR (500 MHz, CDCl₃) δ 9.36 (s, 1H), 7.83 (d, *J* = 2.5, 1H), 7.3 9 (dd, *J* = 8.8, 2.6, 1H), 7.17 - 7.12 (m, 2H), 6.94 (d, *J* = 8.8, 1H), 6.87 - 6.82 (m, 2H), 3.32 (s, 2H), 2.87 (dt, *J=* 16.5, 6.9, 1H), 2.46 (d, *J* = 2.8, 3H), 1.91 (s, 1H), 1.23 (dd, *J* = 6.9, 3.7, 6H). ¹³C NMR (126 MHz, CDCl₃) δ 170.0, 155.2, 148.7, 143.7, 134.3, 127.6, 126.0, 121.7, 121.1, 119.1, 117.4, 54.9, 36.8, 33.4, 24.1. Mass: MS (ESI) m/z calculated [M+H]⁺: 333.82; found [M+H]⁺: 333.31.

### Example 91: N-(4-(4-bromophenoxy)-3-fluorophenyl)-2-(methylamino)acetamide

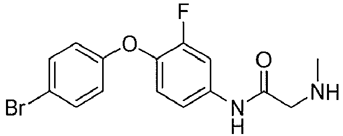

Light brown oil, Yield: 290 mg (71%), ¹H NMR (500 MHz, CDCl₃) δ 9.45 (s, 1H), 7.74 (dt, *J* = 12.4, 3.3, 1H), 7.40 -7.34 (m, 2H), 7.19 (dd, *J* = 10.7, 5.1, 1H), 7.02 (t, *J* = 8.8, 1H), 6.83 - 6.77 (m, 2H), 3.34 (s, 2H), 2.45 (d, *J* = 16.8, 3H), 2.03 (s, 1H). ¹³C NMR (126 MHz, CDCl₃) δ 170.1, 157.0, 154.1, 138.7, 135.4, 132.5, 129.7, 122.6, 118.3, 115.6, 109.0, 54.9, 36.8. Mass: MS (ESI) m/z calculated [M+H]⁺: 353.02; found [M+H]⁺: 353.14.

### Example 92: N-(3-chloro-4-(2,5-dichlorophenoxy)phenyl)-2-(methylamino)acetamide

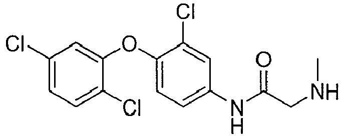

Brown oil, Yield: 717 mg (91%); ¹H NMR (500 MHz, CDCl₃) δ 9.54 (s, 1H), 7.94 (d, *J* = 2.5, 1H), 7.45 (dt, *J* = 7.5, 3.8, 1H), 7.37 - 7.32 (m, 1H), 7.02 - 6.96 (m, 2H), 6.70 (d, *J* = 2.3, 1H), 3.36 (s, 2H), 2.47 (s, 3H), 2.21 (s, 1H). ¹³C NMR (126 MHz, CDCl₃) δ 170.2, 153.3, 146.8, 135.6, 133.1, 131.3, 126.1, 124.0, 122.3, 121.8, 121.5, 119.3, 117.7, 54.9, 36.8. Mass: MS (ESI) m/z calculated [M-H]⁻: 357.00; found [M-H]⁻: 357.24.

### Example 93: N-(4-(3,4-dichlorophenoxy)-3-fluorophenyl)-2-(methylamino)acetamide

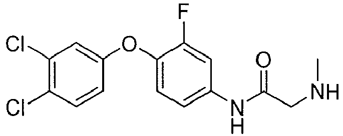

Light brown oil, Yield: 117 mg (38%); ¹H NMR (500 MHz, CDCl₃) δ 9.45 (s, 1H), 7.76 (dd, *J* = 12.3, 2.5, 1H), 7.34 (d, *J* = 8.9, 1H), 7.23 (ddd, *J* = 8.8, 2.3, 1.3, 1H), 7.06 (t, *J* = 8.7, 1H), 7.01 (d, *J* = 2.9, 1H), 6.80 (dd, *J* = 8.9, 2.9, 1H), 3.36 (s, 2H), 2.50 (s, 3H), 1.95 (d, *J* = 18.4, 1H). ¹³C NMR (126 MHz, CDCl₃) δ 170.0, 157.0, 154.2, 138.0, 135.9, 133.1, 130.9, 126.1, 122.9, 118.2, 116.0, 115.5, 109.0, 54.9, 36.9. Mass: MS (ESI) m/z calculated [M+H]⁺: 343.03; found [M+H]⁺: 343.19.

### Example 94: N-(4-(cyclopentylmethoxy)-3-fluorophenyl)-2-(methylamino)acetamide

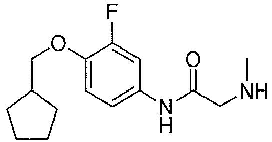

Pink solid, Yield: 354 mg (74%); ¹H NMR (500 MHz, CDCl₃) δ 9.32 (s, 1H), 7.55 (dt, *J* = 8.5, 4.2, 1H), 7.14 (dt, *J* = 7.8, 3.9, 1H), 6.92 - 6.86 (m, 1H), 3.85 (d, *J* = 7.0, 2H), 3.31 (s, 2H), 2.45 (s, 3H), 2.38 - 2.31 (m, 1H), 2.22 (s, 1H), 1.86- 1.77 (m, 2H), 1.68 - 1.52 (m, 4H), 1.39 - 1.30 (m, 2H). ¹³C NMR (126 MHz, CDCl₃) δ 169.7, 152.4, 143.6, 131.4, 115.5, 115.1, 108.6, 74.1, 54.7, 39.0, 36.5, 29.3, 25.3. Mass: MS (ESI) m/z calculated [M+H]⁺: 281.34; found [M+H]⁺: 281.31

### Example 95: N-(3-chloro-4-(cyclopentylmethoxy)phenyl)-2-(methylamino)acetamide

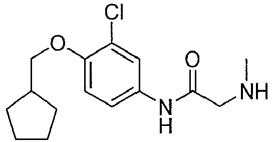

Brown oil, Yield: 193 mg (33%); ¹H NMR (500 MHz, CDCl₃) δ 9.29 (s, 1H), 7.67 (d, *J* = 2.6, 1H), 7.36 (dt, *J* = 9.7, 4.8, 1H), 6.84 (d, *J* = 8.9, 1H), 3.84 (d, *J* = 6.9, 2H), 3.29 (s, 2H), 2.44 (s, 3H), 2.41 - 2.31 (m, 1H), 2.24 (s, 1H), 1.88 - 1.76 (m, 2H), 1.71 - 1.51 (m, 4H), 1.38 (td, *J* = 14.2, 7.2, 2H). ¹³C NMR (126 MHz, CDCl₃) δ 169.8, 151.3, 131.3, 122.9, 121.8, 119.1, 113.8, 73.6, 54.8, 39.0, 36.6, 29.3, 25.4. Mass: MS (ESI) m/z calculated [M+H]⁺: 297.79; found [M+H]⁺: 297.17.

### Example 96: N-(4-(3,5-dimethoxyphenoxy)-3-fluorophenyl)-2-(methylamino)acetamide

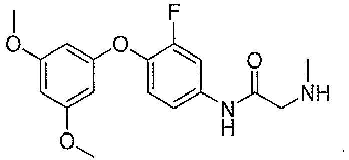

Brown oil, Yield: 193 mg (74%); ¹H NMR (500 MHz, CDCl₃) δ 9.41 (s, 1H), 7.73 (dd, *J* = 12.3, 2.4, 1H), 7.19 (dd, *J* = 5.4, 4.1, 1H), 7.06 (t, *J* = 8.8, 1H), 6.18 (t, *J* = 2.2, 1H), 6.10 (d, *J* = 2.2, 2H), 3.72 (d, *J* = 6.8, 6H), 3.34 (s, 2H), 2.48 (s, 3H), 1.97 (s, 1H). ¹³C NMR (126 MHz, CDCl₃) δ 170.0, 161.6, 159.7, 154.1, 138.8, 135.2, 122.8, 115.3, 108.9, 95.4, 95.0, 55.4, 54.9, 36.8. Mass: MS (ESI) m/z calculated [M+H]⁺: 335.34; found [M+H]⁺: 335.20.

### Example 97: N-(4-(2,3-dimethoxyphenoxy)-3-fluorophenyl)-2-(methylamino)acetamide

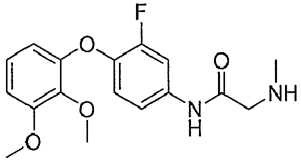

Brown oil, Yield: 400 mg (39%); ¹H NMR (500 MHz, CDCl₃) δ 9.41 (s, 1H), 7.73 (dd, *J* = 12.5, 2.4, 1H), 7.17-7.12 (m, 1H), 6.94 (td, *J* = 8.6, 5.2, 2H), 6.69 (dd, *J* = 8.4, 1.2, 1H), 6.46 (dd, *J* = 8.3, 0.7, 1H), 3.88 (s, 3H), 3.87 (s, 3H), 3.33 (s, 2H), 2.46 (s, 3H), 2.13 (s, 1H). ¹³C NMR (126 MHz, CDCl₃) δ 170.0, 154.0, 153.5, 150.6, 140.2, 139.6, 134.4, 123.7, 121.0, 115.2, 110.7, 108.7, 107.6, 61.0, 56.1, 54.8, 36.7. Mass: MS (ESI) m/z calculated [M-H]⁻: 333.35; found [M-H]⁻: 333.31.

### Example 98: N-(4-(3-chlorophenoxy)-3-fluorophenyl)-2-(methylamino)acetamide

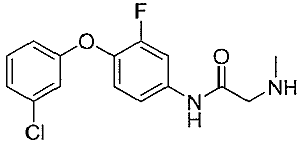

Brown oil, Yield: 336 mg (70%); ¹H NMR (500 MHz, CDCl₃) δ 9.53 (s, 1H), 7.77 (dd, *J* = 12.3, 2.3, 1H), 7.25 - 7.16 (m, 2H), 7.02 (dt, *J* = 8.5, 7.1, 2H), 6.91 (t, *J* = 2.0, 1H), 6.81 (dd, *J* = 8.3, 2.2, 1H), 3.36 (s, 2H), 2.47 (s, 3H), 2.44 - 2.35 (m, 1H). ¹³C NMR (126 MHz, CDCl₃) δ 170.0, 158.6, 154.2, 138.3, 135.6, 134.9, 130.5, 129.7, 122.9, 122.8, 116.7, 115.6, 114.7, 108.9, 54.8, 36.6. Mass: MS (ESI) m/z calculated [M+H]⁺: 309.20; found [M+H]⁺: 309.20.

### Example 99: N-(3-fluoro-4-((3,4,5-trimethoxybenzyl)oxy)phenyl)-2-(methylamino)acetamide

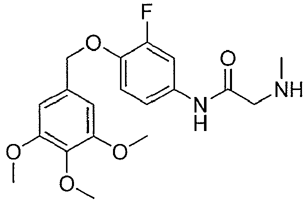

Dark brown oil, Yield: 251 mg (99%); ¹H NMR (500 MHz, CDCl₃) δ 9.24 (s, 1H), 7.56 (dd, *J* = 12.8, 2.2, 1H), 7.16 (d, *J* = 8.8, 1H), 6.95 (t, *J* = 8.9, 1H), 6.66 (s, 2H), 5.04 (s, 2H), 3.87 (s, 6H), 3.84 (s, 3H), 3.36 (s, 2H), 2.50 (s, 3H), 1.96 (d, *J* = 18.0, 1H). ¹³C NMR (126 MHz, CDCl₃) δ 169.4, 153.4, 152.8, 143.0, 137.7, 132.2, 132.1, 116.8, 115.0, 108.7, 104.5, 72.4, 60.9, 56.1, 54.8, 36.8. Mass: MS (ESI) m/z calculated [M+H]⁺: 379.39; found [M+H]⁺: 379.34.

### Example 100: N-(3-chloro-4-(4-chlorophenoxy)phenyl)-2-(methylamino)acetamide

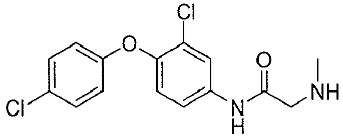

Brown oil, Yield: 345 mg (82%); ¹H NMR (500 MHz, CDCl₃) δ 9.46 (s, 1H), 7.86 (d, *J* = 2.1, 1H), 7.42 (dd, *J* = 8.7, 2.2, 1H), 7.23 (d, *J* = 8.7, 3H), 6.97 (d, *J* = 8.9, 1H), 6.81 (dd, *J* = 13.9, 8.0, 2H), 3.34 (s, 2H), 2.45 (d, *J* = 14.6, 3H), 2.22 (s, 1H). ¹³C NMR (126 MHz, CDCl₃) δ 170.2, 156.0, 147.7, 135.0, 129.7, 127.9, 126.4, 121.8, 121.7, 119.3, 118.3, 54.9, 36.8. Mass: MS (ESI) m/z calculated [M+H]⁺: 325.24; found [M+H]⁺: 325.06.

### Example 101: N-(4-(cyclopentyloxy)-3-fluorophenyl)-2-(methylamino)acetamide

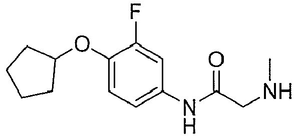

Red oil, Yield: 100 mg (38%); ¹H NMR (500 MHz, CDCl₃) δ 9.24 (s, 1H), 7.51 (dd, *J* = 12.8, 2.4, 1H), 7.15 (d, *J* = 8.7, 1H), 6.93 - 6.86 (m, 1H), 3.33 (s, 2H), 2.45 (d, *J* = 23.2, 3H), 2.16 - 2.05 (m, 1H), 1.92 - 1.76 (m, 6H), 1.67 - 1.54 (m, 2H). ¹³C NMR (126 MHz, CDCl₃) δ 169.6, 153.1, 142.5, 131.3, 117.2, 115.1, 108.6, 81.6, 54.8, 36.7, 32.7, 23.8. Mass: MS (ESI) m/z calculated [M+H]⁺: 267.32; found [M+H]⁺: 267.27.

### Example 102: N-(3-chloro-4-(cyclopentyloxy)phenyl)-2-(methylamino)acetamide

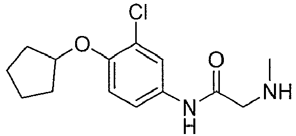

Brown oil, Yield: 292 mg (65%); ¹H NMR (500 MHz, CDCl₃) δ 9.32 (s, 1H), 7.67 (d, *J* = 2.6, 1H), 7.36 (dd, *J* = 8.8, 2.5, 1H), 6.85 (d, *J* = 8.9, 1H), 4.73 (s, 1H), 3.30 (s, 2H), 2.50 (s, 1H), 2.44 (s, 3H), 1.90 - 1.76 (m, 6H), 1.66 - 1.55 (m, 2H). ¹³C NMR (126 MHz, CDCl₃) δ 169.8, 150.2, 131.2, 123.6, 121.9, 119.0, 115.4, 81.2, 54.7, 36.6, 32.7, 23.8. Mass: MS (ESI) m/z calculated [M+H]⁺: 283,77; found [M+H]⁺: 283.06.

### Example 103: N-(4-(2,6-dichlorophenoxy)-3-fluorophenyl)-2-(methylamino)acetamide

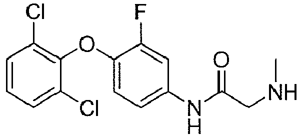

Light brown oil, Yield: 514 mg (90%); ¹H NMR (500 MHz, CDCl₃) δ 9.26 (s, 1H), 7.69 - 7.65 (m, 1H), 7.39 (d, *J* = 8.1, 2H), 7.15 (t, *J* = 8.1, 1H), 7.11 - 7.07 (m, 1H), 6.50 (td, *J* = 8.9, 2.7, 1H), 3.34 (s, 2H), 2.49 (s, 3H), 1.63 (s, 1H). ¹³C NMR (126 MHz, CDCl₃) δ 169.6, 151.7, 140.9, 133.2, 129.7, 129.3, 126.6, 121.6, 115.7, 114.8, 109.0, 54.9, 36.9. Mass: MS (ESI) m/z calculated [M+H]⁺: 343,03; found [M+H]⁺: 343,19.

### Example 104: N-(4-(3,5-dichlorophenoxy)-3-fluorophenyl)-2-(methylamino)acetamide

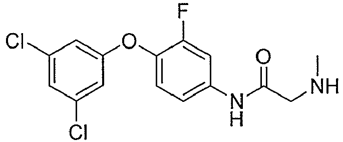

Brown oil, Yield: 256 mg (75%); ¹H NMR (500 MHz, CDCl₃) δ 9.44 (s, 1H), 7.76 (dd, *J* = 12.2, 2.4, 1H), 7.28-7.24 (m, 1H), 7.09 (t, *J* = 8.7, 1H), 7.05 (t, *J* = 1.7, 1H), 6.81 (d, *J* = 1.7, 2H), 3.38 (s, 2H), 2.52 (s, 3H), 1.81 (s, 1H). ¹³C NMR (126 MHz, CDCl₃) δ 169.9, 159.1, 154.4, 137.5, 136.1, 135.6, 123.2, 123.0, 115.5, 115.1, 109.0, 54.9, 36.9. Mass: MS (ESI) m/z calculated [M+H]⁺: 343,03; found [M+H]⁺: 343,19.

### Example 105: N-(3-chloro-4-(2,3-dimethoxyphenoxy)phenyl)-2-(methylamino)acetamide

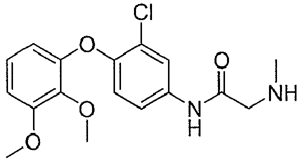

Transparent oil, Yield: 199 mg (51%); ¹H NMR (500 MHz, CDCl₃) δ 9.33 (s, 1H), 7.85 (d, *J* = 2.5, 1H), 7.36 (dd, *J* = 8.8, 2.5, 1H), 6.97 - 6.92 (m, 1H), 6.87 (d, *J* = 8.8, 1H), 6.71 (dd, *J* = 8.4, 1.1, 1H), 6.43 (dd, *J* = 8.3, 1.1, 1H), 3.89 (s, 3H), 3.88 (s, 3H), 3.32 (s, 2H), 2.47 (s, 3H), 1.88 (s, 1H). ¹³C NMR (126 MHz, CDCl₃) δ 169.9, 154.1, 150.1, 148.8, 139.8, 134.1, 125.1, 123.7, 121.6, 119.9, 119.0, 111.3, 107.8, 61.1, 56.1, 54.9, 36.8. Mass: MS (ESI) m/z calculated [M-H]⁻:349,80; found [M-H]⁻: 349,04.

### Example 106: N-(4-(4-bromophenoxy)-3-chlorophenyl)-2-(methylamino)acetamide

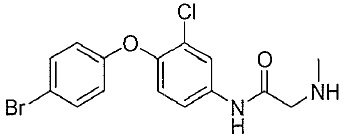

Transparent oil, Yield: 260mg (27%); ¹H NMR (500 MHz, CDCl₃) δ 9.39 (s, 1H), 7.85 (dd, *J* = 4.3, 2.6, 1H), 7.42 (ddd, *J* = 11.2, 8.9, 2.5, 1H), 7.32 - 7.27 (m, 2H), 6.97 (dd, *J* = 8.8, 5.5, 1H), 6.91 (d, *J* = 8.1, 2H), 3.33 (s, 2H), 2.47 (s, 3H), 2.15 (s, 1H). ¹³C NMR (126 MHz, CDCl₃) δ 170.0, 157.3, 148.2, 134.6, 132.6, 129.8, 123.1, 121.6, 119.2, 118.7, 117.3, 54.9, 36.8. Mass: MS (ESI) m/z calculated [M-H]⁻: 368.64; found [M-H]⁻: 368.20.

### Example 107: N-(4-(2,6-dimethoxyphenoxy)-3-fluorophenyl)-2-(methylamino)acetamide

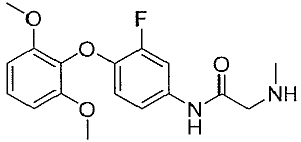

White solid, Yield: 362 mg (72%); ¹H NMR 500 MHz, CDCl₃) δ 9.20 (s, 1H), 7.61 - 7.55 (m, 1H), 7.13 (t, *J* = 8.4, 1H), 7.03 (d, *J* = 8.8, 1H), 6.64 (d, *J* = 8.4, 2H), 6.55 (t, *J* = 9.0, 1H), 3.76 (s, 6H), 3.30 (s, 2H), 2.46 (s, 3H), 1.78 (s, 1H). ¹³C NMR (126 MHz, CDCl₃) δ 169.6, 153.4, 151.7, 142.8, 132.3, 132.0, 125.7, 116.1, 115.6, 114.8, 108.6, 105.4, 56.2, 54.9, 36.8. Mass: MS (ESI) m/z calculated [M-H]⁻: 333.35; found [M-H]⁻: 333.05.

### Example 108: N-(4-(benzo[d][1,3]dioxol-5-yloxy)-3-fluorophenyl)-2-(methylamino)acetamide

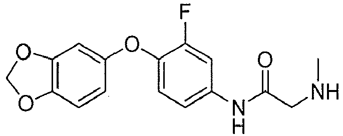

Light brown oil, Yield: 113 mg (64%); ¹H NMR (500 MHz, CDCl₃) δ 9.34 (s, 1H), 7.68 (dd, *J* = 12.4, 2.4, 1H), 7.19 - 7.15 (m, 1H), 6.97 (t, *J* = 8.8, 1H), 6.71 (d, *J* = 8.4, 1H), 6.54 (d, *J* = 2.4, 1H), 6.41 (dd, *J* = 8.4, 2.4, 1H), 5.95 (s, 2H), 3.35 (s, 2H), 2.50 (s, 3H), 1.93 (s, 1H). ¹³C NMR (126 MHz, CDCl₃) δ 189.0, 169.7, 153.8, 152.4, 148.4, 143.4, 140.4, 134.4, 121.5, 115.3, 109.5, 108.9, 108.1, 101.5, 100.3, 54.9, 36.8. Mass: MS (ESI) m/z calculated [M-H]⁻: 317,30; found [M-H]⁻: 317,26.

### Example 109: N-(3-chloro-4-(2,3-dichlorophenoxy)phenyl)-2-(methylamino)acetamide

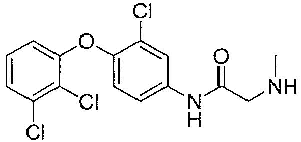

Light brown oil, Yield: 115 mg (58%); ¹H NMR (500 MHz, CDCl₃) δ 9.38 (s, 1H), 7.86 (d, *J* = 2.5, 1H), 7.49 - 7.44 (m, 1H), 7.20 (d, *J* = 8.1, 1H), 7.09 (t, *J* = 8.3, 1H), 6.96 (d, *J* = 8.8, 1H), 6.64 (d, *J* = 8.3, 1H), 3.35 (s, 2H), 2.50 (s, 3H), 1.77 (s, 1H). ¹³C NMR (126 MHz, CDCl₃) δ 169.9, 154.3, 147.3, 135.3, 134.3, 127.4, 124.8, 121.7, 121.4, 121.2, 119.3, 119.1, 115.7, 54.9, 36.9. MS (ESI) m/z calculated [M-H]⁻: 358,63; found [M-H]⁻: 358,93.

### Example 110: N-(4-(2,3-dichlorophenoxy)-3-fluorophenyl)-2-(methylamino)acetamide

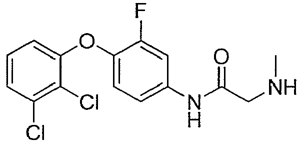

Dark brown oil, Yield: 752 mg (84%); ¹H NMR (500 MHz, CDCl₃) δ 9.54 (s, 1H), 7.81 (dd, *J* = 12.4, 2.4, 1H), 7.21 (dd, *J* = 8.8, 1.1, 1H), 7.17 (dd, *J* = 8.1, 1.1, 1H), 7.08 (t, *J* = 8.2, 1H), 7.00 (t, *J* = 8.8, 1H), 6.69 (d, *J* = 8.3, 1H), 3.35 (s, 2H), 2.47 (s, 3H), 2.12 (s, 1H). ¹³C NMR (126 MHz, CDCl₃) δ 170.2, 154.5, 153.6, 138.3, 135.7, 134.0, 127.5, 124.6, 122.7, 122.0, 115.5, 115.1, 108.8, 54.9, 36.7. MS (ESI) m/z calculated [M-H]⁻: 342.18; found [M-H]⁻: 342.93

### Example 111: N-(4-(4-chlorophenoxy)-3-fluorophenyl)-2-(methylamino)acetamide

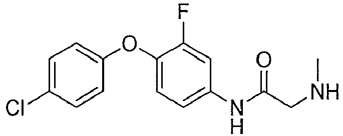

Dark brown oil, Yield: 827 mg (64%); ¹H NMR (500 MHz, CDCl₃) δ 9.48 (s, 1H), 7.69 (dt, J = 12.5, 2.9, 1H), 7.13 - 7.06 (m, 3H), 6.89 (t, J = 8.9, 1H), 6.77 - 6.70 (m, 2H), 3.24 (s, 2H), 2.33 (s, 3H), 2.08 (s, 1H). ¹³C NMR (126 MHz, CDCl₃) δ 170.3, 156.4, 153.9, 138.6, 135.4, 129.5, 127.6, 122.4, 117.8, 115.7, 108.9, 54.8, 36.5. MS (ESI) m/z calculated [M+H]⁺: 309.74; found [M+H]⁺: 309.07.

### Following examples were synthesized by using Method 5: Method for 2-bromo-1,1-diethoxyethane coupling reaction

### Example 112: 1-(2,2-diethoxyethoxy)-2-nitrobenzene

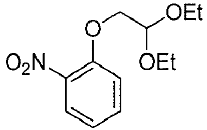

Yellow solid, Yield: 980 mg (77 %); ¹H NMR (500 MHz, CDCl₃) δ 7.83 (dd, *J* = 8.1, 1.6, 1H), 7.57 - 7.46 (m, 1H), 7.10 (d, *J* = 8.5, 1H), 7.07 - 7.00 (m, 1H), 4.85 (t, *J* = 5.2, 1H), 4.12 (d, *J* = 5.2, 2H), 3.87 - 3.76 (m, 2H), 3.71 - 3.64 (m, 2H), 1.24 (t, *J* = 7.0, 6H). ¹³C NMR (126 MHz, CDCl₃) δ 152.1, 134.1, 125.6, 120.7, 114.9, 100.7, 70.7, 63.9, 15.3 ppm.

### Example 113: 1-(2,2-diethoxyethoxy)-4-nitrobenzene

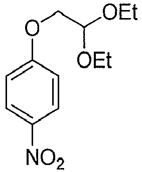

Yellow solid, Yield: 870 mg (68 %); ¹H NMR (500 MHz, CDCl₃) δ 8.20 (d, *J* = 9.2, 2H), 6.99 (d, *J* = 9.2, 2H), 4.85 (t, *J* = 5.1, 1H), 4.09 (d, *J* = 5.1, 2H), 3.78 (dq, *J* = 9.2, 7.1, 2H), 3.65 (dq, *J* = 9.3, 7.1, 2H), 1.25 (t, *J* = 7.0, 6H). ¹³C NMR (126 MHz, CDCl₃) δ 163.59, 141.74, 125.86, 114.66, 100.23, 69.13, 63.03, 15.32 ppm.

### Example 114: 1-(2,2-diethoxyethoxy)-2-methoxybenzene

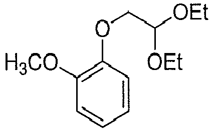

Colorless liquid, Yield: 1040 mg (87 %); ¹H NMR (500 MHz, CDCl₃) δ 6.98 - 6.93 (m, 1H), 6.91 - 6.84 (m, 3H), 4.85 (t, *J* = 5.2, 1H), 4.05 (d, *J* = 5.1, 2H), 3.84 (s, 3H), 3.81 - 3.71 (m, 2H), 3.70 - 3.58 (m, 2H), 1.22 (t, *J* = 7.0, 6H). ¹³C NMR (126 MHz, CDCl₃) δ 150.3, 148.9, 121.8, 121.1, 115.5, 113.2, 100.9, 70.7, 62.6, 56.2, 15.2 ppm.

### Example 115: 1-chloro-2-(2,2-diethoxyethoxy)benzene

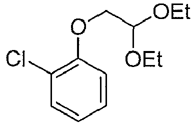

Colorless liquid, Yield: 2.04 gm (87 %); ¹H NMR (500 MHz, CDCl₃) δ 7.31 (d, *J=* 8.0, 2H), 7.18 (t, *J* = 7.8, 2H), 7.02 (d, *J* = 8.1, 2H), 6.87 (t, *J* = 7.7, 2H), 5.62 (s, 2H), 4.67 (t, *J* = 5.4, 1H), 3.71 (dq, *J* = 9.0, 7.0, 2H), 3.59 (dq, *J* = 9.1, 7.0, 2H), 3.37 (d, *J* = 5.6, 2H), 1.24 (t, *J* = 7.1, 6H). ¹³C NMR (126 MHz, CDCl₃) δ 151.4, 129.0, 128.4, 121.3, 116.3, 101.5, 62.5, 31.9, 15.2 ppm.

### Example 116: 1-bromo-4-(2,2-diethoxyethoxy)benzene

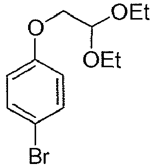

White liquid, Yield: 1040 mg (72 %); MS (ESI) m/z calculated [M+Na]⁺: 311; found [M+Na]⁺: 311.14

### Example 117: 1-(benzyloxy)-2-(2,2-diethoxyethoxy)benzene

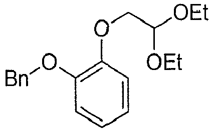

Colorless Liquid, Yield: 470 mg (30 %); ¹H NMR (500 MHz, CDCl₃) δ 7.50 - 7.47 (m, 2H), 7.39 (M, 2H), 7.33 (M, 1H), 7.05 - 6.85 (m, 4H), 5.14 (s, 2H), 4.90 (t, *J* = 5.2, 1H), 4.09 (d, *J* = 5.3, 2H), 3.79 (dq, *J* = 9.3, 7.1, 2H), 3.66 (dq, *J* = 9.3, 7.0, 2H), 1.25 (t, *J* = 7.1, 6H). ¹³C NMR (126 MHz, CDCl₃) δ 149.1, 137.4, 128.5, 128.4, 127.8, 127.4, 127.3, 121.7, 121.7, 121.6, 115.3, 115.2, 114.8, 100.8, 71.3, 71.2, 70.2, 62.9, 15.3 ppm.

### Example 118: 1-(benzyloxy)-4-(2,2-diethoxyethoxy)benzene

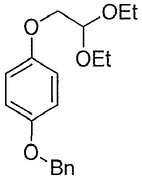

Colorless Liquid, Yield: 890 mg (47 %); ¹H NMR (500 MHz, CDCl₃) δ 7.42 (M, 2H), 7.38 (t, *J* = 7.5, 2H), 7.31 (M, 1H), 6.94 - 6.81 (m, 4H), 5.01 (s, 2H), 4.81 (t, *J* = 5.2, 1H), 3.96 (d, *J* = 5.2, 2H), 3.76 (dq, *J* = 9.3, 7.1, 2H), 3.63 (dq, *J* = 9.3, 7.1, 2H), 1.24 (t, *J* = 7.1, 6H). ¹³C NMR (126 MHz, CDCl₃) δ 153.2, 153.0, 137.3, 128.5, 127.9, 127.5, 115.8, 115.7, 100.6, 70.7, 69.2, 62.5, 15.4 ppm.

### Example 119: 1-(2,2-diethoxyethoxy)-4-methoxy-2-nitrobenzene

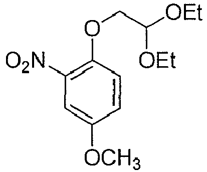

Brown solid, Yield: 1000 mg (88 %); ¹H NMR (500 MHz, CDCl₃) δ 7.37 (d, *J* = 2.8, 1H), 7.14 - 7.00 (m, 2H), 4.83 (t, *J* = 5.2, 1H), 4.07 (d, *J* = 5.2, 2H), 3.82 (s, 3H), 3.81 - 3.73 (m, 2H), 3.72 - 3.61 (m, 2H), 1.24 (t, *J* = 6.9, 6H). ¹³C NMR (126 MHz, CDCl₃) δ 162.5, 153.3, 146.4, 120.8, 117.2, 109.7, 100.8, 71.6, 63.7, 56.0, 15.3 ppm.

### Example 120: 1-(2,2-diethoxyethoxy)-3-methoxybenzene

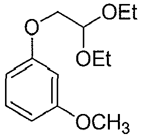

Brown solid, Yield: 730 mg (76 %); ¹H NMR (500 MHz, CDCl₃) δ 7.17 (t, *J* = 8.1, 1H), 6.60 - 6.44 (m, 3H), 4.83 (t, *J* = 5.2, 1H), 3.99 (d, *J* = 5.2, 2H), 3.80 - 3.71 (m, 5H), 3.64 (dq, *J* = 9.3, 7.0, 2H), 1.25 (t, *J* = 7.1, 6H). ¹³C NMR (126 MHz, CDCl₃) δ 160.8, 159.9, 129.9, 106.8, 106.7, 101.1, 100.4, 68.5, 62.5, 55.3, 15.3 ppm.

### Example 121: 1-(2,2-diethoxyethoxy)-3-methylbenzene

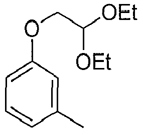

Brown solid, Yield: 640 mg (72 %); ¹H NMR (500 MHz, CDCl₃) δ 7.15 (t, *J* = 7.8, 1H), 6.82 - 6.68 (m, 3H), 4.83 (t, *J* = 5.2, 1H), 4.00 (d, *J* = 5.2, 2H), 3.76 (dq, *J* = 9.3, 7.1, 2H), 3.64 (dq, *J* = 9.3, 7.0, 2H), 2.32 (s, 3H), 1.25 (t, *J* = 7.1, 6H). ¹³C NMR (126 MHz, CDCl₃) δ 158.6, 139.5, 129.2, 121.8, 115.5, 111.5, 100.5, 68.5, 62.5, 21.5, 15.4 ppm.

### Example 122: 1-(2,2-diethoxyethoxy)-4-methoxybenzene

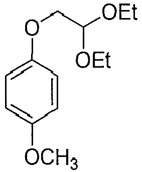

White solid, Yield: 912 mg (95 %); ¹H NMR (500 MHz, CDCl₃) δ 6.91 - 6.84 (m, 2H), 6.84 - 6.78 (m, 2H), 4.82 (t, *J* = 5.2, 1H), 3.96 (d, *J* = 5.2, 2H), 3.82 - 3.71 (m, 5H), 3.63 (dq, *J* = 9.3, 7.1, 2H), 1.25 (t, *J* = 7.1, 6H). ¹³C NMR (126 MHz, CDCl₃) δ 154.0, 152.8, 115.7, 114.6, 100.6, 69.3, 62.5, 55.7, 15.4 ppm.

### Example 123: N-(2,2-diethoxyethyl)aniline

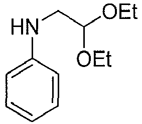

Colorless liquid, Yield: 600 mg (72 %); ¹H NMR (500 MHz, CDCl₃) δ 7.18 (t, *J* = 7.8, 2H), 6.71 (td, *J* = 7.4, 0.7, 1H), 6.64 (d, *J* = 8.4, 2H), 4.68 (t, *J* = 5.6, 1H), 3.88 (s, 1H), 3.78 - 3.67 (m, 2H), 3.62 - 3.53 (m, 2H), 3.25 (d, *J* = 5.5, 2H), 1.24 (t, *J* = 7.1, 6H). ¹³C NMR (126 MHz, CDCl₃) δ 147.9, 129.3, 117.7, 113.1, 100.9, 63.1, 62.4, 46.4, 15.5, 15.4 ppm.

### Following examples were synthesized by using Method 6: Method for hydrolysis reaction

### Example 124: 2-(4-nitrophenoxy)acetaldehyde

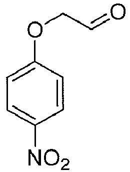

Colorless liquid, Yield: 540 mg (74 %); ¹H NMR (500 MHz, CDCl₃) δ 9.87 (d, *J* = 0.5, 1H), 8.24 (d, *J* = 9.3, 2H), 6.99 (d, *J* = 9.2, 2H), 4.72 (s, 2H). ¹³C NMR (126 MHz, CDCl₃) δ 197.6, 134.3, 126.1, 122.0, 115.1, 73.8 ppm.

### Example 125: 2-(2-nitrophenoxy)acetaldehyde

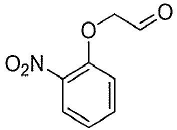

Colorless viscous liquid, Yield: 530 mg (77 %); ¹H NMR (500 MHz, CDCl₃) δ 9.88 (s, 1H), 7.90 (dd, *J* = 8.1, 1.5, 1H), 7.61 - 7.48 (m, 1H), 7.14 (t, *J* = 7.8, 1H), 6.97 (d, *J* = 8.3, 1H), 4.71 (s, 2H). ¹³C NMR (126 MHz, CDCl₃) δ 197.6, 150.9, 134.3, 126.1, 122.0, 121.2, 115.1, 73.8 ppm.

### Example 126: 2-(2-chlorophenoxy)acetaldehyde

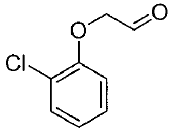

Colorless liquid, Yield: 750 mg (88 %); ¹H NMR (500 MHz, CDCl₃) δ 9.91 (s, 1H), 7.42 (d, *J* = 7.9, 1H), 7.22 (d, *J* = 8.0, 1H), 6.99 (t, *J* = 7.7, 1H), 6.82 (d, *J* = 8.2, 1H), 4.63 (s, 2H). ¹³C NMR (126 MHz, CDCl₃) δ 198.93, 130.83, 127.88, 122.90, 113.91, 73.58 ppm.

### Example 127: 2-(2-methoxyphenoxy)acetaldehyde

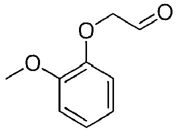

Colorless liquid, Yield: 550 mg (79 %); ¹H NMR (500 MHz, CDCl₃) δ 9.91 (d, *J* = 0.9, 1H), 7.02 (td, *J* = 8.1, 1.3, 1H), 6.94 (d, *J* = 6.8, 1H), 6.91 - 6.89 (m, 1H), 6.84 (d, *J* = 7.9, 1H), 4.60 (s, 2H), 3.90 (s, 3H). ¹³C NMR (126 MHz, CDCl₃) δ 200.3, 123.0, 122.6, 120.9, 116.0, 114.9, 112.2, 74.2, 55.8 ppm.

### Example 128: 2-(4-bromophenoxy)acetaldehyde

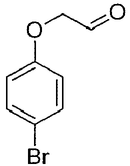

Colorless liquid, Yield: 530 mg (73 %); ¹H NMR (500 MHz, CDCl₃) δ 9.84 (d, *J* = 0.7, 1H), 7.41 (d, *J* = 8.8, 2H), 6.79 (d, *J* = 8.9, 2H), 4.56 (s, 2H). ¹³C NMR (126 MHz, CDCl₃) δ 198.5, 132.6, 116.4, 72.8 ppm.

### Example 129: 2-(2-(benzyloxy)phenoxy)acetaldehyde

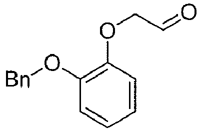

Colorless liquid, Yield: 140 mg (58 %); ¹H NMR (500 MHz, CDCl₃) δ 9.79 (s, 1H), 7.41 (d, *J* = 7.3, 2H), 7.34 (t, *J* = 7.4, 2H), 7.30 - 7.24 (m, 1H), 6.97 - 6.79 (m, 6H), 5.09 (s, 2H), 4.53 (s, 2H) ppm.

### Example 130: 2-(3-methoxyphenoxy)acetaldehyde

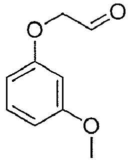

Colorless liquid, Yield: 430 mg (99 %); ¹H NMR (500 MHz, CDCl₃) δ 9.85 (d, *J* = 0.8, 1H), 7.19 (M, 2H), 6.61 - 6.56 (m, 1H), 6.55 (d, *J* = 2.3, 1H), 4.56 (s, 2H), 3.52 (s, 3H). ¹³C NMR (126 MHz, CDCl₃) δ 199.3, 160.8, 159.6, 129.9, 107.1, 106.7, 101.3, 70.2, 55.4 ppm.

### Following examples were synthesized by using Method 7: Method for Ugi reaction

### Example 131: 2-((2-(benzyloxy)-1-(1-(2-cyanoethyl)-1H-tetrazol-5-yl)ethyl)(methyl)-amino)-N-(4-phenoxyphenyl)acetamide

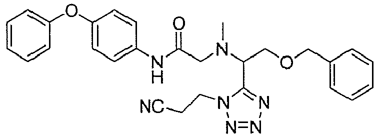

White Solid, Yield: 440 mg (43 %); ¹H NMR (500 MHz, CDCl₃) δ 8.53 (s, 1H), 7.44 - 7.39 (m, 2H), 7.37 - 7.30 (m, 6H), 7.26 - 7.23 (m, 1H), 7.09 (t, *J* = 7.4, 1H), 7.00 - 6.92 (m, 4H), 4.78 (dt, *J* = 13.6, 6.7, 1H), 4.73 - 4.65 (m, 1H), 4.58 - 4.49 (m, 2H), 4.48 - 4.42 (m, 1H), 4.23 (dd, *J* = 9.7, 7.4, 1H), 3.91 (dd, *J* = 9.7, 6.2, 1H), 3.59 (d, *J* = 16.3, 1H), 3.40 (d, *J* = 16.2, 1H), 3.12 - 2.92 (m, 2H), 2.48 (s, 3H). MS (ESI) m/z calculated [M+H]⁺: 459,51; found [M+H]⁺: 459,21.

### Example 132: 2-((2-((2-chlorobenzyl)oxy)-1-(1-(2-cyanoethyl)-1H-tetrazol-5-yl)ethyl)-(methyl)amino)-N-(4-phenoxyphenyl)acetamide

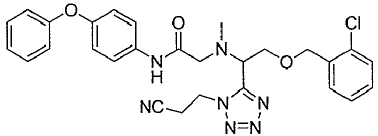

White Solid, Yield: 200 mg (27 %); ¹H NMR (500 MHz, CDCl₃) δ 9.59 (s, 1H), 7.43 - 7.30 (m, 8H), 7.29 - 7.25 (m, 6H), 7.25 - 7.20 (m, 2H), 7.10 (t, *J* = 7.4, 1H), 6.96 (d, *J* = 7.8, 2H), 6.92 - 6.84 (m, 2H), 5.30 (s, 1H), 4.74 (d, *J* = 3.0, 2H), 4.48 (dd, *J* = 9.0, 4.6, 1H), 4.25 - 4.16 (m, 1H), 3.94 (dd, *J* = 10.3, 4.7, 1H), 3.47 (s, 2H), 2.44 (s, 3H), 2.17 (s, 1H). ¹³C NMR (126 MHz, CDCl₃) δ 177.1, 132.4, 130.4, 129.8, 129.8, 127.1, 123.2, 121.9, 119.5, 118.5, 71.2, 68.2, 58.2, 57.7, 38.9; MS (ESI) m/z calculated [M+H]⁺: 493.95; found [M+H]⁺: 493.17.

### Example 133: 2-((1-(1-(2-cyanoethyl)-1H-tetrazol-5-yl)-2-phenoxyethyl)(methyl)amino)-N-(4-phenoxyphenyl)acetamide

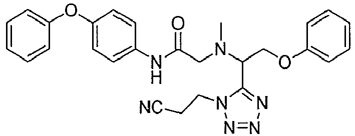

White Solid, Yield: 588 mg (25 %); ¹H NMR (500 MHz, CDCl₃) δ 8.23 (s, 1H), 7.49 (d, *J* = 8.9, 2H), 7.35 - 7.27 (m, 4H), 7.09 (t, *J* = 7.4, 1H), 7.04 - 6.97 (m, 5H), 6.92 (d, *J* = 8.2, 2H), 4.93 (t, *J* = 6.3, 2H), 4.80 - 4.71 (m, 2H), 4.51 (q, *J* = 9.1, 1H), 3.75 (d, *J* = 16.2, 1H), 3.52 (d, *J* = 16.2, 1H), 3.25 - 3.06 (m, 2H), 2.53 (s, 3H), 1.54 (s, 3H); MS (ESI) m/z calculated [M+H]⁺: 497.5; found [M+H]⁺: 497.5.

### Example 134: 2-((2-(2-chlorophenoxy)-1-(1-(2-cyanoethyl)-1H-tetrazol-5-yl)ethyl)-(methyl)amino)-N-(4-phenoxyphenyl)acetamide

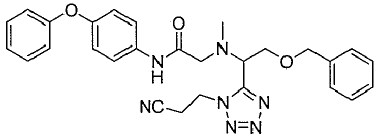

White Solid, Yield: 630 mg (27 %); ¹H NMR (500 MHz, CDCl₃) δ 8.22 (s, 1H), 7.51 (d, *J* = 8.8, 2H), 7.39 (d, *J* = 7.9, 1H), 7.32 (t, *J* = 7.8, 2H), 7.27 (d, *J* = 5.9, 2H), 7.13 - 7.03 (m, 2H), 7.03 - 6.91 (m, 5H), 5.09 (t, *J* = 6.4, 2H), 4.90 - 4.76 (m, 2H), 4.64 - 4.52 (m, 1H), 4.00 (d, *J* = 15.6, 1H), 3.64 (d, *J* = 15.6, 1H), 3.24 - 3.06 (m, 2H), 2.47 (s, 3H). ¹³C NMR (126 MHz, CDCl₃) δ 168.01, 157.37, 153.88, 153.26, 152.93, 132.65, 130.48, 129.74, 128.37, 123.19, 122.73, 122.04, 121.79, 119.60, 118.51, 116.33, 113.49, 77.27, 77.02, 76.77, 66.62, 59.72, 55.51, 43.26, 38.05, 18.98; MS (ESI) m/z calculated [M+H]⁺: 532.18; found [M+H]⁺: 532.25.

### Example 135: 2-((2-((2-chlorobenzyl)oxy)-1-(1-(2-cyanoethyl)-1H-tetrazol-5-yl)ethyl)(methyl)amino)-N-(4-phenoxyphenyl)acetamide

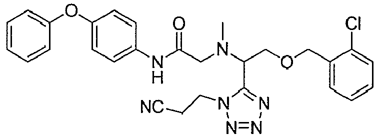

White Solid, Yield: 200 mg (27 %); ¹H NMR (500 MHz, CDCl₃) δ 9.59 (s, 1H), 7.43 - 7.30 (m, 8H), 7.29 - 7.25 (m, 6H), 7.25 - 7.20 (m, 2H), 7.10 (t, *J* = 7.4, 1H), 6.96 (d, *J* = 7.8, 2H), 6.92 - 6.84 (m, 2H), 5.30 (s, 1H), 4.74 (d, *J* = 3.0, 2H), 4.48 (dd, *J* = 9.0, 4.6, 1H), 4.25 - 4.16 (m, 1H), 3.94 (dd, *J* = 10.3, 4.7, 1H), 3.47 (s, 2H), 2.44 (s, 3H), 2.17 (s, 1H). ¹³C NMR (126 MHz, CDCl₃) δ 177.1, 132.4, 130.4, 129.8, 129.8, 127.1, 123.2, 121.9, 119.5, 118.5, 71.2, 68.2, 58.2, 57.7, 38.9; MS (ESI) m/z calculated [M+H]⁺: 493.95; found [M+H]⁺: 493.17.

### Example 136: 2-((2-(2-(benzyloxy)phenoxy)-1-(1-(2-cyanoethyl)-1H-tetrazol-5-yl)ethyl)-(methyl)amino)-N-(4-phenoxyphenyl)acetamide

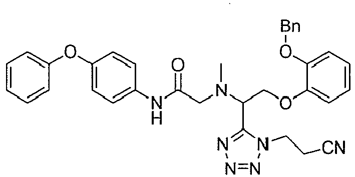

Colorless Solid, Yield: 17 mg (28 %); ¹H NMR (500 MHz, CDCl₃) δ 8.39 (s, 1H), 7.43 - 7.37 (m, 2H), 7.35 - 7.29 (m, 7H), 7.11 - 7.05 (m, 2H), 7.02 - 6.99 (m, 3H), 6.97 (d, *J* = 8.3, 2H), 6.94 (d, *J* = 8.9, 2H), 5.07 (s, 2H), 4.96 - 4.86 (m, 1H), 4.78 - 4.63 (m, 3H), 4.54 (dd, *J* = 9.3, 5.0, 1H), 3.75 - 3.67 (m, 1H), 3.64 - 3.58 (m, 1H), 3.43 (d, *J* = 14.2, 1H), 2.96 - 2.84 (m, 1H), 2.78 (dt, *J* = 17.1, 6.3, 1H), 2.37 (s, 3H).

### Example 137: 2-((1-(1-(2-cyanoethyl)-1H-tetrazol-5-yl)-2-(2-nitrophenoxy)ethyl)-(methyl)amino)-N-(4-phenoxyphenyl)acetamide

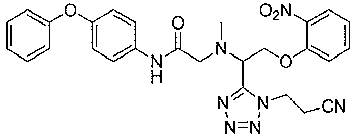

Colorless Solid, Yield: 17 mg (28 %); ¹H NMR (500 MHz, CDCl₃) δ 8.31 (s, 1H), 7.87 (dd, *J* = 8.1, 1.6, 1H), 7.63 - 7.59 (m, 1H), 7.58 (d, *J* = 8.9, 2H), 7.34 - 7.29 (m, 2H), 7.27 (d, *J* = 7.3, 1H), 7.12 (t, *J* = 7.5, 1H), 7.08 (t, *J* = 7.4, 1H), 7.00 - 6.96 (m, 4H), 5.20 (td, *J* = 6.6, 2.5, 2H), 4.97 (t, *J* = 9.5, 1H), 4.86 (dd, *J* = 9.1, 4.2, 1H), 4.67 (dd, *J* = 9.9, 4.2, 1H), 4.04 (d, *J* = 15.6, 1H), 3.61 (d, *J* = 15.5, 1H), 3.23 - 3.09 (m, 2H), 2.44 - 2.36 (m, 3H). ¹³C NMR (126 MHz, CDCl₃) δ 168.1, 157.4, 153.7, 152.8, 151.4, 139.5, 135.0, 133.0, 129.7, 125.9, 123.1, 121.8, 121.6, 119.6, 118.5, 116.6, 114.4, 66.6, 59.4, 54.8, 43.2,37.4, 19.0 ppm. MS (ESI) m/z calculated [M+H]⁺: 543,20; found [M+H]⁺: 543.27.

### Example 138: 2-((1-(1-(2-cyanoethyl)-1H-tetrazol-5-yl)-2-(m-tolyloxy)ethyl)(methyl)-amino)-N-(4-phenoxyphenyl)acetamide

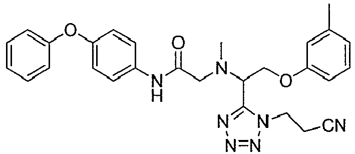

Brown Solid, Yield: 40 mg (39 %); ¹H NMR (500 MHz, CDCl₃) δ 8.48 (s, 1H), 7.49 (d, *J* = 8.9, 2H), 7.35 - 7.28 (m, 4H), 7.16 (t, *J* = 7.8, 1H), 7.13 - 7.04 (m, 2H), 7.00 - 6.93 (m, 6H), 6.81 (d, *J* = 7.5, 1H), 6.75 - 6.69 (m, 2H), 6.63 - 6.55 (m, 1H), 4.92 (t, *J* = 6.6, 2H), 4.76 - 4.67 (m, 2H), 4.46 (dd, *J* = 9.6, 4.9, 1H), 3.74 (d, *J* = 16.4, 1H), 3.52 (d, *J* = 16.3, 1H), 3.20 - 3.06 (m, 2H), 2.50 (s, 3H), 2.31 (s, 3H).

### Example 139: N-(3-chloro-4-(3-chlorophenoxy)phenyl)-2-((2-(2-chlorophenoxy)-1-(1-(2-cyanoethyl)-1H-tetrazol-5-yl)ethyl)(methyl)amino)acetamide

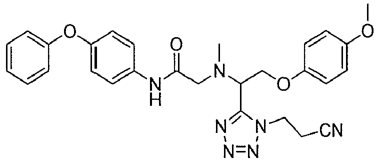

Colorless Solid, Yield: 50 mg (32 %); ¹H NMR (500 MHz, CDCl₃) δ 8.77 (s, 1H), 7.49 (d, *J* = 8.9, 2H), 7.32 (t, *J* = 7.9, 2H), 7.09 (t, *J* = 7.4, 1H), 6.97 (d, *J* = 8.8, 4H), 6.87 - 6.78 (m, 4H), 4.88 (td, *J* = 6.5, 2.0, 2H), 4.73 (dd, *J* = 7.5, 5.8, 1H), 4.64 (dd, *J* = 9.7, 7.8, 1H), 4.40 (dd, *J* = 9.8, 5.7, 1H), 3.74 (s, 3H), 3.71 - 3.65 (m, 1H), 3.51 (d, *J* = 16.5, 1H), 3.15 (dd, *J* = 12.0, 6.4, 2H), 2.77 (t, *J* = 6.6, 1H), 2.51 (s, 3H). ¹³C NMR (126 MHz, CDCl₃) δ 168.7, 157.3, 154.7, 153.8, 153.3, 151.7, 132.7, 129.8, 123.3, 122.0, 119.5, 118.5, 117.3, 116.7, 115.8, 114.9, 66.8, 58.9, 56.7, 55.8, 41.7, 39.1, 19.1 ppm; MS (ESI) m/z calculated [M+Na]⁺: 550.22; found [M+Na]⁺: 550.36.

### Example 140: 2-((1-(1-(2-cyanoethyl)-1H-tetrazol-5-yl)-2-(2-methoxyphenoxy)ethyl)-(methyl)amino)-N-(4-phenoxyphenyl)acetamide

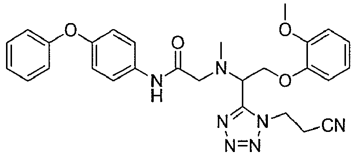

Colorless Solid, Yield: 50 mg (32 %); ¹H NMR (500 MHz, CDCl₃) δ 9.20 (s, 1H), 7.53 (d, *J* = 8.9, 2H), 7.30 (dd, *J* = 8.4, 7.6, 2H), 7.07 (t, *J* = 7.4, 1H), 7.01 - 6.92 (m, 8H), 5.02 (dt, *J* = 13.4, 6.5, 1H), 4.90 (dt, *J* = 13.8, 6.7, 1H), 4.79 (t, *J* = 9.3, 1H), 4.71 (dd, *J* = 8.8, 4.1, 1H), 4.54 (dd, *J* = 9.7, 4.1, 1H), 3.94 (d, *J* = 13.6, 1H), 3.87 (s, 3H), 3.55 (d, *J* = 13.5, 1H), 3.14 (t, *J* = 6.6, 2H), 2.35 (s, 3H). ¹³C NMR (126 MHz, CDCl₃) δ 168.4, 157.4, 153.8, 153.1, 148.4, 147.1, 133.1, 129.8, 123.2, 122.2, 121.8, 121.6, 119.6, 118.5, 116.4, 112.9, 111.7, 66.1, 60.7, 55.7, 54.4, 43.1, 37.6, 18.7 ppm; MS (ESI) m/z calculated [M+Na]⁺: 550.22; found [M+Na]⁺: 550.39.

### Example 141: 2-((1-(1-(2-cyanoethyl)-1H-tetrazol-5-yl)-2-(3-methoxyphenoxy)ethyl)-(methyl)amino)-N-(4-phenoxyphenyl)acetamide

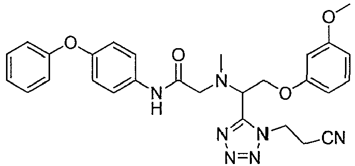

Brown Solid, Yield: 90 mg (27 %); ¹H NMR (500 MHz, CDCl₃) δ 8.72 (s, 1H), 7.49 (d, *J* = 8.9, 2H), 7.30 (t, *J* = 8.0, 2H), 7.16 (t, *J* = 8.2, 1H), 7.07 (t, *J* = 7.4, 1H), 7.01 - 6.93 (m, 4H), 6.51 (ddd, *J* = 13.3, 8.2, 2.2, 2H), 6.46 (t, *J* = 2.3, 1H), 4.88 (t, *J* = 6.5, 2H), 4.74 (dd, *J* = 7.7, 5.6, 1H), 4.71 - 4.62 (m, 1H), 4.43 (dd, *J* = 9.9, 5.5, 1H), 3.78 - 3.67 (m, 4H), 3.51 (d, *J* = 16.4, 1H), 3.18 - 3.09 (m, 2H), 2.48 (s, 3H). ¹³C NMR (126 MHz, CDCl₃) δ 168.5, 161.0, 158.8, 157.4, 153.8, 153.2, 132.8, 130.3, 129.8, 123.2, 121.9, 119.6, 118.5, 116.7, 107.3, 106.7, 101.3, 65.9, 58.9, 56.6, 55.4, 43.3, 38.9, 18.8 ppm; MS (ESI) m/z calculated [M+Na]⁺: 550.22; found [M+Na]⁺: 550.36.

### Example 142: 2-((1-(1-(2-cyanoethyl)-1H-tetrazol-5-yl)-2-(p-tolyloxy)ethyl)(methyl)-amino)-N-(4-phenoxyphenyl)acetamide

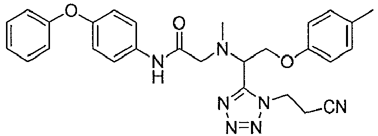

Brown Solid, Yield: 26 mg (26 %); ¹H NMR (500 MHz, CDCl₃) δ 8.64 (s, 1H), 7.48 (d, *J* = 8.8, 2H), 7.31 (t, *J* = 7.8, 2H), 7.13 - 7.05 (m, 3H), 6.97 (d, *J* = 8.7, 4H), 6.80 (d, *J* = 8.4, 2H), 4.89 (t, *J* = 6.5, 2H), 4.74 (dd, *J* = 7.4, 5.7, 1H), 4.70 - 4.63 (m, 1H), 4.42 (dd, *J* = 9.8, 5.5, 1H), 3.72 (d, *J* = 16.4, 1H), 3.51 (d, *J* = 16.4, 1H), 3.19 - 3.02 (m, 2H), 2.50 (s, 3H), 2.26 (s, 3H). ¹³C NMR (126 MHz, CDCl₃) δ 168.4, 157.4, 155.5, 153.8, 153.2, 132.8, 131.4, 130.3, 129.8, 123.2, 121.9, 119.6, 118.5, 116.5, 114.4, 66.1, 59.1, 56.7, 43.3, 39.0, 20.5, 18.9 ppm; MS (ESI) m/z calculated [M+Na]⁺: 534.22; found [M+Na]⁺: 534.36.

### Example 143: 2-((1-(1-(2-cyanoethyl)-1H-tetrazol-5-yl)-2-(2,5-dichlorophenoxy)ethyl)-(methyl)amino)-N-(4-phenoxyphenyl)acetamide

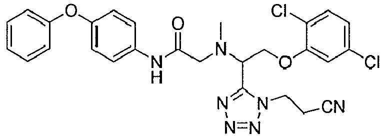

Brown Solid, Yield: 33 mg (35 %); ¹H NMR (500 MHz, CDCl₃) δ 8.31 (s, 1H), 7.50 (d, *J* = 8.9, 2H), 7.35 - 7.27 (m, 3H), 7.07 (dd, *J* = 12.1, 4.8, 2H), 7.01 - 6.92 (m, 5H), 5.14 - 4.99 (m, 2H), 4.88 (dd, *J* = 8.5, 4.9, 1H), 4.80 (t, *J* = 9.1, 1H), 3.93 (d, *J* = 15.9, 1H), 3.62 (d, *J* = 15.9, 1H), 3.23 - 3.04 (m, 2H), 2.47 (s, 3H). ¹³C NMR (126 MHz, CDCl₃) δ 168.1, 157.3, 153.9, 153.8, 152.8, 133.7, 132.7, 130.9, 129.8, 123.2, 122.6, 121.9, 120.6, 119.6, 118.5, 116.5, 114.1, 66.8, 59.5, 55.8, 43.3, 38.1, 18.9 ppm; MS (ESI) m/z calculated [M+Na]⁺: 588.13; found [M+Na]⁺: 588.32.

### Example 144: N-(3-chloro-4-(3-chlorophenoxy)phenyl)-2-((2-(2-chlorophenoxy)-1-(1-(2-cyanoethyl)-1H-tetrazol-5-yl)ethyl)(methyl)amino)acetamide

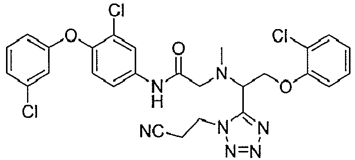

Colorless Solid, Yield: 77 mg (42 %); ¹H NMR (500 MHz, CDCl₃) δ 8.60 (s, 1H), 7.82 (d, *J* = 2.5, 1H), 7.43 (dd, *J* = 8.8, 2.5, 1H), 7.36 (dd, *J* = 7.9, 1.5, 1H), 7.26 - 7.18 (m, 2H), 7.07 - 6.98 (m, 3H), 6.96 (td, *J* = 7.8, 1.2, 1H), 6.87 (t, *J* = 2.1, 1H), 6.79 (dd, *J* = 8.2, 2.0, 1H), 5.05 (td, *J* = 6.6, 1.3, 2H), 4.86 (dd, *J* = 8.2, 5.1, 1H), 4.83 - 4.75 (m, 1H), 4.53 (dd, *J* = 9.7, 5.1, 1H), 3.95 (d, *J* = 16.1, 1H), 3.68 (d, *J* = 16.0, 1H), 3.26 - 3.09 (m, 2H), 2.51 (s, 3H). ¹³C NMR (126 MHz, CDCl₃) δ 168.5, 158.1, 153.2, 153.1, 147.7, 135.1, 134.9, 130.5, 130.5, 128.4, 126.8, 123.2, 122.8, 122.4, 122.2, 122.2, 119.8, 117.3, 116.5, 115.3, 113.7, 66.8, 59.24, 56.1, 43.3, 38.7, 19.0 ppm; MS (ESI) m/z calculated [M+Na]⁺: 622.10; found [M+Na]⁺: 622.24.

### Example 145: 2-((2-(2-chlorophenoxy)-1-(1-(2-cyanoethyl)-1H-tetrazol-5-yl)ethyl)-(cyclobutyl)amino)-N-(4-phenoxyphenyl)acetamide

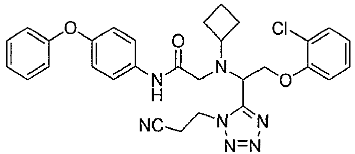

Yellow Solid, Yield: 40 mg (36%); ¹H NMR (500 MHz, CDCl₃) δ 8.52 - 8.42 (m, 1H), 7.48 (d, *J* = 8.9, 2H), 7.37 (dd, *J* = 7.9, 1.4, 1H), 7.32 (d, *J* = 8.3, 2H), 7.25 - 7.21 (m, 1H), 7.09 - 7.06 (m, 1H), 7.03 - 7.00 (m, 1H), 6.96 (dd, *J* = 8.6, 1.9, 5H), 5.10 (dt, *J* = 13.9, 6.9, 1H), 5.02 - 4.91 (m, 1H), 4.82 (dd, *J* = 8.7, 5.1, 1H), 4.72 (t, *J* = 9.2, 1H), 4.53 (dd, *J* = 9.7, 5.1, 1H), 4.02 (d, *J* = 16.2, 1H), 3.64 (d, *J* = 16.1, 1H), 3.51 - 3.46 (m, 1H), 3.17 (t, *J* = 6.9, 2H), 2.08 - 1.96 (m, 2H), 1.76 (dt, *J* = 19.7, 9.9, 2H).

### Example 146: 2-((2-(2-chlorophenoxy)-1-(1-(2-cyanoethyl)-1H-tetrazol-5-yl)ethyl)-(isopropyl)amino)-N-(4-phenoxyphenyl)acetamide

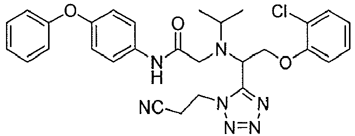

Yellow Solid, Yield: 201 mg (36%); ¹H NMR (500 MHz, CDCl₃) δ 8.92 (s, 1H), 7.56 - 7.50 (m, 2H), 7.35 (dd, *J* = 7.8, 1.5, 1H), 7.33 - 7.27 (m, 2H), 7.25 - 7.18 (m, 1H), 7.08 (t, *J* = 7.4, 1H), 6.96 (dd, *J* = 6.9, 5.0, 5H), 5.18 - 5.07 (m, 1H), 4.93 - 4.82 (m, 2H), 4.72 - 4.62 (m, 1H), 4.43 (dd, *J* = 9.5, 6.0, 1H), 4.00 (d, *J* = 16.7, 1H), 3.61 (d, *J* = 16.7, 1H), 3.22 - 3.08 (m, 3H), 1.14 (d, *J* = 6.6, 3H), 0.99 (d, *J* = 6.5, 3H). ¹³C NMR (126 MHz, CDCl₃) δ 169.8, 157.5, 154.5, 153.6, 153.1, 133.0, 130.5, 129.7, 128.4, 123.1, 123.0, 122.2, 121.6, 119.6, 118.4, 116.2, 114.0, 68.9, 53.6, 50.9, 50.5, 43.3, 20.4, 20.3, 19.0 ppm; MS (ESI) m/z calculated [M+Na]⁺: 582.20; found [M+Na]⁺: 582.27.

### Example 147: 2-((2-(2-chlorophenoxy)-1-(1-(2-cyanoethyl)-1H-tetrazol-5-yl)ethyl)-(cyclopropyl)amino)-N-(4-phenoxyphenyl)acetamide

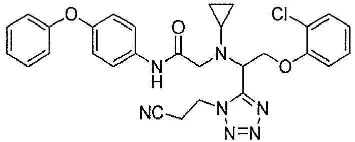

Yellow Solid, Yield: 277 mg (50 %); ¹H NMR (500 MHz, CDCl₃) δ 8.45 (s, 1H), 7.43 (d, *J* = 8.9, 2H), 7.35 (dd, *J* = 7.9, 1.4, 1H), 7.31 (t, *J* = 7.9, 2H), 7.24 (td, *J=* 8.3, 1.4, 1H), 7.08 (t, *J* = 7.4, 1H), 7.02 (d, *J* = 8.3, 1H), 6.99 - 6.90 (m, 5H), 5.03 - 4.95 (m, 1H), 4.94 - 4.91 (m, 1H), 4.50 (dd, *J* = 9.1, 4.5, 1H), 4.08 (d, *J* = 16.4, 1H), 3.57 (d, *J* = 16.4, 1H), 3.33 (t, *J* = 6.5, 1H), 3.14 (t, *J* = 6.6, 2H), 2.65 (t, *J=* 6.5, 1H), 2.42 - 2.30 (m, 1H), 0.70 - 0.51 (m, 3H), 0.28 (td, *J* = 10.3, 4.9, 1H). ¹³C NMR (126 MHz, CDCl₃) δ 169.0, 157.4, 153.8, 153.2, 152.9, 132.6, 130.4, 129.8, 128.4, 123.2, 122.7, 122.2, 122.0, 119.5, 118.5, 116.3, 113.7, 67.3, 57.4, 55.3, 43.2, 41.9, 34.7, 19.1, 18.9, 7.4, 7.2 ppm; MS (ESI) m/z calculated [M+Na]⁺: 580.18; found [M+Na]⁺: 580.26.

### Example 148: 2-((2-(2-chlorophenoxy)-1-(1-(2-cyanoethyl)-1H-tetrazol-5-yl)ethyl)-(cyclopentyl)amino)-N-(4-phenoxyphenyl)acetamide

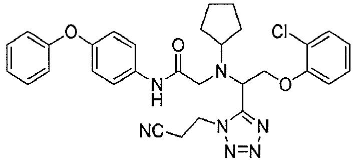

White Solid, Yield: 230 mg (39 %); ¹H NMR (500 MHz, CDCl₃) δ 8.64 (s, 1H), 7.48 (d, *J* = 8.9, 2H), 7.35 (dd, *J* = 7.9, 1.5, 1H), 7.33 - 7.27 (m, 2H), 7.26 - 7.19 (m, 1H), 7.07 (t, *J* = 7.4, 1H), 7.00 (d, *J* = 8.3, 1H), 6.98 - 6.91 (m, 5H), 5.20 - 5.06 (m, *J* = 13.8, 6.8, 1H), 4.96 - 4.85 (m, 2H), 4.72 (t, *J* = 9.0, 1H), 4.50 (dd, *J* = 9.7, 5.4, 1H), 4.08 (d, *J* = 16.7, 1H), 3.62 (d, *J* = 16.7, 1H), 3.27 (p, *J* = 7.8, 1H), 3.16 (t, *J* = 6.7, 2H), 1.81 - 1.70 (m, 2H), 1.69 - 1.58 (m, 2H), 1.58 - 1.49 (m, 2H), 1.48 - 1.36 (m, 2H). ¹³C NMR (126 MHz, CDCl₃) δ 169.7, 157.5, 153.7, 153.6, 153.2, 133.0, 130.4, 129.7, 128.4, 123.1, 122.8, 122.2, 121.7, 119.6, 118.4, 116.4, 113.8, 68.5, 62.2, 54.9, 52.5, 43.3, 30.9, 29.5, 24.1, 23.6, 18.9 ppm; MS (ESI) m/z calculated [M+Na]⁺: 608.22; found [M+Na]⁺: 608.30.

### Example 149: 2-((2-(2-chlorophenoxy)-1-(1-(2-cyanoethyl)-1H-tetrazol-5-yl)ethyl)-(pyridin-3-ylmethyl)amino)-N-(4-phenoxyphenyl)acetamide

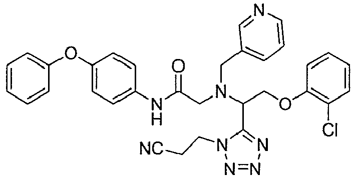

Yellow Solid, Yield: 183 mg (30 %); ¹H NMR (500 MHz, CDCl₃) δ 8.84 (s, 1H), 8.57 (s, 1H), 8.51 (d, *J* = 4.8, 1H), 8.43 (s, 1H), 7.68 (d, *J* = 7.8, 1H), 7.45 - 7.36 (m, 3H), 7.33 - 7.28 (m, *J* = 11.7, 4.3, 2H), 7.28 - 7.26 (m, 1H), 7.11 - 7.04 (m, 2H), 7.00 - 6.91 (m, 5H), 5.15 - 5.01 (m, *J=* 12.5, 11.5, 5.8, 2H), 4.93 (t, *J* = 9.6, 1H), 4.74 (t, *J* = 6.5, 1H), 4.58 (dd, *J* = 10.0, 4.6, 1H), 3.98 (d, *J* = 15.9, 1H), 3.89 (d, *J* = 13.7, 1H), 3.51 (d, *J* = 16.0, 1H), 3.10 (t, *J* = 6.7, 2H), 3.06 (td, *J* = 6.6, 1.1, 1H). ¹³C NMR (126 MHz, CDCl₃) δ 168.3, 157.4, 154.0, 153.3, 152.8, 150.4, 149.5, 143.1, 137.0, 132.9, 132.7, 130.6, 129.8, 128.5, 124.0, 123.3, 122.9, 122.0, 119.6, 118.6, 116.4, 113.6, 67.3, 55.4, 53.5, 52.4, 43.2, 19.0 ppm; MS (ESI) m/z calculated [M+H]⁺: 609.21; found [M+H]⁺: 609.27.

### Example 150: 2-((2-(2-chlorophenoxy)-1-(1-(2-cyanoethyl)-1H-tetrazol-5-yl)ethyl)(2-(pyridin-3-yl)ethyl)amino)-N-(4-phenoxyphenyl)acetamide

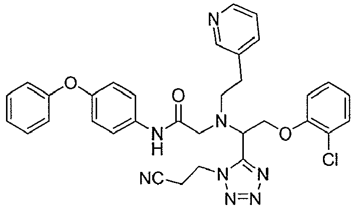

Yellow Solid, Yield: 380 mg (38 %); ¹H NMR (500 MHz, CDCl₃) δ 9.74 (s, 1H), 8.41 (d, *J* = 4.7, 1H), 7.60 (td, *J* = 7.7, 1.7, 1H), 7.52 (d, *J* = 8.9, 2H), 7.35 - 7.28 (m, 3H), 7.19 (td, *J* = 8.2, 1.5, 1H), 7.14 (t, *J* = 7.2, 2H), 7.08 (t, *J* = 7.4, 1H), 7.01 - 6.95 (m, 4H), 6.93 (td, *J* = 7.7, 1.1, 1H), 6.88 (dd, *J* = 8.2, 0.9, 1H), 4.79 (dd, *J* = 7.6, 6.0, 1H), 4.74 - 4.67 (m, 1H), 4.61 - 4.55 (m, 1H), 4.48 (dt, *J* = 14.0, 6.5, 1H), 4.38 (dd, *J* = 9.6, 5.9, 1H), 3.98 (d, *J* = 16.6, 1H), 3.61 (d, *J* = 16.6, 1H), 3.37 - 3.23 (m, 2H), 3.09 - 2.96 (m, 2H), 2.99 - 2.87 (m, 3H). ¹³C NMR (126 MHz, CDCl₃) δ 169.4, 159.5, 157.4, 153.7, 153.22, 152.9, 148.6, 137.1, 133.3, 130.4, 129.8, 128.1, 123.8, 123.2, 122.6, 122.3, 122.1, 121.9, 119.5, 118.5, 116.1, 113.5, 67.4, 57.3, 54.6, 51.4, 42.6, 36.1, 18.7 ppm; MS (ESI) m/z calculated [M+H]⁺: 623.22; found [M+H]⁺: 623.27.

### Example 151: 2-((2-(2-chlorophenoxy)-1-(1-(2-cyanoethyl)-1H-tetrazol-5-yl)ethyl)-(propyl)amino)-N-(4-phenoxyphenyl)acetamide

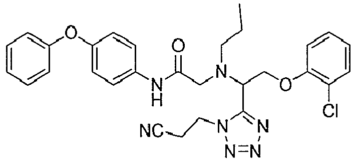

Colorless Solid, Yield: 75 mg (14 %); ¹H NMR (500 MHz, CDCl₃) δ 8.54 (s, 1H), 7.50 (d, *J* = 8.8, 2H), 7.36 (d, *J* = 7.9, 1H), 7.31 (t, *J* = 7.8, 2H), 7.27 - 7.22 (m, 1H), 7.08 (t, *J* = 7.4, 1H), 7.02 (d, *J* = 8.3, 1H), 6.98 - 6.94 (m, 5H), 5.17 (dt, *J* = 13.9, 6.9, 1H), 4.95 - 4.86 (m, 2H), 4.78 (t, *J* = 9.1, 1H), 4.50 (dd, *J* = 9.8, 5.1, 1H), 3.98 (d, *J* = 16.2, 1H), 3.58 (d, *J* = 16.2, 1H), 3.15 (t, *J* = 6.6, 2H), 2.81 - 2.69 (m, 1H), 2.63 - 2.51 (m, 1H), 1.58 - 1.43 (m, 2H), 0.84 (t, *J* = 7.3, 3H). ¹³C NMR (126 MHz, CDCl₃) δ 169.0, 157.4, 153.7, 153.3, 153.2, 132.9, 130.5, 129.8, 128.3, 123.2, 122.7, 121.7, 119.6, 118.5, 116.3, 113.7, 67.5, 56.5, 54.8, 53.4, 43.2, 21.3, 19.0, 11.6 ppm; MS (ESI) m/z calculated [M+H]⁺: 582.20; found [M+H]⁺: 582.35.

### Example 152: 2-((2-(2-chlorophenoxy)-1-(1-(2-cyanoethyl)-1H-tetrazol-5-yl)ethyl)-(phenethyl)amino)-N-(4-phenoxyphenyl)acetamide

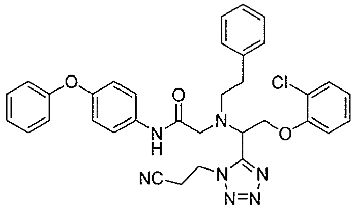

Yellow viscous liquid, Yield: 480 mg (52 %); ¹H NMR (500 MHz, CDCl₃) δ 8.28 (s, 1H), 7.44 (d, *J* = 8.9, 2H), 7.36 (dd, *J* = 7.9, 1.3, 1H), 7.31 (t, *J* = 8.0, 2H), 7.26 - 7.19 (m, 4H), 7.08 (t, *J* = 7.4, 1H), 7.03 - 6.99 (m, 3H), 6.98 - 6.93 (m, 5H), 4.90 - 4.74 (m, 3H), 4.50 (dd, *J* = 9.8, 4.7, 1H), 4.27 - 4.17 (m, 1H), 4.06 (d, *J* = 15.8, 1H), 3.69 (d, *J* = 15.8, 1H), 3.11 (dt, *J* = 11.8, 5.8, 1H), 3.06 - 2.97 (m, 1H), 2.85 (t, *J* = 6.7, 2H), 2.78 (t, *J* = 6.6, 2H). ¹³C NMR (126 MHz, CDCl₃) δ 168.6, 157.4, 153.8, 153.3, 152.7, 139.1, 132.8, 130.5, 129.8, 128.8, 128.6, 128.4, 126.6, 123.2, 122.7, 122.0, 121.7, 119.6, 118.6, 116.4, 113.5, 67.4, 56.5, 54.4, 51.7, 42.6, 34.0, 18.7 ppm; MS (ESI) m/z calculated [M+Na]⁺: 644.22; found [M+Na]⁺: 644.32.

### Example 153: 2-(methyl(4-phenyl-1-(1-(2,4,4-trimethylpentan-2-yl)-1H-tetrazol-5-yl)butyl)amino)-N-(4-phenoxyphenyl)acetamide

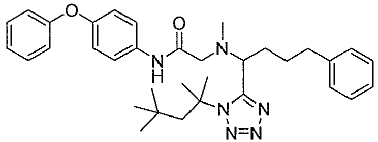

Yellow viscous liquid, Yield: 568 mg (65 %); ¹H NMR (500 MHz, CDCl₃) δ 8.57 (s, 1H), 7.42 (d, *J* = 8.8, 2H), 7.31 (t, *J* = 7.7, 2H), 7.25 (d, *J* = 7.5, 2H), 7.18 (t, *J* = 7.0, 1H), 7.13 (d, *J* = 7.6, 2H), 7.11 - 7.05 (m, 1H), 6.97 (dd, *J* = 8.8, 2.3, 4H), 4.35 (t, *J* = 7.2, 1H), 3.58 (d, *J* = 16.7, 1H), 3.36 (d, *J* = 16.7, 1H), 2.68 (t, *J* = 7.5, 2H), 2.52 (s, 3H), 2.04 (s, 2H), 1.85 (s, 3H), 1.83 (s, 3H), 1.74 - 1.65 (m, 2H), 1.65 - 1.53 (m, 2H), 0.82 (s, 9H). ¹³C NMR (126 MHz, CDCl₃) δ 171.1, 153.4, 133.0, 129.7, 128.4, 128.3, 128.3, 128.2, 126.1, 123.0, 121.1, 119.6, 118.3, 100.0, 99.0, 62.8, 60.3, 59.7, 57.2, 54.1, 40.3, 35.5, 31.5, 30.8, 30.5, 30.4, 29.7, 29.6.

### Example 154: 2-((2-(2-chlorophenoxy)-1-(1-(2-cyanoethyl)-1H-tetrazol-5-yl)ethyl)(3-phenylpropyl)amino)-N-(4-phenoxyphenyl)acetamide

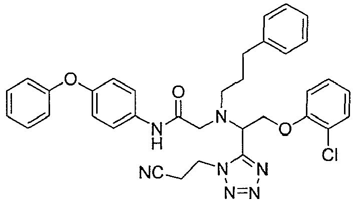

Purple viscous liquid, Yield: 230 mg (36 %); ¹H NMR (500 MHz, CDCl₃) δ 8.54 (s, 1H), 7.52 - 7.46 (m, 2H), 7.35 (dd, *J* = 7.9, 1.5, 1H), 7.33 - 7.29 (m, 2H), 7.23 (dd, *J* = 11.4, 4.3, 3H), 7.18 - 7.14 (m, 2H), 7.08 (d, *J* = 7.3, 3H), 6.98 - 6.95 (m, 5H), 5.02 (dt, *J* = 14.0, 6.9, 1H), 4.86 (dd, *J* = 8.5, 5.0, 1H), 4.82 - 4.71 (m, 2H), 4.50 - 4.42 (m, 1H), 3.97 (d, *J* = 16.2, 1H), 3.52 (d, *J* = 16.2, 1H), 3.07 (t, *J* = 6.6, 2H), 2.89 - 2.79 (m, 2H), 2.72 - 2.60 (m, 2H), 1.92 - 1.84 (m, 1H), 1.83 - 1.75 (m, 1H). ¹³C NMR (126 MHz, CDCl₃) δ 168.9, 157.4, 153.8, 153.2, 152.9, 141.1, 132.8, 130.5, 129.8, 128.8, 128.7, 128.5, 128.4, 128.3, 128.1, 126.1, 123.2, 122.8, 121.8, 119.6, 118.5, 116.3, 113.7, 67.4, 56.4, 54.7, 51.1, 43.1, 33.1, 29.8, 19.0 ppm; MS (ESI) m/z calculated [M+Na]⁺: 658.23; found [M+Na]⁺: 658.32

### Example 155: 2-(benzyl(2-(2-chlorophenoxy)-1-(1-(2-cyanoethyl)-1H-tetrazol-5-yl)ethyl)amino)-N-(4-phenoxyphenyl)acetamide

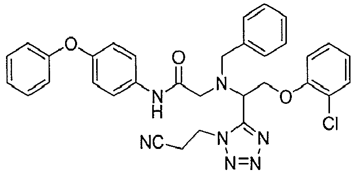

White solid, Yield: 400 mg (55 %); ¹H NMR (500 MHz, CDCl₃) δ 8.37 (s, 1H), 7.43 - 7.40 (m, 2H), 7.39 - 7.34 (m, 5H), 7.32 - 7.27 (m, 3H), 7.25 - 7.21 (m, 1H), 7.07 (t, *J* = 7.4, 1H), 7.03 (dd, *J* = 8.2, 0.9, 1H), 6.97 - 6.91 (m, 5H), 4.94 (dd, *J* = 8.7, 4.7, 1H), 4.88 (t, *J* = 9.2, 1H), 4.74 (dt, *J* = 14.0, 7.0, 1H), 4.55 - 4.46 (m, 2H), 4.12 (d, *J* = 14.3, 1H), 3.90 (d, *J* = 16.1, 1H), 3.81 (d, *J* = 13.3, 1H), 3.57 (d, *J* = 16.1, 1H), 3.06 - 2.91 (m, 2H). ¹³C NMR (126 MHz, CDCl₃) δ 168.4, 157.4, 153.8, 153.3, 152.8, 137.0, 132.7, 130.5, 129.8, 129.4, 129.1, 128.3, 128.3, 123.2, 122.8, 121.9, 119.5, 118.5, 116.2, 113.6, 67.3, 55.9, 55.1, 52.5, 42.9, 18.7 ppm; MS (ESI) m/z calculated [M+Na]⁺: 630.20; found [M+Na]⁺: 630.29.

### Example 156: 2-((2-(2-chlorophenoxy)-1-(1-(2-cyanoethyl)-1H-tetrazol-5-yl)ethyl)-(methyl)amino)-N-(4-phenoxyphenyl)acetamide (A)

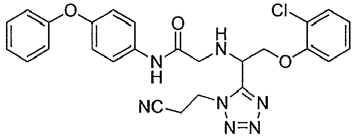

Brown liquid, Yield: 178 mg (17 %); ¹H NMR (500 MHz, CDCl₃) δ 8.38 (s, 1H), 7.48 (d, *J* = 8.9, 2H), 7.36 - 7.30 (m, 3H), 7.23 (td, *J* = 8.0, 1.6, 1H), 7.09 (t, *J* = 7.4, 1H), 7.01 - 6.96 (m, 4H), 6.96 - 6.88 (m, 2H), 5.13 - 5.00 (m, 1H), 4.97 - 4.87 (m, 1H), 4.74 (s, 1H), 4.55 - 4.48 (m, 1H), 4.41 - 4.30 (m, 1H), 3.64 (d, *J* = 16.5, 1H), 3.45 (d, *J* = 17.7, 1H), 3.16 (t, *J* = 6.6, 2H), 2.81 (s, 1H). ¹³C NMR (126 MHz, CDCl₃) δ 168.28, 154.95, 153.77, 152.98, 132.67, 130.58, 129.74, 128.21, 123.16, 123.13, 121.64, 119.59, 118.50, 116.34, 114.19, 70.99, 53.07, 51.08, 43.55, 18.95 ppm. MS (ESI) m/z calculated [M+Na]⁺: 540.15; found [M+Na]⁺: 540.21.

### Method 8: Isocyanide deprotection of Ugi-product

### Example 157: 2-((2-(2-(benzyloxy)phenoxy)-1-(1H-tetrazol-5-yl)ethyl)(methyl)amino)-N-(4-phenoxyphenyl)acetamide

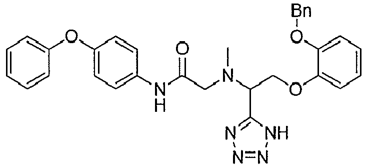

White solid, Yield: 10 mg (63 %); ¹H NMR (500 MHz, CDCl₃) δ 8.40 (s, 1H), 7.40 (dd, *J* = 6.5, 2.9, 2H), 7.37 - 7.29 (m, 7H), 7.08 (dd, *J* = 14.1, 6.7, 2H), 7.04 - 6.99 (m, 3H), 6.99 - 6.91 (m, 4H), 5.07 (s, 2H), 4.95 - 4.87 (m, 1H), 4.73 (m, 1H), 4.54 (dd, *J* = 9.3, 5.0, 1H), 3.83 (d, *J* = 14.2, 1H), 3.43 (d, *J* = 14.2, 1H), 2.37 (s, 3H). ¹³C NMR (126 MHz, CDCl₃) δ 168.0, 157.5, 153.7, 153.3, 147.9, 147.3, 135.8, 132.8, 129.7, 128.9, 128.9, 128.9, 123.1, 122.2, 122.0, 121.8, 119.5, 118.4, 116.4, 112.9, 112.8, 71.3, 66.5, 60.3, 54.8, 43.1 ppm; MS (ESI) m/z calculated [M-H]⁻: 549.61; found [M+H]⁺: 549.11.

### Example 158: 2-(methyl(2-(2-nitrophenoxy)-1-(1H-tetrazol-5-yl)ethyl)amino)-N-(4-phenoxyphenyl)acetamide

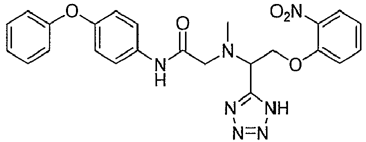

White solid, Yield: 30 mg (88 %); ¹H NMR (500 MHz, CDCl₃) δ 9.28 (s, 1H), 7.86 (d, *J* = 8.0, 1H), 7.56 (t, *J* = 7.6, 1H), 7.49 (d, *J* = 8.1, 2H), 7.30 (t, *J* = 7.8, 2H), 7.20 (d, *J* = 8.2, 1H), 7.07 (t, *J* = 7.2, 2H), 6.98 - 6.87 (m, 4H), 4.79 (s, 1H), 4.72 (t, *J* = 8.9, 1H), 4.63 (d, *J* = 6.4, 1H), 3.63 (d, *J* = 16.8, 1H), 3.52 (d, *J* = 18.4, 1H), 2.50 (s, 2H). ¹³C NMR (126 MHz, CDCl₃) δ 169.9, 157.3, 154.1, 151.5, 139.5, 135.0, 132.3, 129.7, 126.1, 123.2, 122.3, 121.6, 119.4, 118.5, 114.8, 67.2, 58.7, 58.0, 39.2 ppm; MS (ESI) m/z calculated [M+H]⁺: 490.48; found [M+H]⁺: 490.30.

### Example 159: 2-((1-(1H-tetrazol-5-yl)-2-(m-tolyloxy)ethyl)(methyl)amino)-N-(4-phenoxyphenyl)acetamide

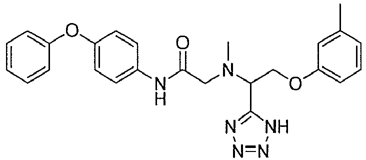

White solid, Yield: 30 mg (93 %); ¹H NMR (500 MHz, CDCl₃) δ 9.73 (s, 1H), 7.49 (d, *J* = 8.8, 2H), 7.30 (t, *J* = 7.9, 3H), 7.14 (t, *J* = 7.8, 1H), 7.08 (t, *J* = 7.3, 1H), 7.00 - 6.88 (m, 4H), 6.83 - 6.68 (m, 3H), 4.70 (dd, *J* = 9.6, 3.7, 1H), 4.62 (t, *J* = 10.0, 1H), 4.39 (dd, *J* = 10.2, 3.8, 1H), 3.58 (s, 2H), 2.47 (s, 3H), 2.27 (s, 3H). ¹³C NMR (126 MHz, CDCl₃) δ 170.4, 157.9, 157.2, 154.4, 139.9, 132.1, 129.9, 129.8, 129.5, 123.3, 122.7, 122.5, 119.5, 119.2, 118.6, 115.6, 111.6, 65.7, 58.3, 58.1, 38.9, 21.6 ppm; MS (ESI) m/z calculated [M+H]⁺: 459.51; found [M+H]⁺: 459.36.

### Example 160: 2-((2-(4-methoxyphenoxy)-1-(1H-tetrazol-5-yl)ethyl)(methyl)amino)-N-(4-phenoxyphenyl)acetamide

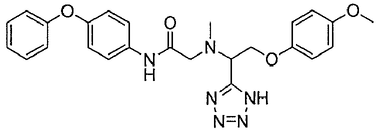

White solid, Yield: 40 mg (94 %); ¹H NMR (500 MHz, CDCl₃) δ 9.76 (s, 1H), 7.47 (d, *J* = 8.8, 2H), 7.30 (dd, *J* = 8.3, 7.6, 2H), 7.08 (t, *J* = 7.4, 1H), 6.98 - 6.90 (m, 4H), 6.86 (d, *J* = 9.1, 2H), 6.78 (d, *J* = 9.1, 2H), 4.70 (dd, *J* = 9.6, 4.0, 1H), 4.58 (t, *J* = 10.0, 1H), 4.35 (dd, *J* = 10.4, 4.1, 1H), 3.72 (s, 3H), 3.58 (d, *J* = 5.1, 2H), 2.48 (s, 3H). ¹³C NMR (126 MHz, CDCl₃) δ 170.3, 157.1, 154.7, 154.4, 151.9, 132.0, 129.8, 123.4, 122.4, 119.4, 118.7, 115.9, 114.9, 66.8, 58.2, 58.0, 55.7, 39.1 ppm; MS (ESI) m/z calculated [M+H]⁺: 475.51; found [M+H]⁺: 475.22.

### Example 161: 2-((2-(2-methoxyphenoxy)-1-(1H-tetrazol-5-yl)ethyl)(methyl)amino)-N-(4-phenoxyphenyl)acetamide

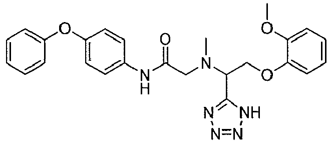

White solid, Yield: 40 mg (93 %); ¹H NMR (500 MHz, CDCl₃) δ 9.83 (s, 1H), 7.52 (d, *J* = 8.8, 2H), 7.30 (t, *J* = 7.9, 2H), 7.07 (t, *J* = 7.4, 1H), 7.04 - 6.98 (m, 2H), 6.92 (dd, *J* = 16.7, 7.7, 5H), 4.70 (dd, *J* = 7.8, 5.1, 1H), 4.65 - 4.56 (m, 1H), 4.40 (dd, *J* = 10.1, 5.0, 1H), 3.78 (s, 3H), 3.61 - 3.48 (m, 2H), 2.50 (s, 3H). ¹³C NMR (126 MHz, CDCl₃) δ 169.8, 157.4, 153.9, 149.4, 147.1, 132.8, 129.8, 123.2, 122.4, 121.5, 119.4, 118.5, 115.5, 111.8, 67.8, 58.5, 57.8, 55.7, 39.3 ppm; MS (ESI) m/z calculated [M+H]⁺: 475.51; found [M+H]⁺: 475.28.

### Example 162: 2-((2-(3-methoxyphenoxy)-1-(1H-tetrazol-5-yl)ethyl)(methyl)amino)-N-(4-phenoxyphenyl)acetamide

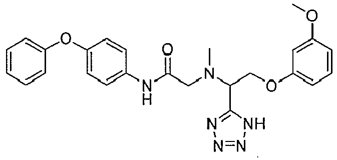

White solid, Yield: 80 mg (99 %); ¹H NMR (500 MHz, CDCl₃) δ 9.74 (s, 1H), 7.48 (d, *J* = 8.8, 2H), 7.29 (t, *J* = 7.9, 2H), 7.13 (t, *J* = 8.2, 1H), 7.07 (t, *J* = 7.4, 1H), 6.92 (t, *J* = 8.6, 4H), 6.56 - 6.42 (m, 3H), 4.72 (dd, *J* = 9.4, 4.0, 1H), 4.62 (t, *J* = 10.0, 1H), 4.40 (dd, *J* = 10.3, 3.9, 1H), 3.68 (s, 3H), 3.60 (d, *J* = 6.1, 2H), 2.47 (s, 3H). ¹³C NMR (126 MHz, CDCl₃) δ 170.3, 161.0, 159.0, 157.2, 154.4, 132.0, 130.3, 129.8, 123.4, 122.5, 119.5, 118.6, 107.5, 106.7, 101.3, 65.8, 58.3, 58.1, 55.3, 39.0 ppm; MS (ESI) m/z calculated [M+H]⁺: 475.51; found [M+H]⁺: 475.41.

### Example 163: 2-((1-(1H-tetrazol-5-yl)-2-(p-tolyloxy)ethyl)(methyl)amino)-N-(4-phenoxyphenyl)acetamide

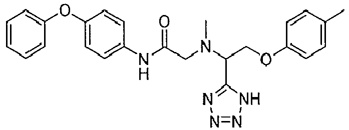

White solid, Yield: 20 mg (89 %); ¹H NMR (500 MHz, CDCl₃) δ 9.74 (s, 1H), 7.48 (d, *J* = 8.8, 2H), 7.31 (t, *J* = 8.0, 2H), 7.09 (t, *J* = 7.4, 1H), 7.06 (d, *J* = 8.4, 2H), 6.95 (t, *J* = 8.2, 4H), 6.83 (d, *J* = 8.5, 2H), 4.69 (dd, *J* = 9.8, 3.9, 1H), 4.61 (t, *J* = 10.1, 1H), 4.36 (dd, *J* = 10.3, 3.9, 1H), 3.57 (d, *J* = 3.2, 2H), 2.48 (s, 3H), 2.26 (s, 3H). ¹³C NMR (126 MHz, CDCl₃) δ 170.5, 157.1, 155.8, 154.5, 131.9, 131.3, 130.2, 129.8, 123.4, 122.5, 119.5, 118.7, 114.7, 66.1, 58.1, 57.9, 39.0, 20.5 ppm; MS (ESI) m/z calculated [M+H]⁺: 459.51; found [M+H]⁺: 459.23.

### Example 164: 2-((2-(2,5-dichlorophenoxy)-1-(1H-tetrazol-5-yl)ethyl)(methyl)amino)-N-(4-phenoxyphenyl)acetamide

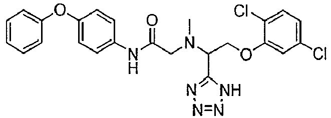

White solid, Yield: 30 mg (83 %); ¹H NMR (500 MHz, CDCl₃) δ 9.49 (s, 1H), 7.47 (d, *J* = 8.6, 2H), 7.30 (t, *J* = 7.9, 2H), 7.24 (d, *J* = 8.5, 1H), 7.08 (t, *J* = 7.4, 1H), 7.04 (d, *J* = 1.7, 1H), 6.99 - 6.87 (m, 5H), 4.78 (d, *J* = 5.6, 1H), 4.65 (t, *J* = 9.9, 1H), 4.49 (dd, *J* = 10.0, 3.7, 1H), 3.67 (d, *J* = 17.1, 1H), 3.58 (d, *J* = 17.1, 1H), 2.50 (s, 3H). ¹³C NMR (126 MHz, CDCl₃) δ 157.2, 154.4, 154.0, 133.5, 131.9, 130.9, 129.8, 123.3, 122.6, 122.6, 121.3, 119.4, 118.6, 114.6, 67.1, 58.6, 58.2, 38.9 ppm; MS (ESI) m/z calculated [M+H]⁺: 514.38; found [M+H]⁺: 514.18.

### Example 165: 2-((furan-2-yl(1H-tetrazol-5-yl)methyl)(methyl)amino)-N-(4-phenoxyphenyl)acetamide

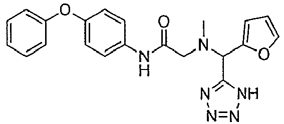

White solid, Yield: 100 mg (95 %); ¹H NMR (500 MHz, CDCl₃) δ 7.41 (d, *J* = 1.3, 1H), 7.34 - 7.30 (m, 2H), 7.22 - 7.18 (m, 2H), 7.10 (t, *J* = 7.4, 1H), 7.04 - 6.95 (m, 3H), 6.94 - 6.89 (m, 2H), 6.40 (d, *J* = 3.2, 1H), 6.32 (dd, *J* = 3.2, 1.8, 1H), 5.41 (s, 1H), 3.81 (dd, *J* = 14.6, 1.0, 1H), 3.41 (dd, *J* = 14.6, 1.0, 1H), 2.43 (s, 3H) ppm.

### Example 166: 2-((2-(benzyloxy)-1-(1H-tetrazol-5-yl)ethyl)(methyl)amino)-N-(4-phenoxyphenyl)acetamide

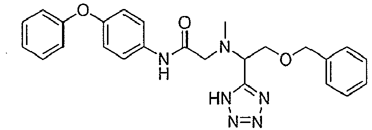

White Solid, Yield: 246 mg (89 %); 1H NMR (500 MHz, CDCl3) δ 9.59 (s, 1H), 7.43 - 7.30 (m, 8H), 7.29 - 7.25 (m, 6H), 7.25 - 7.20 (m, 2H), 7.10 (t, J = 7.4, 1H), 6.96 (d, J = 7.8, 2H), 6.92 - 6.84 (m, 2H), 5.30 (s, 1H), 4.74 (d, J = 3.0, 2H), 4.48 (dd, J = 9.0, 4.6, 1H), 4.25 - 4.16 (m, 1H), 3.94 (dd, J = 10.3, 4.7, 1H), 3.47 (s, 2H), 2.44 (s, 3H), 2.17 (s, 1H). MS (ESI) m/z calculated [M+H]+: 459,51; found [M+H]+: 459,21.

### Example 167: 2-((2-(2-chlorophenoxy)-1-(1H-tetrazol-5-yl)ethyl)(methyl)amino)-N-(4-phenoxyphenyl)acetamide

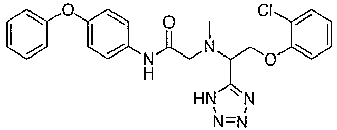

White solid, Yield: 94 mg (98 %); ¹H NMR (500 MHz, CDCl₃) δ 9.42 (s, 1H), 7.51 (d, *J* = 8.9, 2H), 7.37 (dd, *J* = 7.9, 1.4, 1H), 7.35 - 7.29 (m, 2H), 7.25 - 7.19 (m, 1H), 7.09 (t, *J* = 7.4, 1H), 7.05 (d, *J* = 8.2, 1H), 7.01 - 6.92 (m, 5H), 4.76 (dd, *J* = 9.4, 4.3, 1H), 4.65 (t, *J* = 9.9, 1H), 4.52 (dd, *J* = 10.3, 4.3, 1H), 3.71 - 3.53 (m, 2H), 2.54 (s, 3H). ¹³C NMR (126 MHz, CDCl₃) δ 170.5, 157.2, 154.4, 153.5, 132.0, 130.5, 129.8, 128.2, 123.3, 122.9, 122.6, 119.4, 118.7, 114.4, 77.3, 77.0, 76.8, 67.1, 58.4, 58.0, 39.1 ppm; MS (ESI) m/z calculated [M+H]⁺: 479.13; found [M+H]⁺: 479.26.

### Example 168: 2-(methyl(2-phenoxy-1-(1H-tetrazol-5-yl)ethyl)amino)-N-(4-phenoxy-phenyl)acetamide

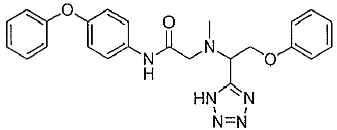

White Solid, Yield: 150 mg (94 %); ¹H NMR (500 MHz, CDCl₃) δ 9.66 (s, 1H), 7.50 (d, *J* = 8.9, 2H), 7.36 - 7.26 (m, 4H), 7.10 (t, *J* = 7.4, 1H), 7.01 - 6.92 (m, 7H), 4.70 (dd, *J* = 9.8, 3.7, 1H), 4.65 (t, *J* = 9.9, 1H), 4.41 (dd, *J* = 10.0, 3.7, 1H), 3.57 (d, *J* = 4.9, 2H), 2.49 (s, 3H); ¹³C NMR (126 MHz, CDCl₃) δ 170.5, 157.9, 157.1, 154.5, 131.9, 129.8, 129.8, 123.4, 122.5, 121.9, 119.5, 118.7, 114.8, 68.1, 65.9, 58.1, 57.9, 39.1. MS (ESI) m/z calculated [M+H]⁺: 445.49; found [M+H]⁺: 445.29.

### Example 169: 2-(methyl(4-phenyl-1-(1H-tetrazol-5-yl)butyl)amino)-N-(4-phenoxy-phenyl)acetamide

Procedure: 2-(methyl(4-phenyl-1-(1-(2,4,4-trimethylpentan-2-yl)-1H-tetrazol-5-yl)butyl)amino)-N-(4-phenoxyphenyl)acetamide (269 mg, 0.47 mmol) was added to 5 ml 4 M HCl in Dioxane in a 25 ml RBF and refluxed for overnight. Then solvent was removed under reduced pressure and residue was dissolved in water and extracted in DCM. The solvent was removed under reduced pressure, the residue was purified by flash column chromatography on silica gel and eluted with methanol:DCM to afford product.

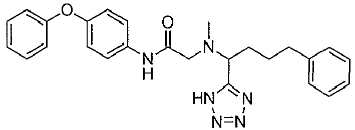

White solid, Yield: 100 mg (47 %); ¹H NMR (500 MHz, CDCl₃) δ 9.16 (s, 1H), 7.50 (d, *J* = 8.8, 2H), 7.33 (t, *J* = 7.7, 2H), 7.23 (d, *J* = 7.5, 2H), 7.20 - 7.12 (m, 4H), 7.10 (t, *J* = 7.8, 1H), 7.04 - 6.93 (m, 4H), 4.26 (t, *J* = 7.6, 1H), 3.38 (d, *J* = 16.8, 1H), 3.28 (d, *J* = 16.8, 1H), 2.76 - 2.60 (m, 3H), 2.39 (s, 3H), 2.23 - 2.02 (m, 3H), 1.87 - 1.63 (m, 3H); ¹³C NMR (126 MHz, CDCl₃) δ 153.9, 140.5, 129.8, 128.5, 128.3, 126.1, 123.2, 121.6, 119.6, 118.5, 72.3, 71.1, 61.7, 42.89, 30.6, 29.9 ppm; MS (ESI) m/z calculated [M+H]⁺: 457.23; found [M+H]⁺: 457.23.

### Example 170: N-(3-chloro-4-(3-chlorophenoxy)phenyl)-2-((2-(2-chlorophenoxy)-1-(1H-tetrazol-5-yl)ethyl)(methyl)amino)acetamide

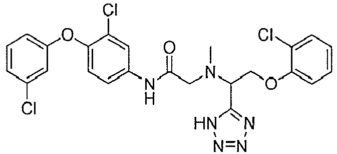

White Solid, Yield: 46 mg (65 %); ¹H NMR (500 MHz, CDCl₃) δ 9.74 (s, 1H), 7.77 (d, *J* = 2.1, 1H), 7.42 (dd, *J* = 8.8, 2.1, 1H), 7.33 (dd, *J* = 7.9, 1.2, 1H), 7.20 (t, *J* = 8.2, 2H), 7.07 - 7.00 (m, 2H), 6.97 - 6.90 (m, 2H), 6.85 (t, *J* = 2.1, 1H), 6.76 (dd, *J* = 8.2, 2.1, 1H), 4.83 (dd, *J* = 9.0, 4.0, 1H), 4.66 (t, *J* = 9.8, 1H), 4.54 (dd, *J* = 10.3, 4.0, 1H), 3.75 (d, *J* = 17.2, 1H), 3.64 (d, *J* = 17.2, 1H), 2.55 (s, 3H); ¹³C NMR (126 MHz, CDCl₃) δ 170.6, 157.9, 153.4, 148.2, 135.1, 134.2, 130.5, 130.5, 128.2, 126.6, 123.3, 122.9, 122.9, 122.0, 120.4, 117.4, 115.4, 114.3, 66.9, 58.6, 58.4, 39.2 ppm. MS (ESI) m/z calculated [M+H]⁺: 547.07; found [M+H]⁺: 547.22.

### Example 171: 2-((2-(2-chlorophenoxy)-1-(1H-tetrazol-5-yl)ethyl)(cyclobutyl)amino)-N-(4-phenoxyphenyl)acetamide

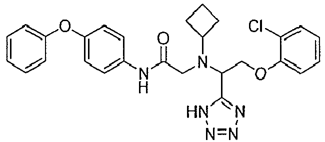

White solid, Yield: 30 mg (88 %); ¹H NMR (500 MHz, CDCl₃) δ 9.69 (s, 1H), 7.45 (d, *J* = 8.9, 2H), 7.38 - 7.28 (m, 4H), 7.17 (t, *J* = 7.1, 1H), 7.08 (t, *J* = 7.4, 1H), 6.98 - 6.91 (m, 4H), 6.90 (d, *J* = 8.8, 2H), 4.81 (dd, *J* = 9.8, 4.0, 1H), 4.55 (t, *J* = 10.1, 1H), 4.42 (dd, *J* = 10.3, 4.1, 1H), 3.69 (d, *J* = 17.9, 1H), 3.65 - 3.55 (m, 1H), 3.42 (d, *J* = 17.8, 1H), 2.14 - 1.90 (m, 4H), 1.77 - 1.65 (m, 1H), 1.64 - 1.52 (m, 1H). ¹³C NMR (126 MHz, CDCl₃) δ 172.0, 157.3, 154.1, 153.4, 132.2, 130.5, 129.7, 128.1, 123.2, 122.9, 122.6, 122.4, 119.4, 118.5, 114.0, 67.2, 56.5, 54.9, 51.4, 28.5, 28.2, 14.4ppm; MS (ESI) m/z calculated [M+H]⁺: 519.99; found [M+H]⁺: 519.42.

### Example 172: 2-(butyl(2-(2-chlorophenoxy)-1-(1H-tetrazol-5-yl)ethyl)amino)-N-(4-phenoxyphenyl)acetamide

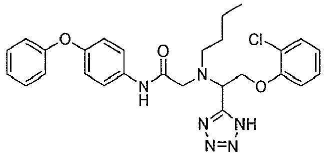

White solid, Yield: 40 mg (85 %); ¹H NMR (500 MHz, CDCl₃) δ 9.75 (s, 1H), 7.46 (d, *J* = 8.9, 2H), 7.36 - 7.28 (m, 3H), 7.19 (t, *J* = 7.2, 1H), 7.08 (t, *J* = 7.4, 1H), 7.00 (d, *J* = 8.0, 1H), 6.97 - 6.88 (m, 5H), 4.87 (dd, *J* = 9.9, 3.9, 1H), 4.64 (t, *J* = 10.2, 1H), 4.43 (dd, *J* = 10.4, 4.0, 1H), 3.74 (d, *J* = 17.8, 1H), 3.44 (d, *J* = 17.8, 1H), 2.91 - 2.79 (m, 1H), 2.75 - 2.57 (m, 1H), 1.69 - 1.49 (m, 2H), 1.49 - 1.38 (m, 1H), 1.38 - 1.29 (m, 1H), 0.88 (t, *J* = 7.3, 3H). ¹³C NMR (126 MHz, CDCl₃) δ 171.5, 157.2, 156.6, 154.3, 153.5, 132.1, 130.5, 129.8, 128.1, 123.3, 122.9, 122.7, 122.7, 119.4, 118.6, 114.2, 67.2, 56.4, 55.5, 52.8, 30.4, 20.2, 13.9 ppm; MS (ESI) m/z calculated [M+H]⁺: 507.18; found [M+H]⁺: 507.39.

### Example 173: 2-((2-(2-chlorophenoxy)-1-(1H-tetrazol-5-yl)ethyl)(phenethyl)amino)-N-(4-phenoxyphenyl)acetamide

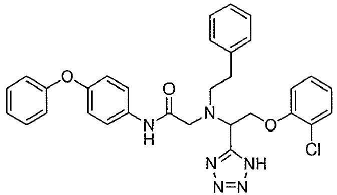

White solid, Yield: 386 mg (88 %); ¹H NMR (500 MHz, CDCl₃) δ 9.29 (d, *J* = 21.2, 1H), 7.35 - 7.28 (m, 5H), 7.23 - 7.18 (m, 3H), 7.17 - 7.13 (m, 3H), 7.10 - 7.06 (m, 1H), 6.98 - 6.91 (m, 4H), 6.90 - 6.87 (m, 2H), 4.88 (dd, *J* = 9.5, 4.2, 1H), 4.57 (t, *J=* 10.0, 1H), 4.39 (dd, *J=* 10.4, 4.2, 1H), 3.77 (d, *J* = 17.8, 1H), 3.53 (d, *J* = 17.8, 1H), 3.16 (dt, *J* = 14.3, 7.3, 1H), 3.02 - 2.92 (m, 1H), 2.88 (t, *J* = 6.9, 2H). ¹³C NMR (126 MHz, CDCl₃) δ 171.2, 157.2, 154.3, 153.4, 138.8, 131.8, 130.4, 129.8, 128.7, 128.6, 128.1, 126.5, 123.3, 123.0, 122.8, 119.3, 118.6, 114.3, 67.5, 56.5, 55.5, 54.1, 34.7 ppm; MS (ESI) m/z calculated [M+H]⁺: 568.20; found [M+H]⁺: 568.27.

### Example 174: 2-((2-(2-chlorophenoxy)-1-(1H-tetrazol-5-yl)ethyl)(3-phenylpropyl)-amino)-N-(4-phenoxyphenyl)acetamide

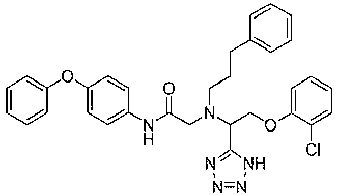

White solid, Yield: 130 mg (62 %); ¹H NMR (500 MHz, CDCl₃) δ 11.08 (s, 1H), 9.72 (s, 1H), 7.43 (d, *J* = 8.9, 2H), 7.33 - 7.27 (m, 3H), 7.20 - 7.15 (m, 3H), 7.12 (d, *J* = 7.2, 1H), 7.10 - 7.06 (m, 3H), 6.96 (d, *J* = 8.3, 1H), 6.94 - 6.90 (m, 3H), 6.88 (d, *J* = 8.8, 2H), 4.84 (dd, *J* = 9.9, 3.8, 1H), 4.60 (t, *J* = 10.2, 1H), 4.40 (dd, *J* = 10.4, 3.8, 1H), 3.71 (d, *J* = 17.8, 1H), 3.43 (d, *J* = 17.7, 1H), 2.88 (dt, *J* = 14.6, 7.5, 1H), 2.75 - 2.59 (m, 3H), 1.95 - 1.82 (m, 2H). ¹³C NMR (126 MHz, CDCl₃) δ 171.4, 157.3, 156.6, 154.3, 153.5, 141.3, 132.1, 130.5, 129.8, 128.5, 128.3, 128.1, 126.0, 123.3, 122.8, 122.7, 119.4, 118.6, 114.2, 67.2, 56.6, 55.6, 52.5, 33.2, 29.9 ppm; MS (ESI) m/z calculated [M+H]⁺: 583.21; found [M+H]⁺: 583.36.

### Example 175: 2-(benzyl(2-(2-chlorophenoxy)-1-(1H-tetrazol-5-yl)ethyl)amino)-N-(4-phenoxyphenyl)acetamide

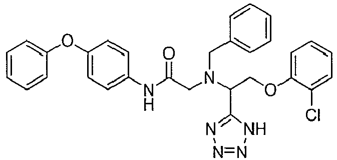

White solid, Yield: 300 mg (82 %); ¹H NMR (500 MHz, CDCl₃) δ 9.55 (s, 1H), 7.45 (d, *J* = 7.3, 2H), 7.37 - 7.32 (m, 4H), 7.31 - 7.28 (m, 3H), 7.26 - 7.22 (m, 1H), 7.21 - 7.15 (m, 1H), 7.09 (t, *J* = 7.4, 1H), 7.00 - 6.96 (m, 1H), 6.95 - 6.92 (m, 3H), 6.91 - 6.88 (m, 2H), 4.89 (dd, *J* = 10.2, 4.1, 1H), 4.70 (t, *J* = 10.4, 1H), 4.45 (dd, *J* = 10.5, 4.1, 1H), 4.10 (d, *J* = 13.3, 1H), 3.78 - 3.63 (m, 2H), 3.47 (d, *J* = 17.5, 1H). ¹³C NMR (126 MHz, CDCl₃) δ 171.2, 157.2, 154.3, 153.4, 138.9, 131.8, 130.5, 129.8, 128.7, 128.6, 128.1, 126.5, 123.3, 122.8, 119.3, 118.6, 114.3, 67.5, 56.5, 55.5, 34.7 ppm; MS (ESI) m/z calculated [M+H]⁺: 555.18; found [M+H]⁺: 555.34.

### Example 176: 2-((2-(2-chlorophenoxy)-1-(1H-tetrazol-5-yl)ethyl)(2-(pyridin-3-yl)ethyl)-amino)-N-(4-phenoxyphenyl)acetamide

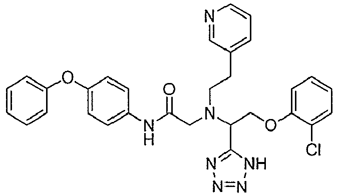

Yellow solid, Yield: 240 mg (69 %); ¹H NMR (500 MHz, CDCl₃) δ 9.29 (s, 1H), 8.67 (d, *J* = 4.6, 1H), 7.71 (t, *J* = 7.4, 1H), 7.35 - 7.25 (m, 5H), 7.23 - 7.13 (m, 3H), 7.06 (t, *J* = 7.4, 1H), 6.96 (d, *J* = 8.1, 1H), 6.93 - 6.87 (m, 3H), 6.82 (d, *J* = 8.8, 2H), 4.92 (d, *J* = 6.2, 1H), 4.63 (dd, *J* = 10.0, 3.1, 1H), 4.53 (t, *J* = 9.9, 1H), 3.93 (d, *J* = 17.7, 1H), 3.69 (d, *J* = 17.6, 1H), 3.40 (t, *J* = 5.3, 2H), 3.31 - 3.10 (m, 2H). ¹³C NMR (126 MHz, CDCl₃) δ 169.9, 158.1, 157.5, 156.0, 153.5, 153.4, 146.4, 139.5, 132.7, 130.4, 129.7, 128.1, 125.3, 123.1, 122.8, 122.6, 122.4, 121.6, 119.3, 118.4, 113.5, 67.3, 58.3, 57.7, 34.4 ppm; MS (ESI) m/z calculated [M+H]⁺: 570.19; found [M+H]⁺: 570.35.

### Example 177: 2-((2-(2-chlorophenoxy)-1-(1H-tetrazol-5-yl)ethyl)(pyridin-3-ylmethyl)-amino)-N-(4-phenoxyphenyl)acetamide

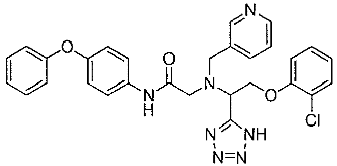

Yellow solid, Yield: 70 mg (46 %); ¹H NMR (500 MHz, CDCl₃) δ 10.00 (s, 1H), 9.62 (s, 1H), 8.81 (s, 1H), 8.56 (d, *J* = 4.1, 1H), 8.02 (d, *J* = 7.8, 1H), 7.43 - 7.39 (m, 2H), 7.35 (dd, *J* = 7.9, 1.4, 1H), 7.33 - 7.28 (m, 2H), 7.24 - 7.16 (m, 1H), 7.08 (t, *J* = 7.4, 1H), 7.00 (d, *J* = 8.2, 1H), 6.96 - 6.91 (m, 3H), 6.87 (d, *J* = 8.9, 2H), 4.87 (dd, *J* = 10.1, 3.8, 1H), 4.72 (t, *J* = 10.4, 1H), 4.57 (dd, *J* = 10.4, 3.8, 1H), 4.17 (d, *J* = 13.9, 1H), 3.91 (d, *J* = 13.9, 1H), 3.83 (d, *J* = 17.3, 1H), 3.46 (d, *J* = 17.2, 1H). ¹³C NMR (126 MHz, CDCl₃) δ 170.0, 157.8, 157.3, 154.0, 153.3, 149.1, 147.8, 138.6, 134.2, 132.3, 130.5, 129.7, 128.2, 124.4, 123.2, 122.6, 122.4, 122.3, 119.4, 118.5, 113.6, 66.9, 55.7, 55.1, 54.0 ppm; MS (ESI) m/z calculated [M+H]⁺: 556.18; found [M+H]⁺: 556.33.

### Example 178: 2-((2-(2-chlorophenoxy)-1-(1H-tetrazol-5-yl)ethyl)(cyclopropyl)amino)-N-(4-phenoxyphenyl)acetamide

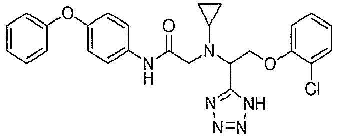

Yellow solid, Yield: 180 mg (72 %); ¹H NMR (500 MHz, CDCl₃) δ 9.48 (s, 1H), 7.42 - 7.37 (m, 2H), 7.32 - 7.27 (m, 3H), 7.17 (td, *J* = 8.3, 1.5, 1H), 7.07 (t, *J* = 7.4, 1H), 6.98 (d, *J* = 8.3, 1H), 6.92 (d, *J* = 8.3, 2H), 6.90 - 6.84 (m, 3H), 4.91 (dd, *J* = 10.2, 4.0, 1H), 4.71 (t, *J* = 10.2, 1H), 4.49 (dd, *J* = 10.2, 4.0, 1H), 3.83 (d, *J* = 17.7, 1H), 3.68 (d, *J* = 17.6, 1H), 2.50 - 2.36 (m, 1H), 0.78 - 0.68 (m, 1H), 0.66 - 0.50 (m, 3H). ¹³C NMR (126 MHz, CDCl₃) δ 172.1, 157.2, 154.5, 153.3, 131.8, 130.1, 129.8, 128.2, 123.3, 123.1, 122.6, 122.5, 119.3, 118.6, 113.7, 66.6, 57.6, 56.5, 35.4, 7.9, 6.6 ppm; MS (ESI) m/z calculated [M+H]⁺: 505.17; found [M+H]⁺: 505.32.

### Example 179: 2-((2-(2-chlorophenoxy)-1-(1H-tetrazol-5-yl)ethyl)(cyclopentyl)amino)-N-(4-phenoxyphenyl)acetamide

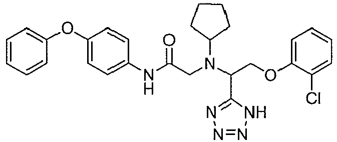

Yellow solid, Yield: 160 mg (77 %); ¹H NMR (500 MHz, CDCl₃) δ 9.76 (s, 1H), 7.47 (d, *J* = 8.9, 2H), 7.37 (dd, *J* = 7.9, 1.5, 1H), 7.36 - 7.31 (m, 2H), 7.25 - 7.20 (m, 1H), 7.11 (t, *J* = 7.4, 1H), 7.05 - 7.00 (m, 1H), 6.99 - 6.96 (m, 3H), 6.94 (d, *J* = 8.9, 2H), 5.01 (dd, *J* = 9.5, 3.6, 1H), 4.62 (t, *J* = 10.1, 1H), 4.47 (dd, *J* = 10.4, 4.1, 1H), 3.81 (d, *J* = 18.2, 1H), 3.53 (d, *J* = 18.1, 1H), 3.49 - 3.37 (m, 1H), 2.01 - 1.82 (m, 2H), 1.79 - 1.65 (m, 2H), 1.64- 1.39 (m, 4H). ¹³C NMR (126 MHz, CDCl₃) δ 172.6, 157.3, 154.2, 153.4, 132.0, 130.5, 129.8, 128.2, 123.2, 122.7, 122.5, 119.4, 118.6, 114.2, 67.5, 56.4, 53.4, 52.4, 30.8, 30.7, 23.6, 23.5 ppm; MS (ESI) m/z calculated [M+H]⁺: 533.20; found [M+H]⁺: 533.33.

### Example 180: 2-((2-(2-chlorophenoxy)-1-(1H-tetrazol-5-yl)ethyl)(propyl)amino)-N-(4-phenoxyphenyl)acetamide

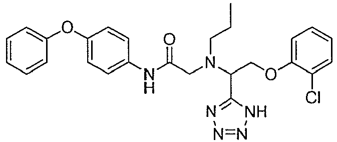

White solid, Yield: 40 mg (60 %); ¹H NMR (500 MHz, CDCl₃) δ 9.72 (s, 1H), 7.49 (d, *J* = 8.8, 2H), 7.37 - 7.31 (m, 3H), 7.24 - 7.19 (m, 1H), 7.10 (t, *J* = 7.4, 1H), 7.03 (d, *J* = 8.3, 1H), 6.98 - 6.92 (m, 5H), 4.89 (dd, *J* = 9.6, 3.5, 1H), 4.66 (t, *J* = 10.1, 1H), 4.46 (dd, *J* = 10.3, 3.8, 1H), 3.75 (d, *J* = 17.7, 1H), 3.45 (d, *J* = 17.7, 1H), 2.88 - 2.76 (m, 1H), 2.71 - 2.63 (m, 1H), 1.68 - 1.58 (m, 2H), 0.97 (t, *J* = 7.3, 3H). ¹³C NMR (126 MHz, CDCl₃) δ 171.4, 157.3, 154.2, 153.5, 132.2, 130.5, 129.7, 128.1, 123.2, 122.6, 122.5, 119.4, 118.6, 114.1, 67.2, 56.4, 55.6, 55.0, 21.5, 11.6 ppm; MS (ESI) m/z calculated [M+H]⁺: 507.18; found [M+H]⁺: 507.33.

### Tetrazole as building blocks:

### Method 9: General procedure for tetrazole synthesis by using Isocyanide, paraformaldehyde and TMS-N3

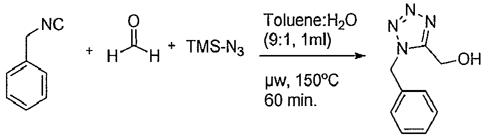

A 5 ml microwave vial equipped with a magnetic stir bar was charged with a benzylisocyanide (117 µl, 1.0 mmol) and paraformaldehyde (60 mg, 2.0 mmol) in toluene:H₂O (9:1, 1 ml) and trimethylsilyl azide (131 µl, 1.0 mmol) was added slowly at room temperature. Vial was sealed with cap containing a septum and subjected to microwave heating at 150 °C for 60 min [*attention: during irradiation, pressure develops*]. The solvent was removed under reduced pressure and residue was purified by silica gel flash chromatography using EtOAc-hexane as eluent on to afford the titled product.

### Following examples were synthesized by using Method 9: Method for tetrazole synthesis by using Isocyanide, paraformaldehyde and TMS-N3

### Example 181: (1-benzyl-1H-tetrazol-5-yl)methanol

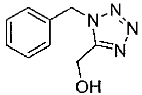

White Solid, Yield: 103 mg (54 %); ¹H NMR (500 MHz, CDCl₃) δ 7.39 - 7.33 (m, 3H), 7.32 - 7.27 (m, 2H), 5.65 (s, 2H), 4.83 (d, *J* = 6.0, 2H), 4.10 (s, 1H). MS (ESI) m/z calculated [M-H]⁻: 189.09; found [M-H]⁻: 189.09.

### Example 182: (1-(2,4,4-trimethylpentan-2-yl)-1H-tetrazol-5-yl)methanol

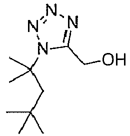

White Solid, Yield: 158 mg (74 %); ¹H NMR (500 MHz, CDCl₃) δ 5.06 (d, *J* = 6.3, 2H), 4.15 (t, *J* = 6.6, 1H), 2.02 (s, 2H), 1.83 (s, 6H), 0.78 (s, 9H). ¹³C NMR (126 MHz, CDCl₃) δ 154.0, 65.1, 55.4, 53.5, 31.7, 30.6, 29.9.

### Example 183: (1-(tert-butyl)-1H-tetrazol-5-yl)methanol

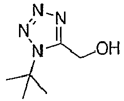

White Solid, Yield: 81 mg (52 %); ¹H NMR (500 MHz, CDCl₃) δ 5.05 (d, *J* = 6.6, 2H), 4.08 - 3.93 (m, 1H), 1.77 (s, 9H). ¹³C NMR (126 MHz, CDCl₃) δ 153.5, 61.8, 55.0, 29.6.

### Example 184: 3-(5-(hydroxymethyl)-1H-tetrazol-1-yl)propanenitrile

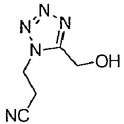

White Solid, Yield: 190 mg (62 %); ¹H NMR (500 MHz, MeOD) δ 4.01 (t, *J* = 2.8, 2H), 3.58 - 3.46 (m, 2H), 2.70 (td, *J* = 6.6, 2.9, 2H). ¹³C NMR (126 MHz, MeOD) δ 175.7, 119.3, 62.5, 36.0, 18.5. MS (ESI) m/z calculated [M+H]⁺: 154.07; found [M+H]⁺: 154.06.

### Example 185: (1-cyclohexyl-1H-tetrazol-5-yl)methanol

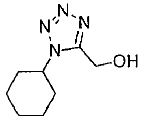

White Solid, Yield: 139 mg (77 %); ¹H NMR (500 MHz, CDCl₃) δ 4.96 (d, *J* = 6.7, 2H), 4.45 (tt, *J* = 11.4, 4.1, 1H), 3.48 (t, *J* = 6.6, 1H), 2.20 - 1.94 (m, 6H), 1.86 - 1.76 (m, 1H), 1.55 - 1.19 (m, 3H). ¹³C NMR (126 MHz, CDCl₃) δ 176.0, 58.5, 53.6, 32.9, 25.2, 24.8.

### Method 10: General procedure for Swern oxidation

### Reference: http://www.ochemonline.com/Swern_oxidation

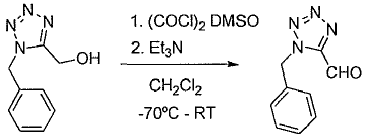

To a solution of oxalyl chloride (5 mmol) and 3 Å MS in CH₂Cl₂ (1 mL) at -78 °C under N₂□was added dropwise a solution of DMSO (10 mmol) in CH₂Cl₂ (1 mL). After 15 min a solution of the alcohol in CH₂Cl₂ (3 mL) was slowly added dropwise. After 30 min, Et₃N (15 mmol) was added dropwise. The reaction was stirred 30 min at -78 °C then slowly allowed to warm to rt. Water was added and stirred for few min to separate organic layer and extracted with DCM. The organic fraction was dried under reduced pressure and residue was purified by silica gel flash chromatography using EtOAc-hexane (40 %) as eluent on to afford the titled product.

### Following examples were synthesized by using Method 10: Method for Swern oxidation

### Example 186: 1-benzyl-1H-tetrazole-5-carbaldehyde

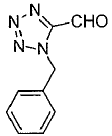

Colorless liquid, Yield: 76 mg (82 %); ¹H NMR (500 MHz, CDCl₃) δ 10.25 (s, 1H), 7.42 - 7.32 (m, 6H), 5.87 (s, 2H). ¹³C NMR (126 MHz, CDCl₃) δ 179.5, 148.7, 132.9, 129.3, 129.1, 129.0, 128.6, 52.7 ppm. MS (ESI) m/z calculated [M+H]⁺: 189.07; found [M+H]⁺: 189.14.

### Example 187: 1-(2,4,4-trimethylpentan-2-yl)-1H-tetrazole-5-carbaldehyde

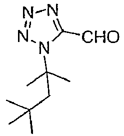

Colorless liquid, Yield: 540 mg (86 %); ¹H NMR (500 MHz, CDCl₃) δ 10.30 (s, 1H), 0.81 (s, 2H), 0.78 (s, 4H), 0.72 (s, 9H). ¹³C NMR (126 MHz, CDCl₃) δ 179.2, 140.6, 54.3, 51.2, 30.6, 29.9, 29.5 ppm.

### Example 188: 1-(tert-butyl)-1H-tetrazole-5-carbaldehyde

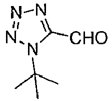

Pale yellow liquid, Yield: 600 mg (80 %); ¹H NMR (500 MHz, CDCl₃) δ 10.30 (s, 1H), 1.78 (s, 9H). ¹³C NMR (126 MHz, CDCl₃) δ 179.1, 150.6, 64.1, 28.7 ppm.

### Example 189: 1-(2,4,4-trimethylpentan-2-yl)-1H-tetrazole-5-carbaldehyde

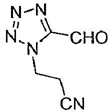

Colorless liquid, Yield: 45 mg (47 %); ¹H NMR (500 MHz, CDCl₃) δ 8.86 (s, 1H), 4.80 (t, *J* = 6.5, 2H), 3.13 (t, *J* = 6.5, 2H).

### Tetrazole-5-carbaldehydes as aldehyde component in Ugi and Passerini reaction

### Following examples were synthesized by using Method 7: Method for Ugi reaction

### Example 190: N-benzyl-2-(N-(3,4-dimethoxyphenethyl)acetamido)-2-(1-(2,4,4-trimethylpentan-2-yl)-1H-tetrazol-5-yl)acetamide

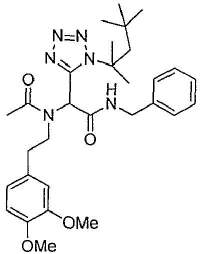

Yellowish solid, Yield: 115 mg (56 %); ¹H NMR (500 MHz, CDCl₃) δ 7.29 (d, *J* = 7.4, 2H), 7.26 - 7.21 (m, 3H), 7.15 (s, 1H), 6.77 (d, *J* = 8.1, 1H), 6.68 (s, 1H), 6.64 (d, *J* = 8.1, 1H), 6.46 (s, 1H), 4.48 (d, *J* = 5.7, 2H), 3.90 (s, 3H), 3.85 (s, 3H), 3.83 - 3.76 (m, 1H), 3.76 - 3.66 (m, 1H), 2.67 (td, *J* = 12.3, 5.2, 1H), 2.26 (s, 3H), 2.15 (td, *J* = 12.3, 5.3, 1H), 2.03 (d, *J* = 2.4, 2H), 1.88 (s, 3H), 1.80 (s, 3H), 0.79 (s, 9H). ¹³C NMR (126 MHz, CDCl₃) δ 171.9, 165.5, 161.1, 150.5, 149.2, 148.0, 137.2, 130.1, 128.8, 128.7, 127.8, 127.8, 127.7, 127.6, 120.6, 112.0, 111.5, 66.3, 56.0, 55.9, 53.6, 52.1, 48.9, 44.2, 35.8, 31.6, 30.7, 30.2, 30.0, 21.5 ppm; MS (ESI) m/z calculated [M+Na]⁺: 573.32; found [M+Na]⁺: 573.40.

### Example 191: 2-((2-cyanoethyl)amino)-2-oxo-1-(1-(2,4,4-trimethylpentan-2-yl)-1H-tetrazol-5-yl)ethyl benzoate

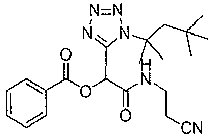

White solid, Yield: 110 mg (567%); ¹H NMR (500 MHz, CDCl₃) δ 8.12 - 8.05 (m, 2H), 7.64 (t, *J* = 7.5, 1H), 7.47 (t, *J* = 7.8, 2H), 7.33 (t, *J* = 6.0, 1H), 6.95 (s, 1H), 3.71 - 3.61 (m, 1H), 3.59 - 3.47 (m, 1H), 2.74 - 2.54 (m, 2H), 2.14 (q*, J* = 15.3, 2H), 1.96 (s, 3H), 1.91 (s, 3H), 0.80 (s, 9H). ¹³C NMR (126 MHz, CDCl₃) δ 165.5, 164.5, 150.4, 134.6, 130.2, 128.9, 127.5, 117.4, 66.8, 66.5, 54.4, 35.8, 31.7, 30.5, 30.4, 30.3, 18.0 ppm; MS (ESI) m/z calculated [M-H]⁻: 411.22; found [M-H]⁻: 411.29.

### Example 192: N-benzyl-2-(N-(3,4-dimethoxyphenethyl)acetamido)-2-(1-(2,4,4-trimethylpentan-2-yl)-1H-tetrazol-5-yl)acetamide

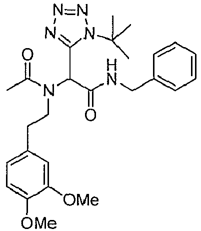

White solid, Yield: 140 mg (57 %); ¹H NMR (500 MHz, CDCl₃) δ 7.37 - 7.19 (m, 6H), 6.81 - 6.72 (m, 2H), 6.61 (s, 1H), 6.59 (s, 1H), 5.29 (s, 1H), 4.47 (dd, *J* = 7.7, 6.1, 2H), 3.87 (s, 3H), 3.83 (s, 3H), 3.81 - 3.75 (m, 1H), 3.75 - 3.65 (m, 1H), 2.64 (td, *J* = 12.4, 5.2, 1H), 2.22 (s, 3H), 2.10 - 2.04 (m, 1H), 1.75 (s, 9H). ¹³CNMR (126 MHz, CDCl₃) δ 172.0, 165.5, 150.4, 149.1, 147.9, 137.3, 129.9, 128.7, 127.8, 127.7, 120.5, 111.9, 111.4, 62.6, 55.9, 55.9, 52.2, 48.9, 44.2, 35.8, 29.8, 29.7, 21.5 ppm; MS (ESI) m/z calculated [M+Na]⁺: 517.25; found [M+Na]⁺: 517.37.

### Example 193: N-benzyl-2-(1(tert-butyl)-1H-tetrazol-5-yl)-2-(N-isopropylacetamido)acetamide

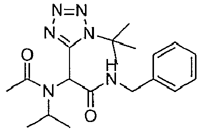

White solid, Yield: 84 mg (48 %); ¹H NMR (500 MHz, CDCl₃) δ 7.35 - 7.31 (m, 2H), 7.30 - 7.27 (m, 3H), 5.30 (s, 2H), 4.51 - 4.44 (m, 2H), 2.24 (s, 3H), 1.80 (s, 2H), 1.74 (s, 9H), 1.30 (d, *J* = 7.0, 3H), 1.26 (br s, 3H). ¹³C NMR (126 MHz, CDCl₃) δ 165.9, 165.8, 150.6, 128.8, 128.1, 127.8, 66.1, 53.4, 44.3, 43.9, 30.0, 29.9, 29.7 ppm; MS (ESI) m/z calculated [M+Na]⁺: 395.22; found [M+Na]⁺: 395.31.

### Example 194: N-((1-benzyl-1H-tetrazol-5-yl)(1-(tert-butyl)-1H-tetrazol-5-yl)methyl)-2-phenylethanamine

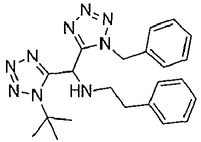

Brown solid, Yield: 162 mg (39 %); ¹H NMR (500 MHz, CDCl₃) δ 7.38 - 7.33 (m, 3H), 7.32 - 7.27 (m, 2H), 7.22 (t, *J=* 7.4, 1H), 7.14 (d, *J=* 7.1, 2H), 7.03 (dd, *J=* 7.1, 1.9, 2H), 5.78 - 5.65 (m, 2H), 5.16 (s, 1H), 2.88 - 2.77 (m, 1H), 2.72 - 2.59 (m, 2H), 2.57 - 2.43 (m, 1H), 2.26 (s, 1H), 1.39 (s, 9H). ¹³C NMR (126 MHz, CDCl₃) δ 179.1, 152.5, 151.5, 139.2, 133.2, 129.3, 129.1, 128.8, 128.6, 127.9, 126.6, 62.1, 51.9, 50.8, 49.7, 36.2, 29.5 ppm; MS (ESI) m/z calculated [M+Na]⁺: 440.23; found [M+Na]⁺: 440.30.

### Example 195: 1-(1-benzyl-1H-tetrazol-5-yl)-2-((2-cyanoethyl)amino)-2-oxoethyl benzoate

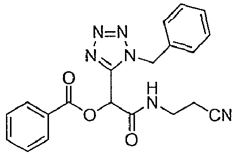

White viscous liquid, Yield: 120 mg (58 %); ¹H NMR (500 MHz, CDCl₃) δ 8.03 (t, *J* = 6.1, 1H), 7.75 (d, *J* = 7.3, 2H), 7.49 (t, *J* = 7.5, 1H), 7.35 - 7.27 (m, 5H), 7.23 (dd, *J* = 7.2, 1.7, 2H), 6.67 (s, 1H), 5.90 (d, *J* = 15.5, 1H), 5.81 (d, *J* = 15.6, 1H), 3.66 - 3.42 (m, 2H), 2.58 (t, *J* = 6.6, 2H). ¹³C NMR (126 MHz, CDCl₃) δ 179.6, 164.8, 164.6, 150.6, 134.2, 133.2, 130.1, 129.1, 128.8, 128.6, 127.7, 127.4, 117.9, 65.9, 52.0, 35.8, 17.9 ppm; MS (ESI) m/z calculated [M+Na]⁺: 413.13; found [M+Na]⁺: 413.21.

**The following further examples have been synthesized:**

| No. | Structure | Name | Chemica 1 formula | Mass [M+Na]⁺ |
|---|---|---|---|---|
| 196 | 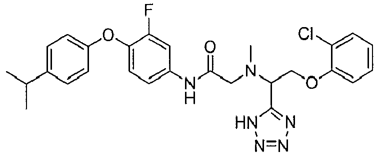 | 2-((2-(2-chlorophenoxy)-1-(1H-tetrazol-5-yl)ethyl)(methyl)amino)-N-(3-fluoro-4-(4-isopropylphenoxy)phenyl) acetamide | C₂₇H₂₈Cl FN₆O₃ | 561.18 |
| 197 | 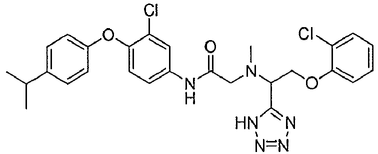 | N-(3-chloro-4-(4-isopropylphenoxy)phenyl) -2-((2-(2-chlorophenoxy)-1-(1H-tetrazol-5-yl)ethyl)(methyl)amino)ac etamide | C₂₇H₂₈Cl ₂N₆O₃ | 577.15 |
| 198 | 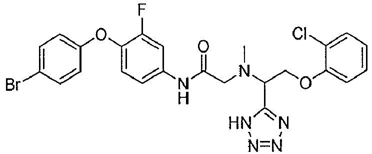 | N-(4-(4-bromophenoxy)-3-fluorophenyl)-2-((2-(2-chlorophenoxy)-1-(1H-tetrazol-5-yl)ethyl)(methyl)amino)ac etamide | C₂₄H₂₁BΓ ClFN₆O₃ | 597.04 |
| 199 | 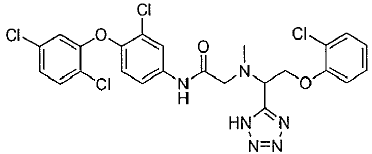 | N-(3-chloro-4-(2,5-dichlorophenoxy)phenyl)-2-((2-(2-chlorophenoxy)-1-(1H-tetrazol-5-yl)ethyl)(methyl)amino)ac etamide | C₂₄H₂₀Cl ₄N₆O₃ | 603.02 |
| 200 | 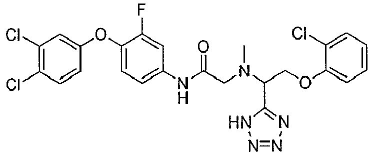 | 2-((2-(2-chlorophenoxy)-1-(1H-tetrazol-5-yl)ethyl)(methyl)amino)-N-(4-(3,4-dichlorophenoxy)-3-fluorophenyl)acetamide | C₂₄H₂₀Cl ₃FN₆O₃ | 587.05 |
| 201 | 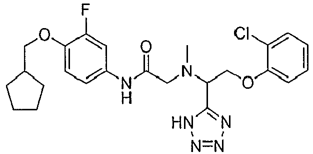 | 2-((2-(2-chlorophenoxy)-1-(1H-tetrazol-5-yl)ethyl)(methyl)amino)-N-(4-(cyclopentylmethoxy)-3-fluorophenyl)acetamide | C₂₄H₂₈Cl FN₆O₃ | 525.18 |
| 202 | 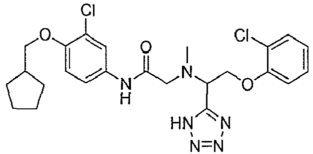 | N-(3-chloro-4-(cyclopentylmethoxy)phen yl)-2-((2-(2-chlorophenoxy)-1-(1H-tetrazol-5-yl)ethyl)(methyl)amino)ac etamide | C₂₄H₂₈Cl ₂N₆O₃ | 541.15 |
| 203 | 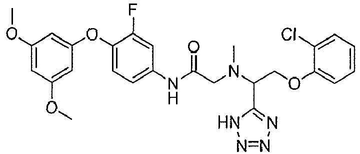 | 2-((2-(2-chlorophenoxy)-1-(1H-tetrazol-5-yl)ethyl)(methyl)amino)-N-(4-(3,5-dimethoxyphenoxy)-3-fluorophenyl)acetamide | C₂₆H₂₆Cl FN₆O₅ | 579.15 |
| 204 | 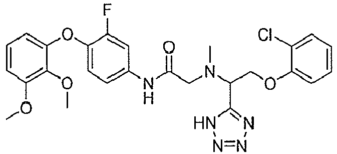 | 2-((2-(2-chlorophenoxy)-1-(1H-tetrazol-5-yl)ethyl)(methyl)amino)-N-(4-(2,3-dimethoxyphenoxy)-3-fluorophenyl)acetamide | C₂₆H₂₆Cl FN₆O₅ | 579.15 |
| 205 | 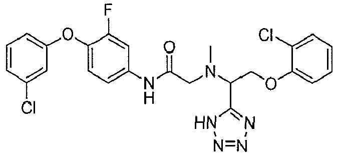 | 2-((2-(2-chlorophenoxy)-1-(1H-tetrazol-5-yl)ethyl)(methyl)amino)-N-(4-(3-chlorophenoxy)-3-fluorophenyl)acetamide | C₂₄H₂₁Cl ₂FN₆O₃ | 553.09 |
| 206 | 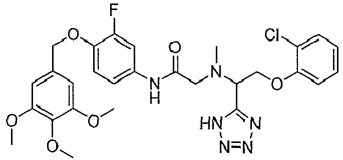 | 2-((2-(2-chlorophenoxy)-1-(1H-tetrazol-5-yl)ethyl)(methyl)amino)-N-(3-fluoro-4-((3,4,5-trimethoxybenzyl)oxy)phe nyl)acetamide | C₂₈H₃₀Cl FN₆O₆ | 623.18 |
| 207 | 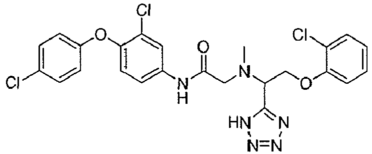 | N-(3-chloro-4-(4-chlorophenoxy)phenyl)-2-((2-(2-chlorophenoxy)-1-(1H-tetrazol-5-yl)ethyl)(methyl)amino)ac etamide | C₂₄H₂₁Cl ₃N₆O₃ | 569.06 |
| 208 | 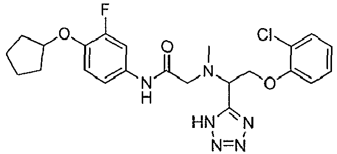 | 2-((2-(2-chlorophenoxy)-1-(1H-tetrazol-5-yl)ethyl)(methyl)amino)-N-(4-(cyclopentyloxy)- 3-fluorophenyl)acetamide | C₂₃H₂₆Cl FN₆O₃ | 511.16 |
| 209 | 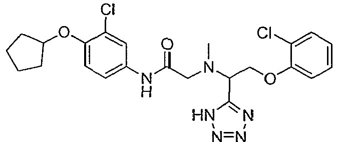 | N-(3-chloro-4-(cyclopentyloxy)phenyl)-2-((2-(2-chlorophenoxy)-1-(1H-tetrazol-5-yl)ethyl)(methyl)amino)acetamide | C₂₃H₂₆Cl ₂N₆O₃ | 527.13 |
| 210 | 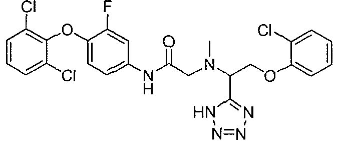 | 2-((2-(2-chlorophenoxy)-1-(1H-tetrazol-5-yl)ethyl)(methyl)amino)-N-(4-(2,6-dichlorophenoxy)-3-fluorophenyl)acetamide | C₂₄H₂₀Cl ₃FN₆O₃ | 587.05 |
| 211 | 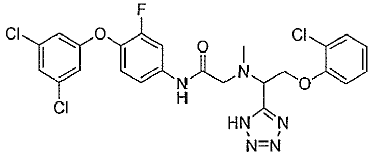 | 2-((2-(2-chlorophenoxy)-1-(1H-tetrazol-5-yl)ethyl)(methyl)amino)-N-(4-(3,5-dichlorophenoxy)-3-fluorophenyl)acetamide | C₂₄H₂₀Cl ₃FN₆O₃ | 587.05 |
| 212 | 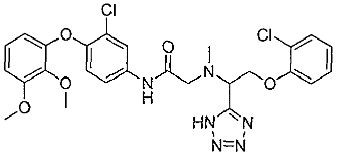 | N-(3-chloro-4-(2,3-dimethoxyphenoxy)phenyl )-2-((2-(2-chlorophenoxy)-1-(1H-tetrazol-5-yl)ethyl) (methyl)amino)ac etamide | C₂₆H₂₆Cl ₂N₆O₅ | 595.12 |
| 213 | 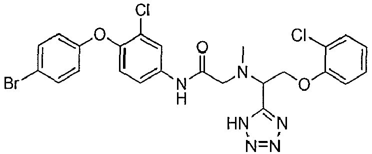 | N-(4-(4-bromophenoxy)-3-chlorophenyl)-2-((2-(2-chlorophenoxy)-1-(1H-tetrazol-5-yl)ethyl)(methyl)amino)ac etamide | C₂₄H₂₁Br Cl₂N₆O₃ | 613.01 |
| 214 | 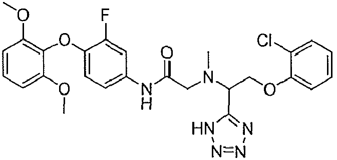 | 2-((2-(2-chlorophenoxy)-1-(1H-tetrazol-5-yl)ethyl)(methyl)amino)-N-(4-(2,6-dimethoxyphenoxy)-3-fluorophenyl)acetamide | C₂₆H₂₆Cl FN₆O₅ | 579.15 |
| 215 | 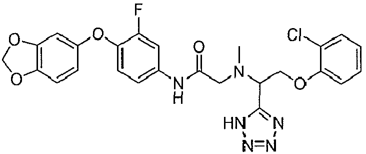 | N-(4-(benzo[d][1,3]dioxol-5-yloxy)-3 -fluorophenyl)-2-((2-(2-chlorophenoxy)-1-(1H-tetrazol-5-yl)ethyl)(methyl)amino)ac etamide | C₂₅H₂₂Cl FN₆O₅ | 563.12 |
| 216 | 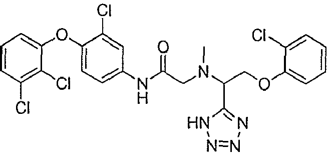 | N-(3-chloro-4-(2,3-dichlorophenoxy)phenyl)-2-((2-(2-chlorophenoxy)-1-(1H-tetrazol-5-yl)ethyl)(methyl)amino)ac etamide | C₂₄H₂₀Cl ₄N₆O₃ | 603.02 |
| 217 | 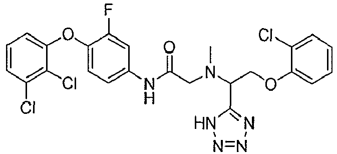 | 2-((2-(2-chlorophenoxy)-1-(1H-tetrazol-5-yl)ethyl)(methyl)amino)-N-(4-(2,3-dichlorophenoxy)-3-fluorophenyl)acetamide | C₂₄H₂₀Cl ₃FN₆O₃ | 587.05 |
| 218 | 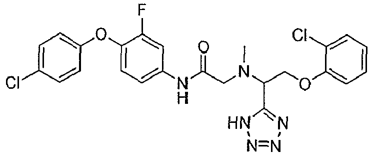 | 2-((2-(2-chlorophenoxy)-1-(1H-tetrazol-5-yl)ethyl)(methyl)amino)-N-(4-(4-chlorophenoxy)-3-fluorophenyl)acetamide | C₂₄H₂₁Cl ₂FN₆O₃ | 553.09 |
| 219 | 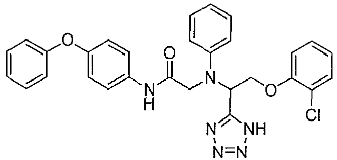 | 2-((2-(2-chlorophenoxy)-1-(1H-tetrazol-5-yl)ethyl)(phenyl)amino)-N-(4-phenoxyphenyl)acetamide | C₂₉H₂₅Cl N₆O₃ | 563.16 |
| 220 | 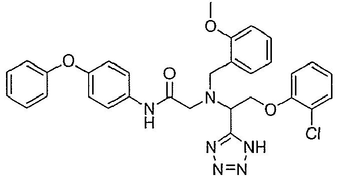 | 2-((2-(2-chlorophenoxy)-1-(1H-tetrazol-5-yl)ethyl) (2-methoxybenzyl)amino)-N-(4-phenoxyphenyl)acetamide | C₃₁H₂₉Cl N₆O₄ | 607.18 |
| 221 | 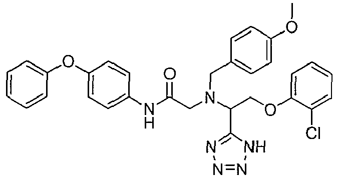 | 2-((2-(2-chlorophenoxy)-1-(1H-tetrazol-5-yl)ethyl)(4-methoxybenzyl)amino)-N-(4-phenoxyphenyl)acetamide | C₃₁H₂₉Cl N₆O₄ | 607.18 |
| 222 | 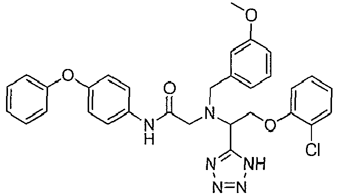 | 2-((2-(2-chlorophenoxy)-1-(1H-tetrazol-5-yl)ethyl)(3-methoxybenzyl)amino)-N-(4-phenoxyphenyl)acetamide | C₃₁H₂₉Cl N₆O₄ | 607.18 |
| 223 | 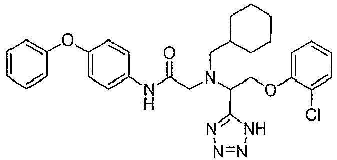 | 2-((2-(2-chlorophenoxy)-1-(1H-tetrazol-5-yl)ethyl) (cyclohexylmethy l)amino)-N-(4-phenoxyphenyl)acetamide | C₃₀H₃₃Cl N₆O₃ | 583.22 |
| 224 | 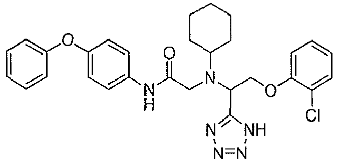 | 2-((2-(2-chlorophenoxy)-1-(1H-tetrazol-5-yl)ethyl)(cyclohexyl)amin o)-N-(4-phenoxyphenyl)acetamide | C₂₉H₃₁Cl N₆O₃ | 569.20 |
| 225 | 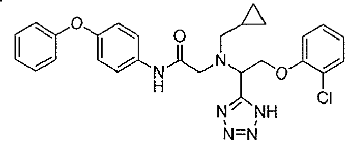 | 2-((2-(2-chlorophenoxy)-1-(1H-tetrazol-5-yl)ethyl)(cyclopropylmeth yl)amino)-N-(4-phenoxyphenyl)acetamide | C₂₇H₂₇Cl N₆O₃ | 541.17 |
| 226 | 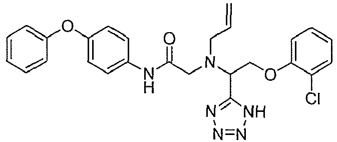 | 2-(allyl(2-(2-chlorophenoxy)-1-(1H-tetrazol-5-yl)ethyl)amino)-N-(4-phenoxyphenyl)acetamide | C₂₆H₂₅Cl N₆O₃ | 527.16 |
| 227 | 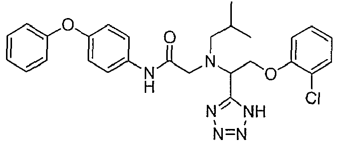 | 2-((2-(2-chlorophenoxy)-1-(1H-tetrazol-5-yl)ethyl)(isobutyl)amino)-N-(4-phenoxyphenyl)acetamide | C₂₇H₂₉Cl N₆O₃ | 543.19 |
| 228 | 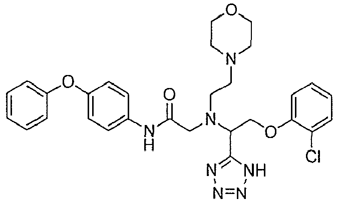 | 2-((2-(2-chlorophenoxy)-1-(1H-tetrazol-5-yl)ethyl)(2-morpholinoethyl)amino)-N-(4-phenoxyphenyl)acetamide | C₂₉H₃₂Cl N₇O₄ | 600.21 |
| 229 | 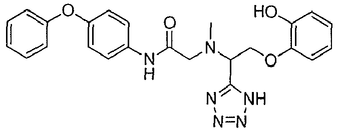 | 2-((2-(2-hydroxyphenoxy)-1-(1H-tetrazol-5-yl)ethyl)(methyl)amino)-N-(4-phenoxyphenyl)acetamide | C₂₄H₂₄N₆ O₄ | 483.18 |
| 230 | 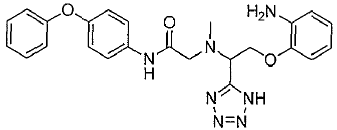 | 2-((2-(2-aminophenoxy)-1-(1H-tetrazol-5-yl)ethyl)(methyl)amino)-N-(4-phenoxyphenyl)acetamide | C₂₄H₂₅N₇ O₃ | 482.19 |
| 231 | 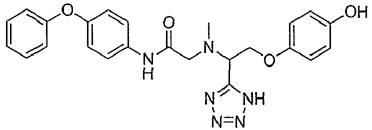 | 2-((2-(4-hydroxyphenoxy)-1-(1H-tetrazol-5-yl)ethyl)(methyl)amino)-N-(4-phenoxyphenyl)acetamide | C₂₄H₂₄N₆ O₄ | 483.18 |
| 232 | 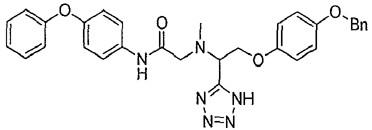 | 2-((2-(4-(benzyloxy)phenoxy)-1-(1H-tetrazol-5-yl)ethyl)(methyl)amino)-N-(4-phenoxyphenyl)acetamide | C₃₁H₃₀N₆ O₄ | 573.22 |
| 233 | 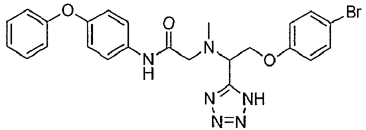 | 2-((2-(4-bromophenoxy)-1-(1H-tetrazol-5-yl)ethyl)(methyl)amino)-N-(4-phenoxyphenyl)acetamide | C₂₄H₂₃Br N₆O₃ | 545.09 |
| 234 | 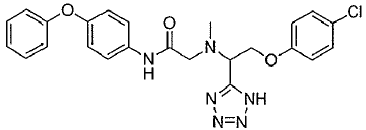 | 2-((2-(4-chlorophenoxy)-1-(1H-tetrazol-5-yl)ethyl)(methyl)amino)-N-(4-phenoxyphenyl)acetamide | C₂₄H₂₃Cl N₆O₃ | 501.14 |
| 235 | 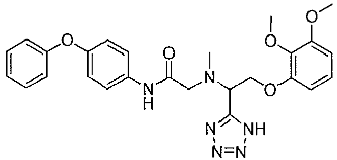 | 2-((2-(2,3-dimethoxyphenoxy)-1-(1H-tetrazol-5-yl)ethyl)(methyl)amino)-N-(4-phenoxyphenyl)acetamide | C₂₆H₂₈N₆ O₅ | 527.20 |
| 236 | 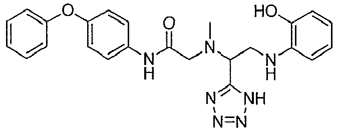 | 2-((2-((2-hydroxyphenyl)amino)-1-(1 H-tetrazol-5-yl)ethyl)(methyl)amino)-N-(4-phenoxyphenyl)acetamide | C₂₄H₂₅N₇ O₃ | 482.19 |
| 237 | 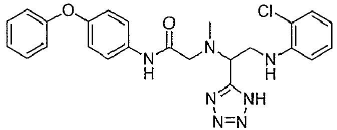 | 2-((2-((2-chlorophenyl)amino)-1-(1H-tetrazol-5-yl)ethyl)(methyl)amino)-N-(4-phenoxyphenyl)acetamide | C₂₄H₂₄Cl N₇O₂ | 500.16 |
| 238 | 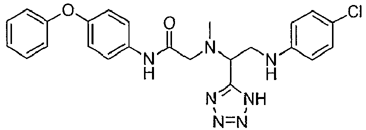 | 2-((2-((4-chlorophenyl)amino)-1-(1H-tetrazol-5-yl)ethyl)(methyl)amino)-N-(4-phenoxyphenyl)acetamide | C₂₄H₂₄Cl N₇O₂ | 500.16 |
| 239 | 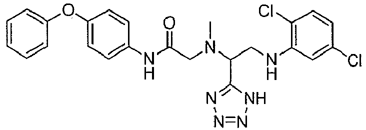 | 2-((2-((2,5-dichlorophenyl)amino)-1-(1H-tetrazol-5-yl)ethyl)(methyl)amino)-N-(4-phenoxyphenyl)acetamide | C₂₄H₂₃Cl ₂N₇O₂ | 534.12 |
| 240 | 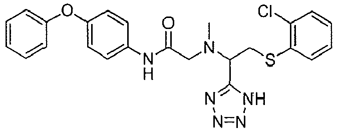 | 2-((2-((2-chlorophenyl)thio)-1-(1H-tetrazol-5-yl)ethyl)(methyl)amino)-N-(4-phenoxyphenyl)acetamide | C₂₄H₂₃Cl N₆O₂S | 517.12 |
| 241 | 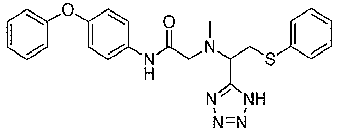 | 2-(methyl(2-(phenylthio)-1-(1H-tetrazol-5-yl)ethyl)amino)-N-(4-phenoxyphenyl)acetamide | C₂₄H₂₄N₆ O₂S | 483.16 |

## Claims

1. A compound of formula (I): 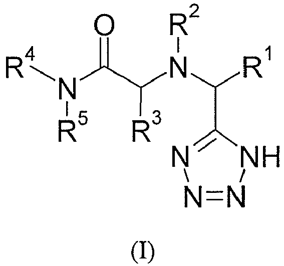 wherein
R¹ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R² is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R³ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R⁴ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted; and
R⁵ is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
or a pharmaceutically acceptable salt, ester, solvate or hydrate or a pharmaceutically acceptable formulation thereof.

2. A compound according to claim 1, wherein R³ is hydrogen.

3. A compound according to claim 1 or 2, wherein R⁴ is hydrogen.

4. A compound according to any one of the preceding claims, wherein R¹ is alkylcycloalkyl containing 4 to 16 carbon atoms; heteroalkylcycloalkyl containing 4 to 16 atoms selected from C, O, S and N; aralkyl containing 7 to 16 carbon atoms; or heteroaralkyl containing 6 to 16 atoms selected from C, O, S and N; wherein all these groups may optionally be substituted.

5. A compound according to any one of the preceding claims 1 to 3, wherein R¹ is a group of formula -CH₂-X-R¹⁰ wherein X is O, S or NH and R¹⁰ is a cycloalkyl, heterocycloalkyl, alkylcycloalkyl, heteroalkylcycloalkyl, aryl, heteroaryl, aralkyl or heteroaralkyl group, all of which groups may optionally be substituted..

6. A compound according to claim 5, wherein R¹⁰ is phenyl, naphthyl or a heteroaryl group containing one or two rings and 5 to 10 ring atoms selected from C, O, S and N, wherein all these groups may optionally be substituted by halogen, OH, NO₂, OMe, Me, NH₂, NHMe or NMe₂ or a group of formula -O-CH₂CH₂-O- or -O-CH₂-O-.

7. A compound according to any one of the preceding claims, wherein R² is C₁₋₆ alkyl; C₂₋₆ alkenyl; C₁₋₆ alkynyl; heteroalkyl containing 1 to 6 carbon atoms and 1 to 4 heteroatoms selected fom O, S and N; C₃₋₇ cycloalkyl; C₄₋₁₀ alkylcycloalkyl; heterocycloalkyl containing 3 to 7 ring atoms selected from C, O, S and N; heteroalkylcycloalkyl containing 4 to 10 atoms selected from C, O, S and N; phenyl; heteroaryl containing 5 or 6 ring atoms selected from C, O, S and N; aralkyl containing 7 to 12 carbon atoms; or heteroaralkyl containing 6 to 12 atoms selected from C, O, S and N; wherein all these groups may optionally be substituted by halogen, OH, NO₂, OMe, Me, =O, NH₂, NHMe or NMe₂.

8. A compound according to any one of the preceding claims 1 to 6, wherein R² is methyl.

9. A compound according to any one of the preceding claims, wherein R⁵ is an aryl, heteroaryl, aralkyl or heteroaralkyl group, all of which groups may optionally be substituted.

10. A compound according to any one of the preceding claims 1 to 8, wherein R⁵ is a group of formula -Ar-L-Cy, wherein Ar is an arylene, heteroarylene, aralkylene or heteroaralkylene group, all of which groups may optionally be substituted; L is a bond, -O-, -NH-, -S- or a C₁-₄ alkyl group or a heteroalkyl group containing 1 to 6 atoms selected from C, O, S and N; and Cy is a cycloalkyl, heterocycloalkyl, alkylcycloalkyl, heteroalkylcycloalkyl, aryl, heteroaryl, aralkyl or heteroaralkyl group, all of which groups may optionally be substituted.

11. A compound according to claim 10, wherein Ar is an optionally substituted phenylene group or an optionally substituted heteroarylene group containing 5 or 6 ring atoms selected from C, O, S and N.

12. A compound according to claim 10 or 11, wherein L is -O-, -CH₂O- or -OCH₂-.

13. A compound according to claim 10, 11 or 12, wherein Cy is cycloalkyl containing one or two rings and 3 to 10 carbon atoms; heterocycloalkyl containing one or two rings and 3 to 10 ring atoms selected from C, O, S and N; phenyl; naphthyl; or heteroaryl containing one or two rings and 5 to 10 ring atoms selected from C, O, S and N; all of which groups may optionally be substituted.

14. A pharmaceutical composition comprising a compound according to anyone of the preceding claims or a pharmaceutically acceptable ester, prodrug, hydrate, solvate or salt thereof, optionally in combination with a pharmaceutically acceptable carrier.

15. A compound or a pharmaceutical composition according to anyone of the preceding claims for use in the treatment of cancer and infectious diseases.
